# EUROPEAN PATENT SPECIFICATION

(11) **EP 4 041 727 B1**
(45) Date of publication and mention of the grant of the patent: **20.05.2026**
(21) Application number: 20790252.9
(22) Date of filing: 09.10.2020
(51) Int. Cl.: C07D 413/14, C07D 417/14, A61K 31/42, A61K 31/433, A61P 3/10, A61P 25/28, A61P 35/00

(54) **ATF6 MODULATORS AND USES THEREOF**
ATF6-MODULATOREN UND VERWENDUNGEN DAVON
MODULATEURS ATF6 ET LEURS UTILISATIONS

(30) Priority: 09.10.2019 US 201962913126 P
(43) Date of publication of application: 17.08.2022
(73) Proprietor: Altos Labs, Inc., Redwood City, CA 94065 (US)
(72) Inventor: PUJALA, Brahmam, San Francisco, California 94158 (US); SATHE, Balaji Dashrath, San Francisco, California 94158 (US); THAKRAL, Pooja, San Francisco, California 94158 (US); BERNALES, Sebastian, San Francisco, California 94158 (US); URETA DÍAZ, Gonzalo Andrés, Santiago, 1482 (CL); BELMAR, Sebastian, Santiago, 1482 (CL)
(74) Representative: Mewburn Ellis LLP
(86) International application number: PCT/EP2020/078478
(87) International publication number: WO 2021/069701

(56) References cited:
- WO-A1-2019/209962
- WO-A2-2019/195810
- GALLAGHER: "Ceapins are a new class of unfolded protein response inhibitors, selectively targeting the ATF6[alpha] branch", ELIFE, vol. 5, 20 July 2016 (2016-07-20), XP055625340

## Description

### CROSS-REFERENCE TO RELATED APPLICATIONS

This application claims prior benefit of U.S. Provisional Patent Application No. 62/913,126, filed October 9, 2019.

### FIELD OF THE INVENTION

This disclosure relates generally to therapeutic agents that may be useful as modulators of Activating Transcription Factor 6 (ATF6).

### SUBMISSION OF SEQUENCE LISTING ON ASCII TEXT FILE

The content of the following submission on ASCII text file is part of the disclosure: a computer readable form (CRF) of the Sequence Listing (file name: 196052001440SEQLIST.TXT, date recorded: October 8, 2020, size: 1 KB).

### BACKGROUND

The accumulation of misfolded proteins in the EP of mammalian cells causes the folding machinery to become overwhelmed and leads to a stress response. Cells attempt to decrease the ER protein load by sending signals from the ER to the nucleus, activating a vast gene expression program that increases the protein-folding capacity in the ER. However, if this system fails and homeostasis cannot be re-established, cells die by engaging apoptosis. The unfolded protein response (UPR) is an evolutionarily conserved signal transduction pathway that maintains protein homeostasis in response to ER stress.

Three intertwined signaling pathways comprise the UPR: (1) PERK (protein kinase RNA-like ER kinase); (2) IRE1 (inositol-requiring enzyme 1α); and (3) ATF6 (Activating transcription factor 6) (McKimpson, W.M. et al, Circ Res, 2017, 120(5): 759-761). Activation of the ATF6 pathway leads to the upregulation of genes, such as BIP (Grp78), CHOP, or XBP-1, that enhance the capacity of the endoplasmic reticulum to fold proteins or mediate quality control. ATF6 works in partnership with IRE1, as one of the target genes of ATF6 is XBP1, the key substrate of IRE1 (Yoshida, H., et al., Cell, 2001, 107(7): 881-891). PERK performs several other roles, including pausing the production of new proteins to temporarily lower the protein-folding burden.

ATF6 is a type-II transmembrane protein localized in the ER that functions as an ER stress sensor and transcription factor (Adachi, Y., et al., Cell Struct Funct, 2008, 33(1): 75-89; Wu, J., et al., Dev Cell, 2007, 13(3): 351-64). When demand exceeds the folding capacity of the ER, ATF6 is transported from the ER to the Golgi apparatus, where sequential cleavage by two Golgi-resident proteases, site-1 and site-2 proteases (S1P and S2P), releases its N-terminal domain (ATF6N) from the Golgi membrane to be imported into the nucleus where it activates transcription of its target genes (Ye, J., et al., Mol Cell, 2000, 6(6): 1355-64). This activation involves binding of ATF6 to a consensus sequence called the ER-stress responsive element (ERSE). The consensus sequence of ERSE is CCAATCGGCGGCGGCCACG (SEQ ID NO. 1).

Unlike the other arms of the UPR regulated by PERK and IRE1, the ATF6 arm has not been substantially linked to proapoptotic signaling (Hetz, C. and Papa, F.R. 2018; Sano, R. and Reed, J.C. 2013). Instead, ATF6 primarily functions in the so-called "adaptive UPR" designed to promote protective, adaptive remodeling of cellular physiology and recovery following acute physiological and pathological insults. As part of the adaptive UPR, ATF6 integrates with multiple other stress-responsive signaling pathways to sensitively adapt cellular physiology to diverse types of ER insult.

In the context of the UPR, this integration can be achieved through heterodimerization of ATF6 with other UPR-regulated bZIP transcription factors, such as XBP1s (Yamamoto, K. et al. 2007; Shoulders, M.D. et al. 2013) or ATF6b (Thuerauf, D.J. et al. 2007; Thuerauf, D.J. et al. 2004; Forouhan, M. et al. 2018; Pieper, L.A. et al. 2017). ATF6 also has the potential to heterodimerize with other bZIP transcription factors similarly regulated through a mechanism involving S1P/S2P-dependent proteolysis, such as CREB-H (Asada, R. et al. 2011; Zhang, K. et al. 2006). Apart from heterodimerization, ATF6 signaling also integrates with other stress-responsive signaling pathways interacting with other transcription factors, such as NRF1, PGC1a, PPARa, and ERRg (Chen, X. et al. 2016; Wu, J. et al. 2011; Misra, J. et al. 2013; Baird, L. et al. 2017).

The capacity for ATF6 to integrate with other signaling pathways through multiple mechanisms reflects a unique potential for this UPR signaling arm to coordinate protective cellular responses, in addition to ER proteostasis remodeling, to a range of pathological insults that induce ER stress. The establishment of new pharmacological approaches to both inhibit and activate ATF6 signaling provides new opportunities to carefully dissect the timing and extent of ATF6 signaling involved in protecting different tissues against cancer, autoimmune diseases, neurodegeneration, metabolic diseases, or I/R.

Several strategies to manipulate the UPR have been exploited to define possible links between ER stress and human disease, with great advances in cancer and neurodegeneration.

In cancer, tumor growth relies on the UPR as a selective force to drive malignant transformation (Cubillos-Ruiz, J.R. et al. 2016), in addition to remodeling the tumor microenvironment and anticancer immune responses (Song, M and Cubillos-Ruiz, J.R. 2019), as well as impacting on other central hallmarks of cancer (Urra, H. et al. 2016).

For example, multiple myeloma (MM) remains a predominantly incurable malignancy despite high-dose chemotherapy, autologous stem cell transplant, and novel agents. Proteasome inhibitors (PI) such as Bortezomib have increased the response rate and survival of patients with MM. The overall patient response rate of newly diagnosed MM to Bortezomib and Dexamethasone is about 67%. In relapsed refractory MM, the response rate is reduced to about 40-60%. Therefore, there is a significant number of MM patients who are resistant to Bortezomib. MM cells are inherently sensitive to PIs because of their large volume of immunoglobulin production, which requires the constitutive expression of physiologic UPR genes. This appears to lower their threshold for the induction of a proapoptotic/terminal UPR in response to PI-induced endoplasmic reticulum (ER) stress. One of the hallmarks of UPR induction is the increased transcription and translation of ER molecular chaperones. These genes are induced by the UPR transcription factors XBP1 and ATF6. Although XBP1 splicing and its resulting activation have been shown to be inhibited in PI-treated MM cells, findings show that the high constitutive expression of 2 XBP1 target gene products, GRP78 and GRP94, is not reduced by PI treatment, and the observation that the XBP1-dependent UPR target gene ERdj4 was normally induced by PIs suggests that the UPR remains functional in PI-treated MM cells. Because both XBP1 and ATF6 can bind to ER stress response elements in the promoters of UPR target genes, it has been suggested that ATF6 may compensate for decreased XBP1 activity in PI-treated MM cells. Consistent with this, it has been shown that the induction of GRP78 and GRP94 is only slightly impaired in XBP1^{-/-} B cells and that the expression of GRP94 requires either, but not both, ATF6 or XBP1. Interestingly, previous studies have shown that XBP1 predicts sensitivity to Bortezomib, and its level correlates proportionally with sensitivity to Bortezomib. Recently, Harnoss JM et al. demonstrated using genetic and pharmacologic disruption that, in vitro and in vivo, the IRE1a-XBP1s pathway plays a critical role in MM growth. Indeed, the inhibition of IRE1α kinase activity using a small molecule was demonstrated to be a potentially effective and safe therapy for treating MM clinically.

In addition to the amount, PI sensitivity also appears to involve the efficiency of immunoglobulin folding within MM cells. The high constitutive expression of the ER resident chaperones GRP78 and GRP94 in MM cell lines is consistent with reports that physiologic UPR gene expression is required for professional secretory cell function. Elevated levels of ER chaperones are characteristic of plasma cells, and their expression is essential for proper antibody assembly and secretion. GRP78 has been shown to stably bind to immunoglobulin heavy chains that have not yet associated with immunoglobulin light chains and to assist in immunoglobulin assembly. Furthermore, both GRP78 and GRP94 are important for immunoglobulin light chain folding and targeting unassembled subunits for degradation. The fact that the expression of GRP78 and GRP94 is only slightly increased in MM cells treated with PIs and classical ER stress agents suggests they already express near-maximal levels of cytoprotective UPR proteins to function as secretory cells. Thus, these cells may have a lower threshold (compared with non-secretory cells) for induction of the terminal UPR following any additional stress to the ER. Hence, more resistant myeloma clones, as well as other non-secretory malignancies, may be sensitized to bortezomib by combining it with agents that interfere with the UPR, such as modulators of the ATF6 signaling pathway.

On the other hand, the protein folding capacity of professional secretory cells is overwhelmed in several diseases, leading to cell degeneration and death through terminal UPR signaling. For example, dysregulated UPR signaling in insulin-secreting pancreatic β cells results in premature cell loss, insulin deficiency, and diabetes. Although neurons are not typically considered to be classical secretory cells, the accumulation of abnormal aggregates may nonetheless induce the terminal UPR in neurons and lead to neurodegeneration (Hetz, C. and Saxena, S. 2017). ER stress has been implicated in ocular diseases and in the death of neuronal photoreceptor cells (Kroeger, H. et al. 2019).

In addition, many diseases lead to impaired circulation, which can cause ischemic conditions in a variety of organs, including the brain, heart, and kidneys (Benjamin, E.J. et al. 2018). In these settings, prolonged ischemia causes irreversible damage, which can be partially mitigated by clinical interventions that restore blood flow through reperfusion (Hausenloy, D.J. and Yellon, D.M. 2016). While reperfusion is necessary to mitigate the damage of continued ischemia, reperfusion itself is known to cause some additional damage, generally as a result of reactive oxygen species (ROS) (Murphy, E. and Steenbergen, C. 2008). The complex nature of cellular injury associated with ischemia or ischemia followed by reperfusion (I/R) has previously been shown to affect the levels and activities of numerous signaling pathways and transcription factors. One I/R-activated pathway that has been more recently studied involves the disruption of proteostasis. To protect against pathological ER stress induced by I/R, tissues activate endogenous adaptive stress-responsive signaling pathways, such as the unfolded protein response (UPR).

Recent evidence highlights a protective role for the ATF6 arm of the UPR in mitigating adverse outcomes associated with ischemia/reperfusion (I/R) injury in multiple disease models (Kudo, T. et al. 2008; Oida, Y. et al. 2008; Prachasilchai, W. et al. 2009; Oida, Y. et al. 2010; Blackwood, E.A. et al. 2019; Yu, Z. et al. 2017; Bi, X. et al. 2018), suggesting ATF6 as a potential therapeutic target for intervening pharmacologically with activator compounds in diverse ischemia-related disorders (Plate, L. et al. 2016; Glembotski, C.C. et al 2019).

ATF6-activated transcription targets play a role in the pathogenesis and development of various diseases, including viral infection, cancer, neurodegeneration, Alzheimer's disease, cerebral ischemia, hereditary cerebellar atrophy and ataxia, type 2 diabetes mellitus, and diabetic nephropathy, as well as cardiovascular diseases, such as myocardial atrophy, heart failure, ischemic heart disease and atherosclerosis (Chu, W.S., et al., Diabetes, 2007, 56(3): 856-62; Vekich, J.A., et al., J Mol Cell Cardiol, 2012, 53(2): 259-67, Liu, C.L., et al., Int J Mol Med, 2016, 37(2): 407-14). Therefore, the modulation of the ATF6-mediated transcription may provide a therapeutic strategy for these and other diseases in which ATF6 activity is implicated.

### RELEVANT BACKGROUND ART

WO 2019/195810 relates to compounds as inhibitors of ATF6. The compounds may find use as therapeutic agents for the treatment of diseases or disorders mediated by ATF6 and may find particular use in the treatment of viral infections, neurodegenerative diseases, vascular diseases, or cancer.

WO 2019/209962 relates to compounds useful in the treatment of neurological disorders. The compounds of the invention, alone or in combination with other pharmaceutically active agents, can be used for treating or preventing neurological disorders.

Gallagher et al. eLife 2016; 5:E11878. DOI: 10.7554/eLife.11878 relates to cell-based screens to discover and develop Ceapins, a class of pyrazole amides, that block ATF6α signaling in response to ER stress.

### BRIEF SUMMARY

In one aspect, provided is a compound of the formula (I): 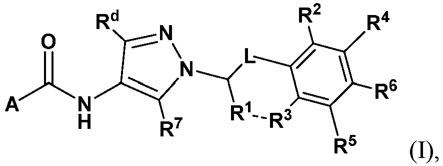 or a stereoisomer thereof, or a pharmaceutically acceptable salt thereof, wherein A, L, R¹, R², R³, R⁴, R⁵, R⁶, R⁷, R^{d}, and --, are as detailed herein.

In another aspect, provided is a compound of formula (I) or a stereoisomer thereof, or a pharmaceutically acceptable salt thereof or a pharmaceutical composition comprising the compound for use in a method of treating a disease or disorder mediated by activating transcription factor 6 (ATF6) in an individual in need thereof, wherein the method comprises administering to the individual an effective amount of a compound of formula (I) or a stereoisomer thereof, or a pharmaceutically acceptable salt thereof or a pharmaceutical composition comprising the compound. In some embodiments, the disease or disorder mediated by activating transcription factor 6 (ATF6) is viral infection, cancer, a neurodegenerative disease, or a vascular disease. In certain embodiments, the disease or disorder is viral infection, hereditary cerebellar atrophy and ataxia, Alzheimer's disease, type 2 diabetes mellitus, diabetic nephropathy, myocardial atrophy, heart failure, atherosclerosis, ischemia, ischemic heart disease, or cerebral ischemia. In some embodiments, the disease or disorder characterized by activating transcription factor 6 (ATF6) is cancer. In some embodiments, ATF6 is ATF6α.

In another aspect, provided is compound of formula (I) or a stereoisomer thereof, or a pharmaceutically acceptable salt thereof or a pharmaceutical composition comprising the compound for use in a method of treating a disease or disorder characterized by activating transcription factor 6 (ATF6) in an individual in need thereof, wherein the method comprises administering to the individual an effective amount of a compound of formula (I) or a stereoisomer thereof, or a pharmaceutically acceptable salt thereof or a pharmaceutical composition comprising the compound. In some embodiments, the disease or disorder characterized by activation of ATF6 is viral infection, cancer, a neurodegenerative disease, or a vascular disease. In certain embodiments, the disease or disorder is viral infection, hereditary cerebellar atrophy and ataxia, Alzheimer's disease, type 2 diabetes mellitus, diabetic nephropathy, myocardial atrophy, heart failure, atherosclerosis, ischemia, ischemic heart disease, or cerebral ischemia. In some embodiments, the disease or disorder characterized by activating transcription factor 6 (ATF6) is cancer. In some embodiments, ATF6 is ATF6α.

In another aspect, provided is a compound of formula (I) or a stereoisomer thereof, or a pharmaceutically acceptable salt thereof or a pharmaceutical composition comprising the compound for use in a method of treating cancer in an individual in need thereof, comprising administering to the individual a therapeutically effective amount of a compound of formula (I) or a stereoisomer thereof, or a pharmaceutically acceptable salt thereof or a pharmaceutical composition comprising the compound.

In some embodiments, the cancer is breast cancer, colorectal cancer, ovarian cancer, prostate cancer, pancreatic cancer, kidney cancer, lung cancer, melanoma, fibrosarcoma, bone sarcoma, connective tissue sarcoma, renal cell carcinoma, giant cell carcinoma, squamous cell carcinoma, leukemia, skin cancer, soft tissue cancer, liver cancer, gastrointestinal carcinoma, or adenocarcinoma. In some embodiments, one or more cancer cells in the individual are dormant cancer cells.

In some embodiments, the individual has had a prior treatment. In some embodiments, the cancer is resistant or refractory to the prior treatment. In some embodiments, the cancer is resistant to treatment with a ubiquitin-proteasome pathway inhibitor, a taxane, a Cox-2 inhibitor, a platinum-based antineoplastic drug, an anthracycline, a pyrimidine analog, a topoisomerase inhibitor, an mTOR inhibitor, an immune-check point inhibitor, or an agent that is used in immune oncology.

In some embodiments, the method further comprises administering radiation. In some embodiments, the method further comprises administering a second anticancer agent. In some embodiments, the second anticancer agent targets an immune checkpoint protein.

In another aspect, provided is a compound of formula (I) or a stereoisomer thereof, or a pharmaceutically acceptable salt thereof or a pharmaceutical composition comprising the compound for use in a method of treating a disease or disorder associated with angiogenesis in an individual in need thereof, wherein the method comprises administering to the individual an effective amount of a compound of formula (I) or a stereoisomer thereof, or a pharmaceutically acceptable salt thereof or a pharmaceutical composition comprising the compound. In some embodiments, the method further comprises administering a second anti-angiogenesis agent.

In some embodiments of the methods disclosed herein, the method further comprises administering a second agent that modulates the Unfolded Protein Response or the Integrated Stress Response. In some embodiments, the second agent inhibits the IRE1/XBP1 pathway.

In another aspect, provided is a compound of formula (I) or a stereoisomer thereof, or a pharmaceutically acceptable salt thereof or a pharmaceutical composition comprising the compound for use in a method of modulating (activating or inhibiting) ATF6 in an individual comprising administering to the individual a compound of formula (I) or a stereoisomer thereof, or a pharmaceutically acceptable salt thereof or a pharmaceutical composition comprising the compound.

In another aspect, provided is a compound of formula (I) or a stereoisomer thereof, or a pharmaceutically acceptable salt thereof or a pharmaceutical composition comprising the compound for use in a method of modulating (activating or inhibiting) ATF6 in a cell comprising delivering to the cell a compound of formula (I) or a stereoisomer thereof or a pharmaceutically acceptable salt thereof or a pharmaceutical composition comprising the compound.

Also provided are pharmaceutical compositions comprising: (A) a compound detailed herein, such as a compound of formula (I) or a stereoisomer thereof, or a pharmaceutically acceptable salt thereof, or a compound of formula (I) or a stereoisomer thereof, or a pharmaceutically acceptable salt thereof; and (B) a pharmaceutically acceptable carrier or excipient. Kits comprising a compound detailed herein or a stereoisomer thereof, or a pharmaceutically acceptable salt thereof and optionally instructions for use are also provided.

### DETAILED DESCRIPTION

### Definitions

Reference to "about" a value or parameter herein includes (and describes) embodiments that are directed to that value or parameter per se. For example, description referring to "about X" includes description of "X".

"Alkyl" as used herein refers to and includes, unless otherwise stated, a saturated linear (*i.e.,* unbranched) or branched univalent hydrocarbon chain or combination thereof, having the number of carbon atoms designated (*i.e.,* C₁-C₁₀ means one to ten carbon atoms). Particular alkyl groups are those having 1 to 20 carbon atoms (a "C₁-C₂₀ alkyl"), having 1 to 10 carbon atoms (a "C₁-C₁₀ alkyl"), having 6 to 10 carbon atoms (a "C₆-C₁₀ alkyl"), having 1 to 6 carbon atoms (a "C₁-C₆ alkyl"), having 2 to 6 carbon atoms (a "C₂-C₆ alkyl"), or having 1 to 4 carbon atoms (a "C₁-C₄ alkyl"). Examples of alkyl groups include, but are not limited to, groups such as methyl, ethyl, n-propyl, isopropyl, n-butyl, t-butyl, isobutyl, sec-butyl, n-pentyl, n-hexyl, n-heptyl, n-octyl, n-nonyl, n-decyl, and the like.

"Alkoxy" refers to the group R-O-, where R is alkyl; and includes, by way of example, methoxy, ethoxy, n-propoxy, iso-propoxy, n-butoxy, tert-butoxy, sec-butoxy, n-pentoxy, n-hexyloxy, 1,2-dimethylbutoxy, and the like.

"Aryl" or "Ar" as used herein refers to an unsaturated aromatic carbocyclic group having a single ring (*e.g.,* phenyl) or multiple condensed rings (*e.g.,* naphthyl or anthryl) which condensed rings may or may not be aromatic. Particular aryl groups are those having from 6 to 14 annular carbon atoms (a "C₆-C₁₄ aryl"). An aryl group having more than one ring where at least one ring is non-aromatic may be connected to the parent structure at either an aromatic ring position or at a non-aromatic ring position. In one variation, an aryl group having more than one ring where at least one ring is non-aromatic is connected to the parent structure at an aromatic ring position.

"Cycloalkyl" as used herein refers to and includes, unless otherwise stated, saturated cyclic univalent hydrocarbon structures, having the number of carbon atoms designated (*i.e.,* C₃-C₁₀ means three to ten carbon atoms). Cycloalkyl can consist of one ring, such as cyclohexyl, or multiple rings, such as adamantyl. A cycloalkyl comprising more than one ring may be fused, spiro or bridged, or combinations thereof. Particular cycloalkyl groups are those having from 3 to 12 annular carbon atoms. A preferred cycloalkyl is a cyclic hydrocarbon having from 3 to 8 annular carbon atoms (a "C₃-C₈ cycloalkyl"), having 3 to 6 carbon atoms (a "C₃-C₆ cycloalkyl"), or having from 3 to 4 annular carbon atoms (a "C₃-C₄ cycloalkyl"). Examples of cycloalkyl include, but are not limited to, cyclopropyl, cyclobutyl, cyclopentyl, cyclohexyl, cycloheptyl, norbornyl, and the like.

"Halo" or "halogen" refers to elements of the Group 17 series having atomic number 9 to 85. Preferred halo groups include the radicals of fluorine, chlorine, bromine and iodine. Where a residue is substituted with more than one halogen, it may be referred to by using a prefix corresponding to the number of halogen moieties attached, *e.g.,* dihaloaryl, dihaloalkyl, trihaloaryl etc. refer to aryl and alkyl substituted with two ("di") or three ("tri") halo groups, which may be but are not necessarily the same halogen; thus 4-chloro-3-fluorophenyl is within the scope of dihaloaryl. An alkyl group in which each hydrogen is replaced with a halo group is referred to as a "perhaloalkyl." A preferred perhaloalkyl group is trifluoromethyl (-CF₃). Similarly, "perhaloalkoxy" refers to an alkoxy group in which a halogen takes the place of each H in the hydrocarbon making up the alkyl moiety of the alkoxy group. An example of a perhaloalkoxy group is trifluoromethoxy (-OCF₃).

The term "haloalkyl" refers to an alkyl group with one or more halo substituents, or one, two, or three halo substituents. Examples of haloalkyl groups include -CF₃, -(CH₂)F, -CHF₂, -CH₂Br, -CH₂CF₃, and -CH₂CH₂F.

"Heteroaryl" as used herein refers to an unsaturated aromatic cyclic group having from 1 to 14 annular carbon atoms and at least one annular heteroatom, including but not limited to heteroatoms such as nitrogen, oxygen and sulfur. A heteroaryl group may have a single ring (*e.g.,* pyridyl, furyl) or multiple condensed rings (*e.g.,* indolizinyl, benzothienyl) which condensed rings may or may not be aromatic. Particular heteroaryl groups are 5 to 14-membered rings having 1 to 12 annular carbon atoms and 1 to 6 annular heteroatoms independently selected from nitrogen, oxygen and sulfur, 5 to 10-membered rings having 1 to 8 annular carbon atoms and 1 to 4 annular heteroatoms independently selected from nitrogen, oxygen and sulfur, or 5, 6 or 7-membered rings having 1 to 5 annular carbon atoms and 1 to 4 annular heteroatoms independently selected from nitrogen, oxygen and sulfur. In one variation, particular heteroaryl groups are monocyclic aromatic 5-, 6- or 7-membered rings having from 1 to 6 annular carbon atoms and 1 to 4 annular heteroatoms independently selected from nitrogen, oxygen and sulfur. In another variation, particular heteroaryl groups are polycyclic aromatic rings having from 1 to 12 annular carbon atoms and 1 to 6 annular heteroatoms independently selected from nitrogen, oxygen and sulfur. A heteroaryl group having more than one ring where at least one ring is non-aromatic may be connected to the parent structure at either an aromatic ring position or at a non-aromatic ring position. In one variation, a heteroaryl group having more than one ring where at least one ring is non-aromatic is connected to the parent structure at an aromatic ring position. A heteroaryl group may be connected to the parent structure at a ring carbon atom or a ring heteroatom.

"Heterocycle", "heterocyclic", or "heterocyclyl" as used herein refers to a saturated or an unsaturated non-aromatic cyclic group having a single ring or multiple condensed rings, and having from 1 to 14 annular carbon atoms and from 1 to 6 annular heteroatoms, such as nitrogen, sulfur or oxygen, and the like. A heterocycle comprising more than one ring may be fused, bridged or spiro, or any combination thereof, but excludes heteroaryl groups. The heterocyclyl group may be optionally substituted independently with one or more substituents described herein. Particular heterocyclyl groups are 3 to 14-membered rings having 1 to 13 annular carbon atoms and 1 to 6 annular heteroatoms independently selected from nitrogen, oxygen and sulfur, 3 to 12-membered rings having 1 to 11 annular carbon atoms and 1 to 6 annular heteroatoms independently selected from nitrogen, oxygen and sulfur, 3 to 10-membered rings having 1 to 9 annular carbon atoms and 1 to 4 annular heteroatoms independently selected from nitrogen, oxygen and sulfur, 3 to 8-membered rings having 1 to 7 annular carbon atoms and 1 to 4 annular heteroatoms independently selected from nitrogen, oxygen and sulfur, or 3 to 6-membered rings having 1 to 5 annular carbon atoms and 1 to 4 annular heteroatoms independently selected from nitrogen, oxygen and sulfur. In one variation, heterocyclyl includes monocyclic 3-, 4-, 5-, 6- or 7-membered rings having from 1 to 2, 1 to 3, 1 to 4, 1 to 5, or 1 to 6 annular carbon atoms and 1 to 2, 1 to 3, or 1 to 4 annular heteroatoms independently selected from nitrogen, oxygen and sulfur. In another variation, heterocyclyl includes polycyclic non-aromatic rings having from 1 to 12 annular carbon atoms and 1 to 6 annular heteroatoms independently selected from nitrogen, oxygen and sulfur.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" or "treating" is an approach for obtaining beneficial or desired results including clinical results. For example, beneficial or desired results include, but are not limited to, one or more of the following: decreasing symptoms resulting from the disease, increasing the quality of life of those suffering from the disease, decreasing the dose of other medications required to treat the disease, delaying the progression of the disease, and/or prolonging survival of an individual. In reference to cancers or other unwanted cell proliferation, beneficial or desired results include shrinking a tumor (reducing tumor size); decreasing the growth rate of the tumor (such as to suppress tumor growth); reducing the number of cancer cells; inhibiting, retarding or slowing to some extent and preferably stopping cancer cell infiltration into peripheral organs; inhibiting (slowing to some extent and preferably stopping) tumor metastasis; inhibiting tumor growth; preventing or delaying occurrence and/or recurrence of tumor; and/or relieving to some extent one or more of the symptoms associated with the cancer. In some embodiments, beneficial or desired results include preventing or delaying recurrence, such as of unwanted cell proliferation.

As used herein, an "effective dosage" or "effective amount" of compound stereoisomer thereof, or a pharmaceutically acceptable salt thereof or pharmaceutical composition is an amount sufficient to effect beneficial or desired results. For prophylactic use, beneficial or desired results include results such as eliminating or reducing the risk, lessening the severity of, or delaying the onset of the disease, including biochemical, histological and/or behavioral symptoms of the disease, its complications and intermediate pathological phenotypes presenting during development of the disease. For therapeutic use, beneficial or desired results include ameliorating, palliating, lessening, delaying or decreasing one or more symptoms resulting from the disease, increasing the quality of life of those suffering from the disease, decreasing the dose of other medications required to treat the disease, enhancing effect of another medication such as via targeting, delaying the progression of the disease, and/or prolonging survival. In reference to cancers or other unwanted cell proliferation, an effective amount comprises an amount sufficient to cause a tumor to shrink and/or to decrease the growth rate of the tumor (such as to suppress tumor growth) or to prevent or delay other unwanted cell proliferation. In some embodiments, an effective amount is an amount sufficient to delay development. In some embodiments, an effective amount is an amount sufficient to prevent or delay recurrence. An effective amount can be administered in one or more administrations, in the case of cancer, the effective amount of the drug or composition may: (i) reduce the number of cancer cells; (ii) reduce tumor size; (iii) inhibit, retard, slow to some extent and preferably stop cancer cell infiltration into peripheral organs; (iv) inhibit (*i.e.,* slow to some extent and preferably stop) tumor metastasis; (v) inhibit tumor growth; (vi) prevent or delay recurrence of tumor; and/or (vii) relieve to some extent one or more of the symptoms associated with the cancer. An effective dosage can be administered in one or more administrations. For purposes of this disclosure, an effective dosage of compound or a stereoisomer thereof, or a pharmaceutically acceptable salt thereof, or pharmaceutical composition is an amount sufficient to accomplish prophylactic or therapeutic treatment either directly or indirectly. It is intended and understood that an effective dosage of a compound or a stereoisomer thereof, or a pharmaceutically acceptable salt thereof, or pharmaceutical composition may or may not be achieved in conjunction with another drug, compound, or pharmaceutical composition. Thus, an "effective dosage" may be considered in the context of administering one or more therapeutic agents, and a single agent may be considered to be given in an effective amount if, in conjunction with one or more other agents, a desirable result may be or is achieved.

As used herein, the term "individual" is a mammal, including humans. An individual includes, but is not limited to, human, bovine, horse, feline, canine, rodent, or primate. In some embodiments, the individual is human. The individual (such as a human) may have advanced disease or lesser extent of disease, such as low tumor burden. In some embodiments, the individual is at an early stage of a proliferative disease (such as cancer). In some embodiments, the individual is at an advanced stage of a proliferative disease (such as an advanced cancer).

It is understood that aspects and variations described herein also include "consisting" and/or "consisting essentially of" aspects and variations.

### Compounds

In one aspect, a compound of formula (I): 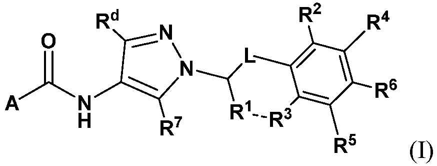
or a stereoisomer thereof, or a pharmaceutically acceptable salt of any of the foregoing,
wherein:
   R^{d} is H or C₁-C₆alkyl;
   R¹ is C₁-C₆alkyl, C₃-C₈cycloalkyl, or C₁-C₆ haloalkyl;
   L is -CH₂- or is absent;
   -- is a bond or is absent;
   wherein: 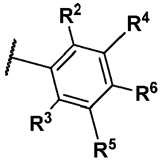 is 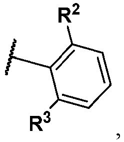
   wherein R² and R³ are each halo,
   A is 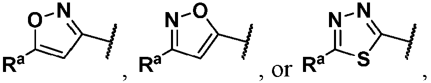
   R^{a} is 5- or 6-membered heteroaryl, wherein the 5- or 6-membered heteroaryl is unsubstituted or substituted with one to four groups selected from the group consisting of OH, halo, C₁-C₆alkyl and C₁-C₆alkoxy, wherein when A is 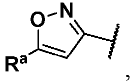 R^{a} is selected from the group consisting of 2-furyl, 2-pyridinyl, 2-pyrimidinyl, 4-pyrimidinyl, and 2-pyrazinyl, each of which is unsubstituted or substituted with one to four groups selected from the group consisting of OH, halo, C₁-C₆alkyl and C₁-C₆alkoxy;
      provided that, when A is 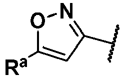 and R^{a} is 2-furyl, at least one of (*i*.)-(*ii*.) applies:
      (*i*.) L is absent, and R¹ is C₁-C₆alkyl;
      (*ii*.) R² and R³ are each Cl; and
   R⁷ is H, C₁-C₆alkyl or C₁-C₆haloalkyl,
      provided that, when R^{d} is C₁-C₆alkyl, R⁷ is H, and, when R⁷ is C₁-C₆alkyl, R^{d} is H.

In one variation is provided a compound of the formula (I), or a pharmaceutically acceptable salt thereof, wherein the carbon bearing R¹ (*i.e.,* C₁-C₆alkyl, C₃-C₈cycloalkyl, or C₁-C₆haloalkyl) is in the "*S*" configuration. In another variation is provided a compound of the formula (I), or a pharmaceutically acceptable salt thereof, wherein the carbon bearing R¹ (*i.e.,* C₁-C₆alkyl, C₃-C₈cycloalkyl, or C₁-C₆haloalkyl) is in the *"R"* configuration. Mixtures of a compound of the formula (I) are also embraced, including racemic or non-racemic mixtures of a given compound, and mixtures of two or more compounds of different chemical formulae.

In the descriptions herein, it is understood that every description, variation, embodiment, or aspect of a moiety may be combined with every description, variation, embodiment, or aspect of other moieties the same as if each and every combination of descriptions is specifically and individually listed. For example, every description, variation, embodiment, or aspect provided herein with respect to R¹ of formula (I) may be combined with every description, variation, embodiment or aspect of A of formula (I) the same as if each and every combination were specifically and individually listed.

In some embodiments, provided is a compound of the formula (I), wherein A is 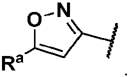 In some embodiments, provided is a compound of the formula (I) wherein A is 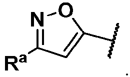 In other embodiments, provided is a compound of the formula (I) wherein A is

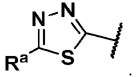

In some embodiments of formula (I), R¹ is C₁-C₆alkyl. In other embodiments, R¹ is C₃-C₈cycloalkyl. In some embodiments, R¹ is C₁-C₆haloalkyl. In certain embodiments, R¹ is methyl, ethyl, n-propyl, isopropyl, n-butyl, isobutyl, sec-butyl, or tert-butyl. In some embodiments, R¹ is methyl. In some embodiments, R¹ is ethyl. In some embodiments, R¹ is cyclopropyl, cyclobutyl, cyclopentyl, or cyclohexyl. In some embodiments, R¹ is cyclopropyl.

In some embodiments of a compound of formula (I), L is absent. In other embodiments, L is -CH₂-. In some embodiments, L is absent, and R¹ is C₁-C₆alkyl. In other embodiments, L is absent, and R¹ is C₃-C₈cycloalkyl. In some embodiments, L is absent, and R¹ is C₁-C₆haloalkyl. In certain embodiments, L is absent, and R¹ is methyl, ethyl, n-propyl, isopropyl, n-butyl, isobutyl, sec-butyl, or tert-butyl. In some embodiments, L is absent, and R¹ is methyl. In some embodiments, L is absent, and R¹ is ethyl. In some embodiments, L is absent, and R¹ is cyclopropyl, cyclobutyl, cyclopentyl, or cyclohexyl. In some embodiments, L is absent, and R¹ is cyclopropyl.

In some embodiments, L is -CH₂-, and R¹ is C₁-C₆alkyl. In other embodiments, L is -CH₂-, and R¹ is C₃-C₈cycloalkyl. In some embodiments, L is -CH₂-, and R¹ is C₁-C₆haloalkyl. In certain embodiments, L is -CH₂-, and R¹ is methyl, ethyl, n-propyl, isopropyl, n-butyl, isobutyl, sec-butyl, or tert-butyl. In some embodiments, L is -CH₂-, and R¹ methyl. In some embodiments, L is -CH₂-, and R¹ ethyl. In some embodiments, L is -CH₂-, and R¹ is cyclopropyl, cyclobutyl, cyclopentyl, or cyclohexyl. In some embodiments, L is -CH₂-, and R¹ is cyclopropyl.

In some embodiments of formula (I), A is 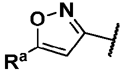 and R^{a} is 2-furyl. In another embodiment of formula (I), A is 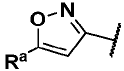 and R^{a} is 2-pyridinyl. In some embodiments of formula (I), A is 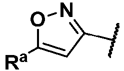 and R^{a} is 2-pyrimidinyl. In other embodiments of formula (I), A is 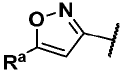 and R^{a} is 4-pyrimidinyl. In still other embodiments of formula (I), A is 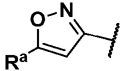 and R^{a} is 2-pyrazinyl.

In some embodiments of formula (I), A is 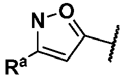 and R^{a} is 2-furyl. In another embodiment of formula (I), A is 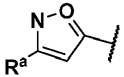 and R^{a} is 2-pyridinyl. In some embodiments of formula (I), A is 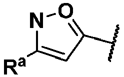 and R^{a} is 2-pyrimidinyl. In other embodiments of formula (I), A is 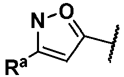 and R^{a} is 4-pyrimidinyl. In still other embodiments of formula (I), A is 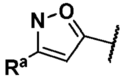 and R^{a} is 2-pyrazinyl.

In other embodiments of formula (I), A is 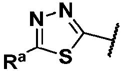 and R^{a} is 2-furyl. In another embodiment of formula (I), A is 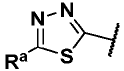 and R^{a} is 2-pyridinyl. In some embodiments of formula (I), A is 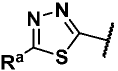 and R^{a} is 2-pyrimidinyl. In other embodiments of formula (I), A is 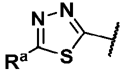 and R^{a} is 4-pyrimidinyl. In still other embodiments of formula (I), A is 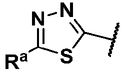 and R^{a} is 2-pyrazinyl.

In some embodiments of formula (I), wherein A is 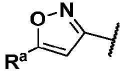 and R^{a} is 2-furyl, L is absent, and R¹ is C₁-C₆alkyl.

In certain embodiments of formula (I), when R^{d} is C₁-C₆alkyl, R⁷ is H. In other embodiments of formula (I), when R⁷ is C₁-C₆alkyl, R^{d} is H.

In some embodiments, R² and R³ are each Cl. In other embodiments, R² and R³ are each F. In some embodiments, one of R² and R³ is F and one of R² and R³ is Cl.

In all embodiments, 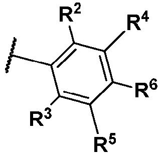 is 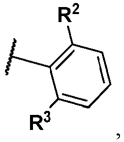 wherein R² and R³ are each halo. , In some embodiments, 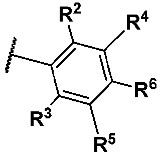 is 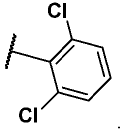 In other embodiments, 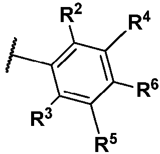 is 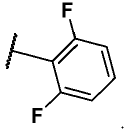 In still other embodiments, 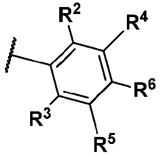 is 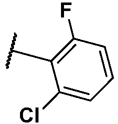 or

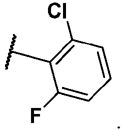

In some embodiments of a compound of formula (I), R^{a} is 5- or 6-membered heteroaryl, wherein the 5- or 6-membered heteroaryl is unsubstituted or substituted with one to four groups selected from OH, halo, and C₁-C₆alkyl. In some embodiments, R^{a} is pyrrolyl, pyrazolyl, imidazolyl, triazolyl, tetrazolyl, furanyl, isoxazolyl, oxazolyl, oxadiazolyl, thiophenyl, isothiazolyl, thiazolyl, thiadiazolyl, pyridyl, pyridazinyl, pyrimidinyl, pyrazinyl, triazinyl, or tetrazinyl, each of which is unsubstituted or is substituted with one to four groups selected from the group consisting of OH, halo, C₁-C₆alkyl, and C₁-C₆alkoxy. In some embodiments, R^{a} is selected from the group consisting of 2-furyl, 2-pyridinyl, 2-pyrimidinyl, 4-pyrimidinyl, and 2-pyrazinyl, each of which is unsubstituted or is substituted with one to four groups selected from the group consisting of OH, halo, C₁-C₆alkyl, and C₁-C₆alkoxy.

In some embodiments, R^{a} is a 5- or 6-membered heteroaryl containing one heteroatom.

In some embodiments, A is 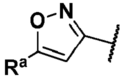 and R^{a} is 2-furyl, 2-pyridinyl, 2-pyrimidinyl, 4-pyrimidinyl, and 2-pyrazinyl, each unsubstituted or substituted with one to four groups selected from the group consisting of OH, halo, and C₁-C₆alkyl. In some embodiments, A is 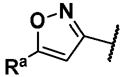 and R^{a} is 2-furyl.

In some embodiments of a compound of formula (I), A is 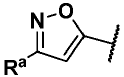 and R^{a} is 5- or 6-membered heteroaryl, wherein the 5- or 6-membered heteroaryl of R^{a} is unsubstituted or is substituted with one to four groups selected from the group consisting of OH, halo, and C₁-C₆alkyl. In some embodiments, A is 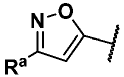 and R^{a} is 2-furyl, 2-pyridinyl, 2-pyrimidinyl, 4-pyrimidinyl, and 2-pyrazinyl, each unsubstituted or substituted with one to four groups selected from the group consisting of OH, halo, and C₁-C₆alkyl. In some embodiments, A is 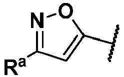 and 2-pyrazinyl.

In some embodiments of a compound of formula (I), A is 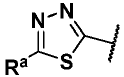 and R^{a} is 5- or 6-membered heteroaryl, wherein the 5- or 6-membered heteroaryl of R^{a} is unsubstituted or is substituted with one to four groups selected from the group consisting of OH, halo, and C₁-C₆alkyl. In some embodiments, A is 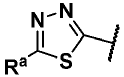 and R^{a} is 2-furyl, 2-pyridinyl, 2-pyrimidinyl, 4-pyrimidinyl, and 2-pyrazinyl, each unsubstituted or substituted with one to four groups selected from the group consisting of OH, halo, and C₁-C₆alkyl. In some embodiments, A is 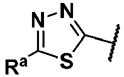 and R^{a} is 2-furyl.

In some embodiments of any of the formulae provided herein, R^{d} and R⁷ are H, A is 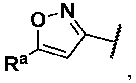 R^{a} is 2-furyl, R¹ is methyl, and L is absent. In some embodiments of any of the formulae provided herein, R^{d} is methyl, R⁷ is H, A is 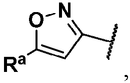 R^{a} is 2-furyl, R¹ is methyl, and L is absent. In some embodiments of any of the formulae provided herein, R^{d} is H, R⁷ is methyl, A is 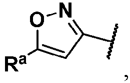 2-furyl, R¹ is methyl, and L is absent.

In some embodiments of any of the formulae provided herein, R^{d} and R⁷ are H, A is 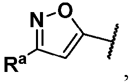 R^{a} is 2-furyl, R¹ is methyl, and L is absent. In some embodiments of any of the formulae provided herein, R^{d} is methyl, R⁷ is H, A is 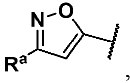 R^{a} is 2-furyl, R¹ is methyl, and L is absent. In some embodiments of any of the formulae provided herein, R^{d} is H, R⁷ is methyl, A is 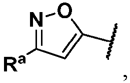 R^{a} is 2-furyl, R¹ is methyl, and L is absent. In some embodiments of any of the formulae provided herein, R^{d} and R⁷ are H, A is 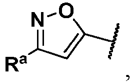 R^{a} is 2-pyrazinyl, R¹ is methyl, and L is absent.

In some embodiments of any of the formulae provided herein, R^{d} and R⁷ are H, A is 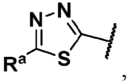 R^{a} is 2-furyl, R¹ is methyl, and L is absent. In some embodiments of any of the formulae provided herein, R^{d} is methyl, R⁷ is H, A is 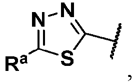 R^{a} is 2-furyl, R¹ is methyl, and L is absent. In some embodiments of any of the formulae provided herein, R^{d} is H, R⁷ is methyl, A is 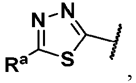 2-furyl, R¹ is methyl, and L is absent.

In all embodiments described herein, the compounds of formula (I) are not any of the following compounds:
*N*-(1-(1-(2,4-bis(trifluoromethyl)phenyl)propyl)-1H-pyrazol-4-yl)-5-(pyridin-2-yl)isoxazole-3-carboxamide;
*N*-(1-(2,4-bis(trifluoromethyl)benzyl)-1H-pyrazol-4-yl)-5-(2-hydroxypropan-2-yl)isoxazole-3-carboxamide;
5-acetyl-*N*-(1-(2,4-bis(trifluoromethyl)benzyl)-1H-pyrazol-4-yl)isoxazole-3-carboxamide;
*N*-(1-(2,4-bis(trifluoromethyl)benzyl)-1H-pyrazol-4-yl)-3-(3-fluorophenyl)-1,2,4-oxadiazole-5-carboxamide;
*N*-(1-(1-(2,4-bis(trifluoromethyl)phenyl)ethyl)-1H-pyrazol-4-yl)-5-(pyridin-2-yl) isoxazole-3-carboxamide;
*N*-(1-(1-(2,4-bis(trifluoromethyl)phenyl)ethyl)-1H-pyrazol-4-yl)-5-(furan-2-yl)isoxazole-3-carboxamide;
*N*-(1-(1-(2,4-bis(trifluoromethyl)phenyl)ethyl)-1H-pyrazol-4-yl)-5-(furan-2-yl)isoxazole-3-carboxamide;
*N*-(1-(2,4-bis(trifluoromethyl)benzyl)-1H-pyrazol-4-yl)-5-(pyridin-4-yl)isoxazole-3-carboxamide;
*N*-(1-(2,4-bis(trifluoromethyl)benzyl)-1H-pyrazol-4-yl)-5-(pyridin-2-yl)isoxazole-3-carboxamide;
*N*-(1-{1-[2,4-bis(trifluoromethyl)phenyl]ethyl}-1H-pyrazol-4-yl)-5-(furan-2-yl) isoxazole-3-carboxamide;
*N*-{1-[2,4-bis(trifluoromethyl)benzyl]-1H-pyrazol-4-yl}-5-tert-butyl-1,2-oxazole-3-carboxamide;
*N*-(1-(2,4-bis(trifluoromethyl)benzyl)-1H-pyrazol-4-yl)-5-(thiophen-2-yl)isoxazole -3-carboxamide;
*N*-(1-(2,4-bis(trifluoromethyl)benzyl)-1H-pyrazol-4-yl)-[3,3'-bipyridine]-5-carboxamide;
*N*-(1-(2,4-bis(trifluoromethyl)benzyl)-1H-pyrazol-4-yl)-5-(2,4-difluorophenyl)isoxazole-3-carboxamide;
*N*-(1-(2,4-bis(trifluoromethyl)benzyl)-1H-imidazol-4-yl)-5-(furan-2-yl)isoxazole-3-carboxamide;
*N*-(1-(2, 3-dihydro-1H-inden-2-yl)-1H-pyrazol-4-yl)-5-(furan-2-yl) isoxazole-3-carboxamide;
*N*-(1-(2, 3-dihydro-1H-inden-1-yl)-1H-pyrazol-4-yl)-5-(furan-2-yl) isoxazole-3-carboxamide;
*N*-(1-(2,4-bis(trifluoromethyl)benzyl)-1H-pyrazol-4-yl)-5-(4 fluorophenyl)nicotinamide;
*N*-(1-(2,4-bis(trifluoromethyl)benzyl)-1H-pyrazol-4-yl)-5-phenylnicotinamide;
*N*-(1-(2,4-bis(trifluoromethyl)benzyl)-1H-pyrazol-4-yl)-5-(5-methylfuran-2-yl)isoxazole-3-carboxamide;
*N*-(1-(2,4-bis(trifluoromethyl)benzyl)-1H-pyrazol-4-yl)isoxazole-3-carboxamide;
*N*-(1-(2, 6-dichlorobenzyl)-1H-pyrazol-4-yl)-5-(furan-2-yl) isoxazole-3-carboxamide;
*N*-(1-(4-cyanobenzyl)-1H-pyrazol-4-yl)-5-(furan-2-yl)isoxazole-3-carboxamide;
*N*-(1-(4-cyano-3-(trifluoromethyl)benzyl)-1H-pyrazol-4-yl)-5-(furan-2-yl)isoxazole-3-carboxamide;
*N*-(1-(2,4-bis(trifluoromethyl)phenyl)-1H-pyrazol-4-yl)-5-(furan-2-yl)isoxazole-3-carboxamide;
*N*-(1-(3,5-bis(trifluoromethyl)benzyl)-1H-pyrazole-4-yl)-5-(furan-2-yl)isoxazole-3-carboxamide;
*N*-(1-(2,4-bis(trifluoromethyl)benzyl)-3-methyl-1H-pyrazol-4-yl)-5-(pyridin-2-yl)isoxazole-3-carboxamide;
*N*-(1-(2,4-bis(trifluoromethyl)benzyl)-5-methyl-1H-pyrazol-4-yl)-5-(pyridin-2-yl)isoxazole-3-carboxamide;
*N*-(1-(2,4-bis(trifluoromethyl)benzyl)-1H-pyrazol-4-yl)-5-(3-chloropyridin-2-yl)isoxazole-3-carboxamide;
*N*-(1-(1-(2,4-bis(trifluoromethyl)phenyl)ethyl)-1H-pyrazol-4-yl)-5-(3-chloropyridin-2-yl)isoxazole-3-carboxamide;
*N*-(1-((2,4-bis(trifluoromethyl)phenyl)(cyclopropyl)methyl)-1H-pyrazol-4-yl)-5-(pyridin-2-yl)isoxazole-3-carboxamide;
*N*-(1-(1-(2,4-bis(trifluoromethyl)phenyl)ethyl)-1H-pyrazol-4-yl)-5-(3-chloropyridin-2-yl)isoxazole-3-carboxamide;
*N*-(1-(1-(2,4-bis(trifluoromethyl)phenyl)ethyl)-1H-pyrazol-4-yl)-5-(pyridin-3-yl)isoxazole-3-carboxamide;
*N*-(1-(2,4-bis(trifluoromethyl)benzyl)-5-methyl-1H-pyrazol-4-yl)-5-(furan-2-yl)isoxazole-3-carboxamide;
*N-(1-(1-(2,4-bis(trifluoromethyl)phenyl)ethyl)-5-methyl-1H-pyrazol-4-yl)-5-(furan-2-*yl)isoxazole-3-carboxamide;
*N*-(1-(2,4-bis(trifluoromethyl)phenyl)-5-methyl-1H-pyrazol-4-yl)-5-(furan-2-yl)isoxazole-3-carboxamide;
*N*-(1-(2,4-bis(trifluoromethyl)benzyl)-3-methyl-1H-pyrazol-4-yl)-5-(furan-2-yl)isoxazole-3-carboxamide;
*N*-(1-(1-(2,4-bis(trifluoromethyl)phenyl)ethyl)-3-methyl-1H-pyrazol-4-yl)-5-(furan-2-yl)isoxazole-3-carboxamide;
*N*-(1-(2,4-bis(trifluoromethyl)benzyl)-1H-pyrazol-4-yl)-5-(furan-2-yl)nicotinamide;
*N*-(1-(1-(2,4-bis(trifluoromethyl)phenyl)ethyl)-1H-pyrazol-4-yl)-5-(furan-2-yl)nicotinamide;
*N*-(1-(2,4-bis(trifluoromethyl)phenyl)-1H-pyrazol-4-yl)-5-(furan-2-yl)nicotinamide;
*N*-(1-(2,4-bis(trifluoromethyl)phenyl)-5-methyl-1H-pyrazol-4-yl)-5-(furan-2-yl)nicotinamide;
*N*-(1-(2,4-bis(trifluoromethyl)benzyl)-3-methyl-1H-pyrazol-4-yl)-5-(furan-2-yl)nicotinamide;
*N*-(1-(2,4-bis(trifluoromethyl)phenyl)-3-methyl-1H-pyrazol-4-yl)-5-(furan-2-yl)isoxazole-3-carboxamide;
*N*-(1-(2,4-bis(trifluoromethyl)benzyl)-5-methyl-1H-pyrazol-4-yl)-5-(furan-2-yl)nicotinamide;
*N-(1-(1-(2,4-bis(trifluoromethyl)phenyl)ethyl)-5-methyl-1H-pyrazol-4-yl)-5-(furan-2-*yl)nicotinamide;
*N*-(1-(1-(2,4-bis(trifluoromethyl)phenyl)ethyl)-3-methyl-1H-pyrazol-4-yl)-5-(furan-2-yl)nicotinamide;
*N*-(1-(2,4-bis(trifluoromethyl)phenyl)-3-methyl-1H-pyrazol-4-yl)-5-(furan-2-ylnicotinamide;
*N*-(1-(1-(2,4-bis(trifluoromethyl)phenyl)ethyl)-1H-pyrazol-4-yl)-3-(furan-2-yl)acrylamide;
*N*-(1-(2,4-bis(trifluoromethyl)phenyl)-1H-pyrazol-4-yl)-3-(furan-2-yl)acrylamide;
*N*-(1-(2,4-bis(trifluoromethyl)benzyl)-5-methyl-1H-pyrazol-4-yl)-3-(furan-2-yl)acrylamide;
*N*-(1-(1-(2,4-bis(trifluoromethyl)phenyl)ethyl)-5-methyl-1H-pyrazol-4-yl)-3-(furan-2-yl)acrylamide;
*N*-(1-(2,4-bis(trifluoromethyl)phenyl)-5-methyl-1H-pyrazol-4-yl)-3-(furan-2-yl)acrylamide;
*N*-(1-(2,4-bis(trifluoromethyl)benzyl)-3-methyl-1H-pyrazol-4-yl)-3-(furan-2-yl)acrylamide;
*N*-(1-(1-(2,4-bis(trifluoromethyl)phenyl)ethyl)-3-methyl-1H-pyrazol-4-yl)-3-(furan-2-yl)acrylamide
*N*-(1-(2,4-bis(trifluoromethyl)phenyl)-3-methyl-1H-pyrazol-4-yl)-3-(furan-2-yl)acrylamide;
*N*-(1-(2,4-bis(trifluoromethyl)phenyl)-1H-pyrazol-4-yl)-5-(pyridin-2-yl)isoxazole-3-carboxamide;
*N*-(1-(1-(2,4-bis(trifluoromethyl)phenyl)ethyl)-5-methyl-1H-pyrazol-4-yl)-5-(pyridin-2-yl)isoxazole-3-carboxamide;
*N*-(1-(2,4-bis(trifluoromethyl)phenyl)-5-methyl-1H-pyrazol-4-yl)-5-(pyridin-2-yl)isoxazole-3-carboxamide;
*N*-(1-(1-(2,4-bis(trifluoromethyl)phenyl)ethyl)-3-methyl-1H-pyrazol-4-yl)-5-(pyridin-2-yl)isoxazole-3-carboxamide;
*N*-(1-(2,4-bis(trifluoromethyl)phenyl)-3-methyl-1H-pyrazol-4-yl)-5-(pyridin-2-yl)isoxazole-3-carboxamide;
*N*-(1-(1-(2,4-bis(trifluoromethyl)phenyl)ethyl)-1H-pyrazol-4-yl)-[2,3'-bipyridine]-5'-carboxamide;
*N*-(1-(2,4-bis(trifluoromethyl)phenyl)-1H-pyrazol-4-yl)-[2,3'-bipyridine]-5'-carboxamide;
*N*-(1-(2,4-bis(trifluoromethyl)benzyl)-5-methyl-1H-pyrazol-4-yl)-[2,3'-bipyridine]-5'-carboxamide;
*N*-(1-(1-(2,4-bis(trifluoromethyl)phenyl)ethyl)-5-methyl-1H-pyrazol-4-yl)-[2,3'-bipyridine]-5'-carboxamide;
*N*-(1-(2,4-bis(trifluoromethyl)phenyl)-5-methyl-1H-pyrazol-4-yl)-[2,3'-bipyridine]-5'-carboxamide;
*N*-(1-(2,4-bis(trifluoromethyl)benzyl)-3-methyl-1H-pyrazol-4-yl)-[2,3'-bipyridine]-5'-carboxamide;
*N*-(1-(1-(2,4-bis(trifluoromethyl)phenyl)ethyl)-3-methyl-1H-pyrazol-4-yl)-[2,3'-bipyridine]-5'-carboxamide;
*N*-(1-(2,4-bis(trifluoromethyl)phenyl)-3-methyl-1H-pyrazol-4-yl)-[2,3'-bipyridine]-5'-carboxamide;
*N*-(1-(2,4-bis(trifluoromethyl)phenyl)-1H-pyrazol-4-yl)-5-(thiophen-2-yl)isoxazole-3-carboxamide;
*N*-(1-(2,4-bis(trifluoromethyl)benzyl)-5-methyl-1H-pyrazol-4-yl)-5-(thiophen-2-yl)isoxazole-3-carboxamide;
*N*-(1-(1-(2,4-bis(trifluoromethyl)phenyl)ethyl)-1H-pyrazol-4-yl)-3-(pyridin-2-yl)isoxazole-5-carboxamide;
*N-(1-(1-(2,4-bis(trifluoromethyl)phenyl)ethyl)-1H-pyrazol-4-yl)-2-(pyridin-2-yl)thiazole-5-*carboxamide;
*N-(1-(1-(2,4-bis(trifluoromethyl)phenyl)ethyl)-1H-pyrazol-4-yl)-2-(pyridin-2-yl)thiazole-4-*carboxamide;
*N-(1-(1-(2,4-bis(trifluoromethyl)phenyl)ethyl)-1H-pyrazol-4-yl)-2-(furan-2-yl)thiazole-5-*carboxamide;
*N*-(1-(1-(2,4-bis(trifluoromethyl)phenyl)ethyl)-1H-pyrazol-4-yl)-[2,4'-bipyridine]-2'-carboxamide;
*N*-(1-(1-(2,4-bis(trifluoromethyl)phenyl)ethyl)-1H-pyrazol-4-yl)-[2,3'-bipyridine]-6'-carboxamide;
*N*-(1-(1-(2,4-bis(trifluoromethyl)phenyl)ethyl)-1H-pyrazol-4-yl)-3-(pyridin-2-yl)acrylamide;
*N*-(1-(2,4-bis(trifluoromethyl)benzyl)-1H-pyrazol-4-yl)-3-(furan-2-yl)acrylamide;
*N*-(1-(1-(2,4-bis(trifluoromethyl)phenyl)ethyl)-1H-pyrazol-4-yl)-3-(furan-2-yl)acrylamide;
*N*-(1-(2,4-bis(trifluoromethyl)phenyl)-3-methyl-1H-pyrazol-4-yl)-3-(furan-2-yl)acrylamide;
*N*-(1-(1-(2,4-bis(trifluoromethyl)phenyl)ethyl)-1H-pyrazol-4-yl)-5-(2-methoxyphenyl)isoxazole-3-carboxamide;
*N*-(1-(2,4-bis(trifluoromethyl)phenyl)-3-methyl-1H-pyrazol-4-yl)-5-(pyridin-2-yl)isoxazole-3-carboxamide;
*N*-(1-(2,4-bis(trifluoromethyl)benzyl)-1H-pyrazol-4-yl)-5-(2-methoxyphenyl)isoxazole-3-carboxamide;
*N*-(1-(2,4-bis(trifluoromethyl)benzyl)-3-methyl-1H-pyrazol-4-yl)-5-(furan-2-yl)isoxazole-3-carboxamide;
*N*-(1-(2,4-bis(trifluoromethyl)benzyl)-5-methyl-1H-pyrazol-4-yl)-5-(furan-2-yl)isoxazole-3-carboxamide;
*N*-(1-(2,4-bis(trifluoromethyl)phenyl)-3-methyl-1H-pyrazol-4-yl)-5-(furan-2-yl)isoxazole-3-carboxamide;
*N*-(1-(2,4-bis(trifluoromethyl)benzyl)-5-methyl-1H-pyrazol-4-yl)-3-(furan-2-yl)acrylamide;
*N*-(1-(2,4-bis(trifluoromethyl)benzyl)-3-methyl-1H-pyrazol-4-yl)-3-(furan-2-yl)acrylamide;
*N-(1-(1-(2,4-bis(trifluoromethyl)phenyl)ethyl)-5-methyl-1H-pyrazol-4-yl)-5-(pyridin-2-*yl)isoxazole-3-carboxamide;
*N*-(1-(1-(2,4-bis(trifluoromethyl)phenyl)ethyl)-3-methyl-1H-pyrazol-4-yl)-5-(pyridin-2-yl)isoxazole-3-carboxamide;
*N-(1-(1-(2,4-bis(trifluoromethyl)phenyl)ethyl)-1H-pyrazol-4-yl)-5-(pyrimidin-2-yl)isoxazole-*3-carboxamide;
*N*-(1-(2,4-bis(trifluoromethyl)benzyl)-1H-pyrazol-4-yl)-N-methyl-5-(pyridin-2-yl)isoxazole-3-carboxamide;
*N-(1-(1-(2,4-bis(trifluoromethyl)phenyl)ethyl)-1H-pyrazol-4-yl)-2-(pyridin-2-yl)thiazole-5-*carboxamide;
*N-(1-(1-(2,4-bis(trifluoromethyl)phenyl)ethyl)-1H-pyrazol-4-yl)-4-methyl-2-(pyridin-2-*yl)thiazole-5-carboxamide;
*N-(1-(1-(2-fluoro-4-(trifluoromethyl)phenyl)ethyl)-1H-pyrazol-4-yl)-5-(pyridin-2-*yl)isoxazole-3-carboxamide;
*N-(1-(1-(4-fluoro-2-(trifluoromethyl)phenyl)ethyl)-1H-pyrazol-4-yl)-5-(pyridin-2-*yl)isoxazole-3-carboxamide;
*N*-(1-(1-(2,4-bis(trifluoromethyl)phenyl)ethyl)-1H-pyrazol-4-yl)-1-methyl-3-(pyridin-2-yl)-1H-pyrazole-5-carboxamide;
*N*-(1-(1-(2,4-bis(trifluoromethyl)phenyl)ethyl)-1H-pyrazol-4-yl)-1-methyl-5-(pyridin-2-yl)-1H-pyrazole-3-carboxamide;
*N*-(1-(2,4-bis(trifluoromethyl)benzyl)-1H-pyrazol-4-yl)-5-(pyrazin-2-yl)isoxazole-3-carboxamide;
*N*-(1-(2,4-bis(trifluoromethyl)benzyl)-1H-pyrazol-4-yl)-5-(6-methylpyridin-2-yl)isoxazole-3-carboxamide;
*N-(1-(1-(2,4-bis(trifluoromethyl)phenyl)ethyl)-1H-pyrazol-4-yl)-5-(6-methylpyridin-2-*yl)isoxazole-3-carboxamide;
*N*-(1-(2,4-bis(trifluoromethyl)benzyl)-1H-pyrazol-4-yl)-2-(pyridin-2-yl)thiazole-5-carboxamide;
*N*-(1-(1-(2,4-bis(trifluoromethyl)phenyl)ethyl)-1H-pyrazol-4-yl)-5-(pyridin-2-yl)-1H-pyrazole-3-carboxamide;
*N*-(1-(1-(2,4-bis(trifluoromethyl)phenyl)ethyl)-1H-pyrazol-4-yl)-1-(pyridin-2-yl)-1H-1,2,3-triazole-4-carboxamide;
*N*-(1-(1-(2,4-bis(trifluoromethyl)phenyl)ethyl)-1H-pyrazol-4-yl)-5-(pyridin-2-yl)-1,3,4-thiadiazole-2-carboxamide;
*N*-(1-(1-(2,4-bis(trifluoromethyl)phenyl)ethyl)-1H-pyrazol-4-yl)-2-(pyridin-2-yl)oxazole-4-carboxamide;
*N-(1-(1-(2,4-bis(trifluoromethyl)phenyl)ethyl)-1H-pyrazol-4-yl)-2-(pyridin-2-yl)oxazole-5-*carboxamide;
*N*-(1-(2,4-bis(trifluoromethyl)benzyl)-1H-pyrazol-4-yl)-5-(pyrimidin-2-yl)isoxazole-3-carboxamide;
*N*-(1-(2,4-bis(trifluoromethyl)benzyl)-1H-pyrazol-4-yl)-2-(pyridin-2-ylamino)thiazole-5-carboxamide;
*N-(1-(1-(2,4-bis(trifluoromethyl)phenyl)ethyl)-1H-pyrazol-4-yl)-5-(pyrazin-2-yl)isoxazole-3-*carboxamide;
*N*-(1-(1-(2,4-bis(trifluoromethyl)phenyl)ethyl)-1H-pyrazol-4-yl)-5-(pyridin-2-yl)-1,3,4-oxadiazole-2-carboxamide;
*N*-(1-(2,4-bis(trifluoromethyl)benzyl)-1H-pyrazol-4-yl)-4-methyl-5-(pyridin-2-yl)isoxazole-3-carboxamide;
*N*-(1-(1-(2,4-bis(trifluoromethyl)phenyl)ethyl)-1H-pyrazol-4-yl)-5-(pyrazin-2-yl)-1,3,4-thiadiazole-2-carboxamide;
*N-(1-(1-(4-fluoro-2-(trifluoromethyl)phenyl)ethyl)-1H-pyrazol-4-yl)-5-(pyridin-2-yl)-1,3,4-*thiadiazole-2-carboxamide;
*N*-(1-(1-(2,4-bis(trifluoromethyl)phenyl)ethyl)-3-methyl-1H-pyrazol-4-yl)-5-(pyridin-2-yl)-1,3,4-thiadiazole-2-carboxamide;
*N*-(1-(1-(2,4-bis(trifluoromethyl)phenyl)ethyl)-1H-pyrazol-4-yl)picolinamide;
*N*-(1-(1-(2,4-bis(trifluoromethyl)phenyl)ethyl)-1H-pyrazol-4-yl)pyrazine-2-carboxamide;
*N*-(1-(2,3-dihydro-1H-inden-1-yl)-1H-pyrazol-4-yl)-5-(pyridin-2-yl)isoxazole-3-carboxamide;
*N*-(1-(2,3-dihydro-1H-inden-1-yl)-1H-pyrazol-4-yl)-5-(furan-2-yl)-1,3,4-thiadiazole-2-carboxamide;
*N*-(1-(2,6-dichlorobenzyl)-1H-pyrazol-4-yl)-5-(pyridin-2-yl)-1,3,4-thiadiazole-2-carboxamide;
*N*-(1-(2,6-dichlorobenzyl)-1H-pyrazol-4-yl)-5-(pyridin-2-yl)isoxazole-3-carboxamide;
*N*-(1-(2,6-dichlorobenzyl)-1H-pyrazol-4-yl)-2-(furan-2-yl)thiazol e-5-carboxamide;
*N*-(1-(2,6-dichlorobenzyl)-1H-pyrazol-4-yl)-5-(furan-2-yl)-1,3,4-thiadiazole-2-carboxamide;
*N*-(1-(2,3-dihydro-1H-inden-1-yl)-1H-pyrazol-4-yl)-5-(furan-2-yl)isoxazole-3-carboxamide;
*N*-(1-(2,4-bis(trifluoromethyl)benzyl)-1H-pyrazol-4-yl)-5-(pyridin-2-yl)-1,3,4-oxadiazole-2-carboxamide;
*N*-(1-(1-(2,4-bis(trifluoromethyl)phenyl)ethyl)-1H-pyrazol-4-yl)-5-(pyrazin-2-yl)-1,3,4-thiadiazole-2-carboxamide;
*N*-(1-(1-(2,4-bis(trifluoromethyl)phenyl)ethyl)-1H-pyrazol-4-yl)-4-methyl-5-(pyridin-2-yl)isoxazole-3-carboxamide;
*N*-(1-(1-(2,4-bis(trifluoromethyl)phenyl)ethyl)-1H-pyrazol-4-yl)-5-(pyrimidin-4-yl)-1,3,4-thiadiazole-2-carboxamide;
*N*-(1-(1-(2,4-bis(trifluoromethyl)phenyl)ethyl)-3-methyl-1H-pyrazol-4-yl)-5-(pyridin-2-yl)-1,3,4-thiadiazole-2-carboxamide; and
*N*-(1-(1-(2,4-bis(trifluoromethyl)phenyl)ethyl)-1H-pyrazol-4-yl)-5-(furan-2-yl)-1,3,4-thiadiazole-2-carboxamide,
or a stereoisomer thereof, or a pharmaceutically acceptable salt thereof.

Representative compounds are listed in Table 1.

**Table 1**

| **Cmpd No.** | **Structure** | **Chemical Name** |
|---|---|---|
| 1-1 | 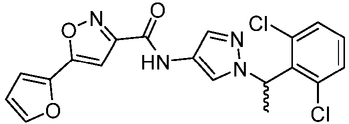 | N-(1-(1-(2,6-dichlorophenyl)ethyl)-1H-pyrazol-4-yl)-5-(furan-2-yl)isoxazole-3-carboxamide |
| Referen ce 1-2 | 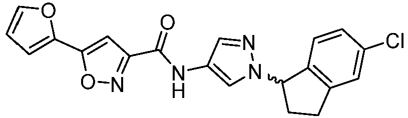 | N-(1-(5-chloro-2,3-dihydro-1H-inden-1-yl)-1H-pyrazol-4-yl)-5-(furan-2-yl)isoxazole-3-carboxamide |
| Referen ce 1-3 | 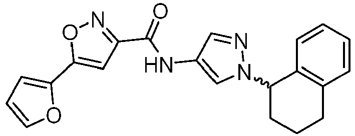 | 5-(furan-2-yl)-N-(1-(1,2,3,4-tetrahydronaphthalen-1-yl)-1H-pyrazol-4-yl)isoxazole-3-carboxamide |
| 1-4 | 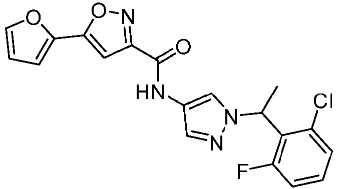 | N-(1-(1-(2-chloro-6-fluorophenyl)ethyl)-1H-pyrazol-4-yl)-5-(furan-2-yl)isoxazole-3-carboxamide |
| Referen ce 1-5 | 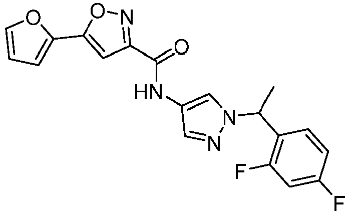 | N-(1-(1-(2,4-difluorophenyl)ethyl)-1H-pyrazol-4-yl)-5-(furan-2-yl)isoxazole-3-carboxamide |
| Referen ce 1-6 | 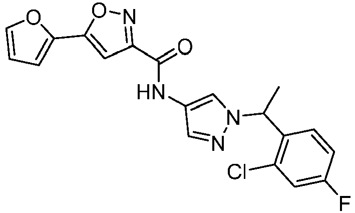 | N-(1-(1-(2-chloro-4-fluorophenyl)ethyl)-1H-pyrazol-4-yl)-5-(furan-2-yl)isoxazole-3-carboxamide |
| Referen ce 1-7 | 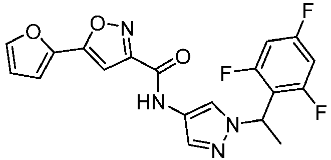 | 5-(furan-2-yl)-N-(1-(1-(2,4,6-trifluorophenyl)ethyl)-1H-pyrazol-4-yl)isoxazole-3-carboxamide |
| 1-8 | 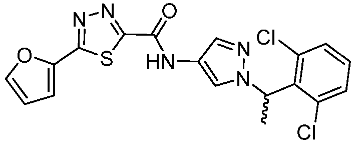 | N-(1-(1-(2,6-dichlorophenyl)ethyl)-1H-pyrazol-4-yl)-5-(furan-2-yl)-1,3,4-thiadiazole-2-carboxamide |
| 1-9 | 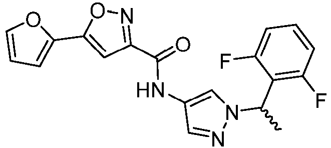 | N-(1-(1-(2,6-difluorophenyl)ethyl)-1H-pyrazol-4-yl)-5-(furan-2-yl)isoxazole-3-carboxamide |
| 1-10 | 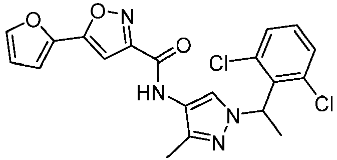 | N-(1-(1-(2,6-dichlorophenyl)ethyl)-3-methyl-1H-pyrazol-4-yl)-5-(furan-2-yl)isoxazole-3-carboxamide |
| Referen ce 1-11 | 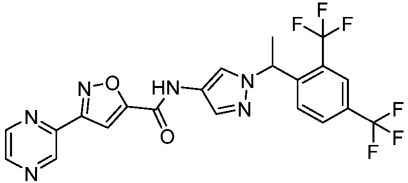 | N-(1-(1-(2,4-bis(trifluoromethyl)phenyl)ethyl)-1H-pyrazol-4-yl)-3-(pyrazin-2-yl)isoxazole-5-carboxamide |
| Referen ce 1-12 | 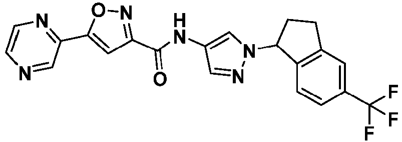 | 5-(pyrazin-2-yl)-N-(1-(5-(trifluoromethyl)-2,3-dihydro-1H-inden-1-yl)-1H-pyrazol-4-yl)isoxazole-3-carboxamide |
| Referen ce 1-13 | 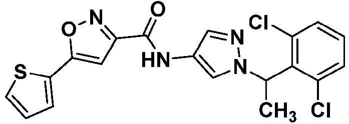 | N-(1-(1-(2,6-dichlorophenyl)ethyl)-1H-pyrazol-4-yl)-5-(thiophen-2-yl)isoxazole-3-carboxamide |
| 1-14 | 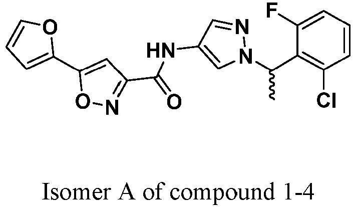 | N-(1-(1-(2-chloro-6-fluorophenyl)ethyl)-1H-pyrazol-4-yl)-5-(furan-2-yl)isoxazole-3-carboxamide |
| 1-15 | 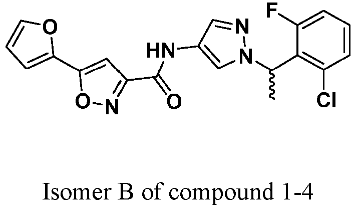 | N-(1-(1-(2-chloro-6-fluorophenyl)ethyl)-1H-pyrazol-4-yl)-5-(furan-2-yl)isoxazole-3-carboxamide |
| Referen ce 1-16 | 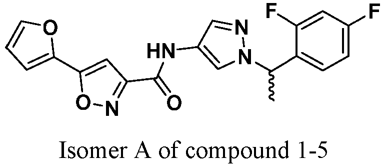 | N-(1-(1-(2,4-difluorophenyl)ethyl)-1H-pyrazol-4-yl)-5-(furan-2-yl)isoxazole-3-carboxamide |
| Referen ce 1-17 | 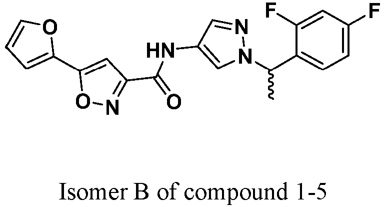 | N-(1-(1-(2,4-difluorophenyl)ethyl)-1H-pyrazol-4-yl)-5-(furan-2-yl)isoxazole-3-carboxamide |
| Referen ce 1-18 | 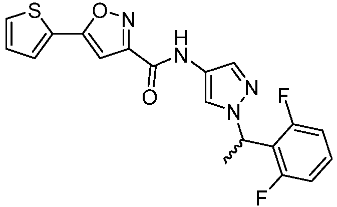 | N-(1-(1-(2,6-difluorophenyl)ethyl)-1H-pyrazol-4-yl)-5-(thiophen-2-yl)isoxazole-3-carboxamide |
| Referen ce 1-19 | 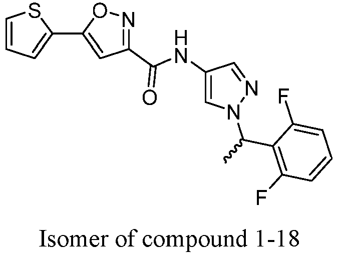 | N-(1-(1-(2,6-difluorophenyl)ethyl)-1H-pyrazol-4-yl)-5-(thiophen-2-yl)isoxazole-3-carboxamide |
| Referen ce 1-20 | 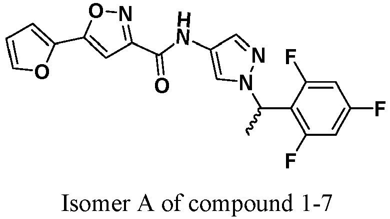 | 5-(furan-2-yl)-N-(1-(1-(2,4,6-trifluorophenyl)ethyl)-1H-pyrazol-4-yl)isoxazole-3-carboxamide |
| Referen ce 1-21 | 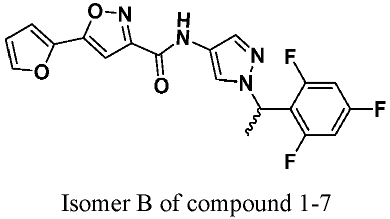 | 5-(furan-2-yl)-N-(1-(1-(2,4,6-trifluorophenyl)ethyl)-1H-pyrazol-4-yl)isoxazole-3-carboxamide |
| 1-22 | 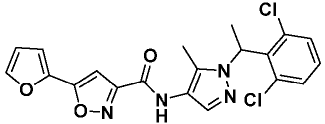 | N-(1-(1-(2,6-dichlorophenyl)ethyl)-5-methyl-1H-pyrazol-4-yl)-5-(furan-2-yl)isoxazole-3-carboxamide |
| 1-23 | 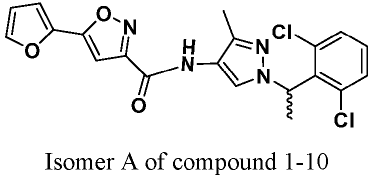 | N-(1-(1-(2,6-dichlorophenyl)ethyl)-3-methyl-1H-pyrazol-4-yl)-5-(furan-2-yl)isoxazole-3-carboxamide |
| 1-24 | 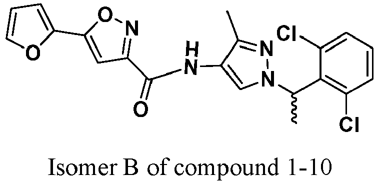 | N-(1-(1-(2,6-dichlorophenyl)ethyl)-3-methyl-1H-pyrazol-4-yl)-5-(furan-2-yl)isoxazole-3-carboxamide |
| Referen ce 1-25 | 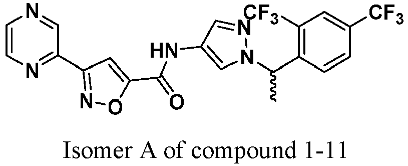 | N-(1-(1-(2,4-bis(trifluoromethyl)phenyl)ethyl)-1H-pyrazol-4-yl)-3-(pyrazin-2-yl)isoxazole-5-carboxamide |
| Referen ce 1-26 | 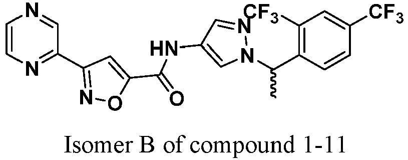 | N-(1-(1-(2,4-bis(trifluoromethyl)phenyl)ethyl)-1H-pyrazol-4-yl)-3-(pyrazin-2-yl)isoxazole-5-carboxamide |

In some embodiments, provided herein are compounds described in **Table 1,** or a stereoisomer thereof, or a pharmaceutically acceptable salt thereof, and uses thereof.

The embodiments and variations described herein are suitable for compounds of any formulae detailed herein, where applicable.

Provided herein is a compound selected from the group consisting of:
N-(1-(1-(2,6-dichlorophenyl)ethyl)-1H-pyrazol-4-yl)-5-(furan-2-yl)isoxazole-3-carboxamide;
N-(1-(1-(2-chloro-6-fluorophenyl)ethyl)-1H-pyrazol-4-yl)-5-(furan-2-yl)isoxazole-3-carboxamide;
N-(1-(1-(2,6-dichlorophenyl)ethyl)-1H-pyrazol-4-yl)-5-(furan-2-yl)-1,3,4-thiadiazole-2-carboxamide;
N-(1-(1-(2,6-difluorophenyl)ethyl)-1H-pyrazol-4-yl)-5-(furan-2-yl)isoxazole-3-carboxamide;
N-(1-(1-(2,6-dichlorophenyl)ethyl)-3-methyl-1H-pyrazol-4-yl)-5-(furan-2-yl)isoxazole-3-carboxamide;and
N-(1-(1-(2,6-dichlorophenyl)ethyl)-5-methyl-1H-pyrazol-4-yl)-5-(furan-2-yl)isoxazole-3-carboxamide,
or a pharmaceutically acceptable salt thereof. Also provided herein are, where applicable, any and all stereoisomers of the compounds depicted herein, including geometric isomers (e.g., cis/trans isomers or E/Z isomers), enantiomers, diastereomers, or mixtures thereof in any ratio, including racemic mixtures.

The embodiments and variations described herein are suitable for compounds of any formulae detailed herein, where applicable.

All representative examples of compounds detailed herein, including intermediates and final compounds according to the present disclosure are depicted herein. It is understood that in one aspect, any of the compounds may be used in the methods detailed herein, including, where applicable, intermediate compounds that may be isolated and administered to an individual.

The compounds depicted herein may be present as salts even if salts are not depicted and it is understood that the present disclosure embraces all salts and solvates of the compounds depicted here, as well as the non-salt and non-solvate form of the compound, as is well understood by the skilled artisan. In all embodiments, the salts of the compounds provided herein are pharmaceutically acceptable salts. Where one or more tertiary amine moiety is present in the compound, the N-oxides are also provided and described.

Where tautomeric forms may be present for any of the compounds described herein, each and every tautomeric form is intended even though only one or some of the tautomeric forms may be explicitly depicted. The tautomeric forms specifically depicted may or may not be the predominant forms in solution or when used according to the methods described herein.

The present disclosure also includes any or all of the stereochemical forms, including any enantiomeric or diastereomeric forms of the compounds described. Compounds of any formula given herein may have asymmetric centers and therefore exist in different enantiomeric or diastereomeric forms. All optical isomers and stereoisomers of the compounds of the general formula, and mixtures thereof in any ratio, are considered within the scope of the formula. Thus, any formula given herein is intended to represent a racemate, one or more enantiomeric forms, one or more diastereomeric forms, one or more atropisomeric forms, and mixtures thereof in any ratio, unless a specific stereochemistry is otherwise indicated. Where a compound of Table 1 is depicted with a particular stereochemical configuration, also provided herein is any alternative stereochemical configuration of the compound, as well as a mixture of stereoisomers of the compound in any ratio. For example, where a compound of Table 1 has a stereocenter that is in an "S" stereochemical configuration, also provided herein is the enantiomer of the compound wherein that stereocenter is in an "R" stereochemical configuration. Likewise, when a compound of Table 1 has a stereocenter that is in an "R" configuration, also provided herein is enantiomer of the compound in an "S" stereochemical configuration. Also provided are mixtures of the compound with both the "S" and the "R" stereochemical configuration. Furthermore, certain structures may exist as geometric isomers (i.e., cis and trans isomers), as tautomers, or as atropisomers. For example, compounds of any formula given herein may contain bonds with restricted rotation and therefore exist in different geometric configurations. Where a compound of Table 1 is depicted as a particular geometric isomer (e.g., E or Z isomer, or cis or trans isomer), also provided herein is any alternative geometric configuration of the compound, as well as a mixture of geometric isomers of the compound in any ratio. For example, where a compound of Table 1 is depicted as a "Z" isomer, also provided herein is the "E" isomer of the compound. Likewise, where a compound of Table 1 is depicted as an "E" isomer, also provided herein is the "Z" isomer of the compound. Also provided are mixtures of the compound with both the "E" and the "Z" stereochemical configuration, wherein the mixtures are in any ratio. Similarly, where a compound of Table 1 is depicted as a *"cis"* isomer, also provided herein is the *"trans"* isomer of the compound; and where a compound is depicted as a *"trans"* isomer, also provided herein is the *"cis"* isomer of the compound. Also provided are mixtures of the compound with both the *"cis"* and the *"trans"* stereochemical configuration, wherein the mixtures are in any ratio. All forms of the compounds are also embraced by the invention, such as crystalline or non-crystalline forms of the compounds. Compositions comprising a compound of the invention are also intended, such as a composition of substantially pure compound, including a specific stereochemical form thereof, or a composition comprising mixtures of compounds of the invention in any ratio, including two or more stereochemical forms, such as in a racemic or non-racemic mixture.

The invention also intends isotopically-labeled and/or isotopically-enriched forms of compounds described herein. The compounds herein may contain unnatural proportions of atomic isotopes at one or more of the atoms that constitute such compounds. In some embodiments, the compound is isotopically-labeled, such as an isotopically-labeled compound of the formula (I) or variations thereof described herein, where a fraction of one or more atoms are replaced by an isotope of the same element. Exemplary isotopes that can be incorporated into compounds of the invention include isotopes of hydrogen, carbon, nitrogen, oxygen, phosphorus, sulfur, chlorine, such as ²H, ³H, ¹¹C, ¹³C, ¹⁴C ¹³N, ¹⁵O, ¹⁷O, ³²P, ³⁵S, ¹⁸F, ³⁶Cl. Certain isotope labeled compounds (e.g. ³H and ¹⁴C) are useful in compound or substrate tissue distribution study. Incorporation of heavier isotopes such as deuterium (²H) can afford certain therapeutic advantages resulting from greater metabolic stability, for example, increased in vivo half-life, or reduced dosage requirements and, hence may be preferred in some instances.

Isotopically-labeled compounds of the present invention can generally be prepared by standard methods and techniques known to those skilled in the art or by procedures similar to those described in the accompanying Examples substituting appropriate isotopically-labeled reagents in place of the corresponding non-labeled reagent.

Articles of manufacture comprising a compound described herein, or a stereoisomer thereof, or a pharmaceutically acceptable salt or solvate thereof, in a suitable container are provided. The container may be a vial, jar, ampoule, preloaded syringe, I.V. bag, and the like.

Preferably, the compounds detailed herein are orally bioavailable. However, the compounds may also be formulated for parenteral (*e.g.,* intravenous) administration.

One or several compounds described herein can be used in the preparation of a medicament by combining the compound or compounds as an active ingredient with a pharmacologically acceptable carrier, which are known in the art. Depending on the therapeutic form of the medication, the carrier may be in various forms. In one variation, the manufacture of a medicament is for use in any of the methods disclosed herein, *e.g.,* for the treatment of cancer.

### General synthetic methods

The compounds of the invention may be prepared by a number of processes as generally described below. In the following process descriptions, the symbols when used in the formulae depicted are to be understood to represent those groups described above in relation to the formulae herein.

Where it is desired to obtain a particular enantiomer of a compound, this may be accomplished from a corresponding mixture of enantiomers using any suitable conventional procedure for separating or resolving enantiomers. Thus, for example, diastereomeric derivatives may be produced by reaction of a mixture of enantiomers, *e.g.,* a racemate, and an appropriate chiral compound. The diastereomers may then be separated by any convenient means, for example by crystallization and the desired enantiomer recovered. In another resolution process, a racemate may be separated using chiral High Performance Liquid Chromatography. Alternatively, if desired a particular enantiomer may be obtained by using an appropriate chiral intermediate in one of the processes described.

Chromatography, recrystallization, and other conventional separation procedures may also be used with intermediates or final products where it is desired to obtain a particular isomer of a compound or to otherwise purify a product of a reaction.

Solvates and/or polymorphs of a compound provided herein or a stereoisomer thereof, or a pharmaceutically acceptable salt thereof are also contemplated. Solvates contain either stoichiometric or non-stoichiometric amounts of a solvent and are often formed during the process of crystallization. Hydrates are formed when the solvent is water, and alcoholates are formed when the solvent is alcohol. Polymorphs include the different crystal packing arrangements of the same elemental composition of a compound. Polymorphs usually have different X-ray diffraction patterns, infrared spectra, melting points, density, hardness, crystal shape, optical and electrical properties, stability, and/or solubility. Various factors such as the recrystallization solvent, rate of crystallization, and storage temperature may cause a single crystal form to dominate.

In some embodiments, compounds of the formula (I) may be synthesized according to Scheme 1. 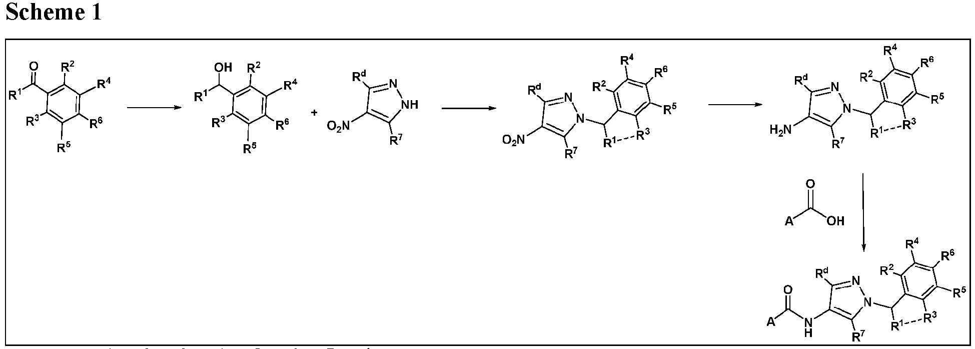 wherein R¹, R², R³, R⁴, R⁵, R⁶, R⁷, R^{d}, and A are as defined for formula (I), or any variation thereof detailed herein.

An exemplary embodiment of the preparative method in Scheme 1 is shown in Scheme 1a. 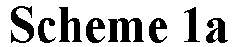 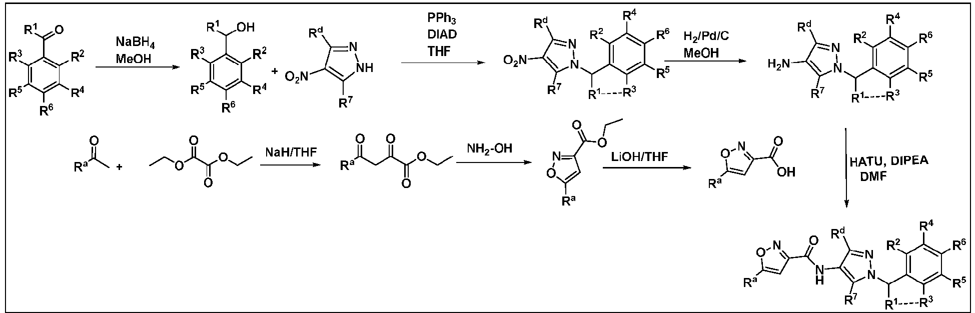 wherein R¹, R², R³, R⁴, R⁵, R⁶, R⁷, R^{d}, and R^{a} are as defined for formula (I), or any variation thereof detailed herein.

In some embodiments, compounds of the formula (I) may be synthesized according to Scheme 2. 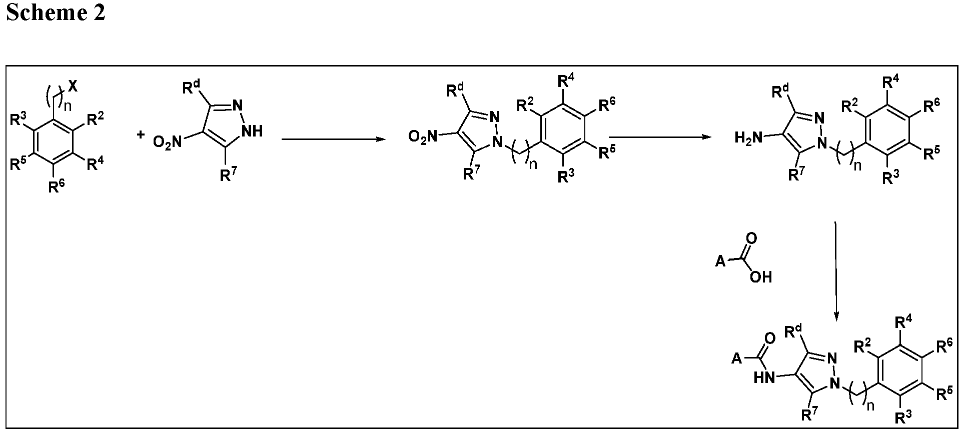 wherein A, R², R³, R⁴, R⁵, R⁶, R⁷, R^{d}, and n are as defined for formula (I), or any variation thereof detailed herein, and X is a halogen.

An exemplary embodiment of the preparative method in Scheme 2 is shown in Scheme 2a. 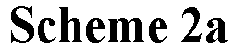 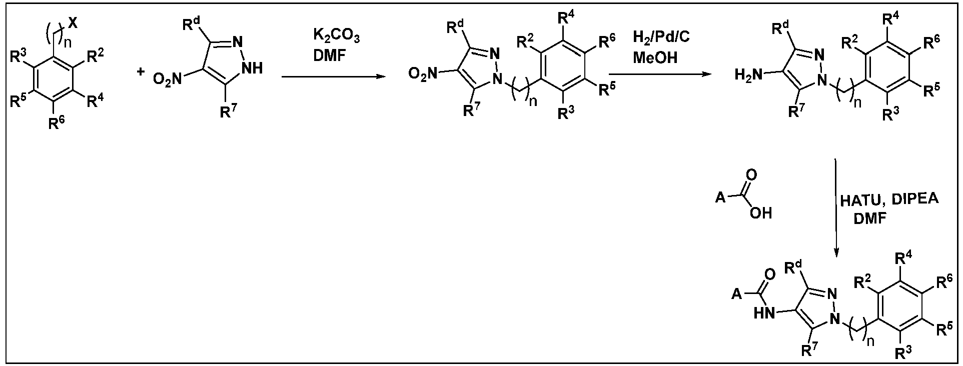 wherein A, R², R³, R⁴, R⁵, R⁶, R⁷, R^{d}, and n are as defined for formula (I), or any variation thereof detailed herein, and X is a halogen.

In some embodiments, compounds of the formula (I) may be synthesized according to Scheme 4. 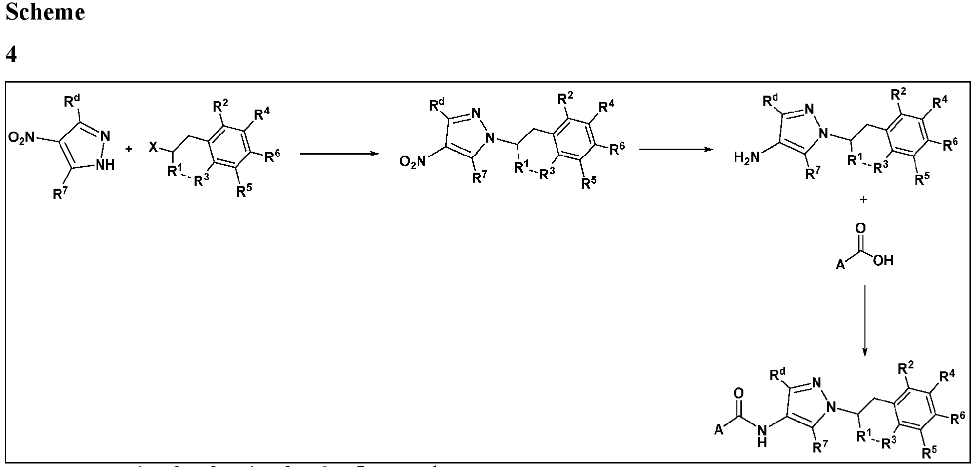 wherein A, R¹, R², R³, R⁴, R³, R⁶, R⁷, are R^{d} are as defined for formula (I), or any variation thereof detailed herein, and X is a halogen.

Particular examples are provided in the Example section below. It is understood that the schemes above may be modified to arrive at various compounds of the invention by selection of appropriate reagents and starting materials. For a general description of protecting groups and their use, see P.G.M. Wuts and T.W. Greene, Greene's Protective Groups in Organic Synthesis 4th edition, Wiley-Interscience, New York, 2006.

### Pharmaceutical Compositions and Formulations

Pharmaceutical compositions of any of the compounds detailed herein are embraced by this disclosure. Thus, the present disclosure includes pharmaceutical compositions comprising a compound as detailed herein or a stereoisomer thereof, or a pharmaceutically acceptable salt thereof and a pharmaceutically acceptable carrier or excipient. In one aspect, the pharmaceutically acceptable salt is an acid addition salt, such as a salt formed with an inorganic or organic acid. Pharmaceutical compositions may take a form suitable for oral, buccal, parenteral, nasal, topical or rectal administration or a form suitable for administration by inhalation.

A compound as detailed herein may in one aspect be in a purified form and compositions comprising a compound in purified forms are detailed herein. Compositions comprising a compound as detailed herein or a stereoisomer thereof, or a pharmaceutically acceptable salt thereof are provided, such as compositions of substantially pure compounds. In some embodiments, a composition containing a compound as detailed herein or a stereoisomer thereof, or a pharmaceutically acceptable salt thereof is in substantially pure form.

In one variation, the compounds herein are synthetic compounds prepared for administration to an individual. In another variation, compositions are provided containing a compound in substantially pure form. In another variation, the present disclosure embraces pharmaceutical compositions comprising a compound detailed herein and a pharmaceutically acceptable carrier. In another variation, methods of administering a compound are provided. The purified forms, pharmaceutical compositions and methods of administering the compounds are suitable for any compound or form thereof detailed herein.

A compound detailed herein or a stereoisomer thereof, or a pharmaceutically acceptable salt thereof may be formulated for any available delivery route, including an oral, mucosal (*e.g.,* nasal, sublingual, vaginal, buccal or rectal), parenteral (*e.g.,* intramuscular, subcutaneous or intravenous), topical or transdermal delivery form. A compound or stereoisomer thereof, or a pharmaceutically acceptable salt thereof may be formulated with suitable carriers to provide delivery forms that include, but are not limited to, tablets, caplets, capsules (such as hard gelatin capsules or soft elastic gelatin capsules), cachets, troches, lozenges, gums, dispersions, suppositories, ointments, cataplasms (poultices), pastes, powders, dressings, creams, solutions, patches, aerosols (*e.g.,* nasal spray or inhalers), gels, suspensions (*e.g.,* aqueous or non-aqueous liquid suspensions, oil-in-water emulsions or water-in-oil liquid emulsions), solutions and elixirs.

One or several compounds described herein or a stereoisomer thereof, or a pharmaceutically acceptable salt thereof can be used in the preparation of a formulation, such as a pharmaceutical formulation, by combining the compound or compounds, or a stereoisomer thereof, or a pharmaceutically acceptable salt thereof, as an active ingredient with a pharmaceutically acceptable carrier, such as those mentioned above. Depending on the therapeutic form of the system (*e.g.,* transdermal patch vs. oral tablet), the carrier may be in various forms. In addition, pharmaceutical formulations may contain preservatives, solubilizers, stabilizers, re-wetting agents, emulgators, sweeteners, dyes, adjusters, and salts for the adjustment of osmotic pressure, buffers, coating agents or antioxidants. Formulations comprising the compound may also contain other substances which have valuable therapeutic properties. Pharmaceutical formulations may be prepared by known pharmaceutical methods. Suitable formulations can be found, *e.g.,* in Remington's Pharmaceutical Sciences, Mack Publishing Company, Philadelphia, PA, 20th ed. (2000).

Compounds as described herein may be administered to individuals in a form of generally accepted oral compositions, such as tablets, coated tablets, and gel capsules in a hard or in soft shell, emulsions or suspensions. Examples of carriers, which may be used for the preparation of such compositions, are lactose, corn starch or its derivatives, talc, stearate or its salts, *etc.* Acceptable carriers for gel capsules with soft shell are, for instance, plant oils, wax, fats, semisolid and liquid poly-ols, and so on. In addition, pharmaceutical formulations may contain preservatives, solubilizers, stabilizers, re-wetting agents, emulgators, sweeteners, dyes, adjusters, and salts for the adjustment of osmotic pressure, buffers, coating agents or antioxidants.

Any of the compounds described herein can be formulated in a tablet in any dosage form described, for example, a compound as described herein or a stereoisomer thereof, or a pharmaceutically acceptable salt thereof can be formulated as a 10 mg tablet.

Compositions comprising a compound provided herein are also described. In one variation, the composition comprises a compound or stereoisomer thereof, or a pharmaceutically acceptable salt thereof and a pharmaceutically acceptable carrier or excipient. In another variation, a composition of substantially pure compound is provided.

### Methods of Use and Uses

Compounds and compositions detailed herein, such as a pharmaceutical composition containing a compound of any formula provided herein or a stereoisomer thereof, or a pharmaceutically acceptable salt thereof and a pharmaceutically acceptable carrier or excipient, may be for use in methods of administration and treatment as provided herein. The compounds and compositions may also be used in *in vitro* methods, such as *in vitro* methods of administering a compound or composition to cells for screening purposes and/or for conducting quality control assays.

In some embodiments, provided herein is a compound or composition described herein for use in a method of modulating the ATF6 pathway. In some embodiments, provided herein is a compound or composition described herein for use in a method of modulating ATF6. In some embodiments, provided herein is a compound or composition described herein for use in a method of activating the ATF6 pathway. In some embodiments, provided herein is a compound or composition described herein for use in a method of activating ATF6. In some embodiments, provided herein is a compound or composition described herein for use in a method of inhibiting the ATF6 pathway. In some embodiments, provided herein is a compound or composition described herein for use in a method of inhibiting ATF6. In some embodiments, the ATF6 is ATF6α. The compounds or stereoisomer thereof, or a pharmaceutically acceptable salts thereof described herein and compositions described herein are believed to be effective for use in inhibiting the ATF6 pathway, ATF6, and/or ATF6α.

In some embodiments, the compound or composition described herein for use in the method of modulating the ATF6 pathway, ATF6, or ATF6α comprises administering or delivering to a cell comprising ATF6 or ATF6α a compound described herein, or a stereoisomer thereof, or a pharmaceutically acceptable salt thereof, or a pharmaceutical composition described herein. In some embodiments, the compound or composition described herein for use in the method of activating the ATF6 pathway, ATF6, or ATF6α comprises administering or delivering to a cell comprising ATF6 or ATF6α a compound described herein, or a stereoisomer thereof, or a pharmaceutically acceptable salt thereof, or a pharmaceutical composition described herein. In some embodiments, the compound or composition described herein for use in the method of inhibiting the ATF6 pathway, ATF6, or ATF6α comprises administering or delivering to a cell comprising ATF6 or ATF6α a compound described herein, or a stereoisomer thereof, or a pharmaceutically acceptable salt thereof, or a pharmaceutical composition described herein. In some embodiments, the cell is a diseased cell, such as a cancer cell. In some embodiments, the cell has an activated ATF6 pathway. In some embodiments, the cell has been exposed to an ER stress-inducing condition. Several ER stress-inducing conditions are known in the art, such as glucose deprivation, aberrant Ca²⁺ regulation, viral infection, hypoxia, and exposure to an ER stress-inducing molecule such as thapsigargin, ionomycin, or tunicamycin.

In some embodiments, the compound or composition described herein for use in the method of modulating the ATF6 pathway, ATF6, or ATF6α comprises administering or delivering a compound described herein, or a stereoisomer thereof, or a pharmaceutically acceptable salt thereof, or a pharmaceutical composition described herein to a tumor. In some embodiments, the compound or composition described herein for use in the method of activating the ATF6 pathway, ATF6, or ATF6α comprises administering or delivering a compound described herein, or a stereoisomer thereof, or a pharmaceutically acceptable salt thereof, or a pharmaceutical composition described herein to a tumor. In some embodiments, the compound or composition described herein for use in the method of inhibiting the ATF6 pathway, ATF6, or ATF6α comprises administering or delivering a compound described herein, or a stereoisomer thereof, or a pharmaceutically acceptable salt thereof, or a pharmaceutical composition described herein to a tumor.

In some embodiments, the modulation of the ATF6 pathway, ATF6, or ATF6α comprises modulating expression of an ATF6 and/or ATF6α target gene. In some embodiments, the modulation of the ATF6 pathway, ATF6, or ATF6α comprises modulating expression of an ATF6α target gene. In some embodiments, the expression of the ATF6 and/or ATF6α target gene is modulated by at least about 10%, 20%, 30%, 40%, 50%, 60%, 70%, 80%, 90%, 95%, or 98%. In some embodiments, the activation of the ATF6 pathway, ATF6, or ATF6α comprises activating expression of an ATF6 and/or ATF6α target gene. In some embodiments, the activation of the ATF6 pathway, ATF6, or ATF6α comprises activating expression of an ATF6α target gene. In some embodiments, the expression of the ATF6 and/or ATF6α target gene is activated by at least about 10%, 20%, 30%, 40%, 50%, 60%, 70%, 80%, 90%, 95%, or 98%. In some embodiments, the inhibition of the ATF6 pathway, ATF6, or ATF6α comprises inhibiting expression of an ATF6 and/or ATF6α target gene. In some embodiments, the inhibition of the ATF6 pathway, ATF6, or ATF6α comprises inhibiting expression of an ATF6α target gene. In some embodiments, the expression of the ATF6 and/or ATF6α target gene is inhibited by at least about 10%, 20%, 30%, 40%, 50%, 60%, 70%, 80%, 90%, 95%, or 98%.

In some embodiments, the ATF6 and/or ATF6α target gene comprises a promoter comprising an ER-stress responsive element (ERSE). In some embodiments, the promoter comprises a sequence that shares at least about 70%, 75%, 80%, 85%, 90%, 95%, 96%, 97%, 98%, or 99% sequence identity with CCAATCGGCGGCGGCCACG (SEQ ID NO. 1). In some embodiments, the promoter comprises SEQ ID NO. 1. In some embodiments, the ATF6 and/or ATF6α target gene is GRP78, HERPUD1, or ERO1B. In some embodiments, the ATF6α target gene is GRP78. Modulation, activation, or inhibition of expression of an ATF6 and/or ATF6α target gene can be determined by methods known in the art, such as by detection of the mRNA of the target gene using a techniques such as PCR, qPCR, or northern blotting, or by detection of polypeptide gene product, such as by western blotting or mass spectrometry.

In some embodiments, the compound, stereoisomer thereof, or a pharmaceutically acceptable salt thereof, or composition modulates, activates, or inhibits the ATF6 pathway, ATF6, or ATF6α with an IC₅₀ of less than about 10 µM, such as less than about 5 µM, 2 µM, 1 µM, 900 nM, 800 nM, 700 nM, or 600 nM. In some embodiments, the compound, stereoisomer thereof, or a pharmaceutically acceptable salt thereof, or composition inhibits the ATF6 pathway, ATF6, or ATF6α with an IC₅₀ between about 10 nM and 5 µM, such between about 50 nM and 2 µM, 100 nM and 1 µM, or 20 nM and 1 µM. The half maximal inhibitory concentration (IC₅₀) is a measure of the effectiveness of a substance in inhibiting a specific biological or biochemical function. The IC₅₀ is a quantitative measure that indicates how much of an inhibitor is needed to inhibit a given biological process or component of a process such as an enzyme, cell, cell receptor or microorganism by half. Methods of determining IC₅₀ *in vitro* and *in vivo* are known in the art.

In some embodiments, the compounds or stereoisomers thereof, or pharmaceutically acceptable salts thereof described herein and compositions described herein are administered in an amount wherein ATF6β activity is not modulated (activated or inhibited) or is modulated (activated or inhibited) to a lesser extent. In some embodiments, modulation (activation or inhibition) of ATF6α is at least or at least about 2 fold greater than inhibition of ATF6α activity, for example at least or at least about 3 fold, 4 fold, 5 fold, 8 fold, 10 fold, 15 fold, 30 fold, 50 fold, 60 fold, 75 fold, or 100 fold greater.

Provided herein is a compound of Formula (I) or stereoisomer thereof, or a pharmaceutically acceptable salt thereof for use in a method of treating a disease in an individual comprising administering an effective amount of a compound of formula (I) or any embodiment, variation or aspect thereof (collectively, a compound of Formula (I) or the present compounds or the compounds detailed or described herein), or a stereoisomer thereof, or a pharmaceutically acceptable salt thereof, to the individual.

In some embodiments, provided herein is a compound of Formula (I) or stereoisomer thereof, or a pharmaceutically acceptable salt thereof for use in a method of treating a disease mediated by the ATF6 pathway in an individual comprising administering an effective amount of a compound of Formula (I), or a stereoisomer thereof, or a pharmaceutically acceptable salt thereof, to the individual. In some embodiments, provided herein is a compound of Formula (I) or stereoisomer thereof, or a pharmaceutically acceptable salt thereof for use in a method of treating a disease mediated by the activation of the ATF6 pathway in an individual comprising administering an effective amount of a compound of Formula (I), or a stereoisomer thereof, or a pharmaceutically acceptable salt thereof, to the individual. In some embodiments, provided herein is a compound of Formula (I) or stereoisomer thereof, or a pharmaceutically acceptable salt thereof for use in a method of treating a disease mediated by the activation of ATF6 in an individual comprising administering an effective amount of a compound of Formula (I), or a stereoisomer thereof, or a pharmaceutically acceptable salt thereof, to the individual. In some embodiments, provided herein is a compound of Formula (I) or stereoisomer thereof, or a pharmaceutically acceptable salt thereof for use in a method of treating a disease mediated by the activation of ATF6α in an individual comprising administering an effective amount of a compound of Formula (I), or a stereoisomer thereof, or a pharmaceutically acceptable salt thereof, to the individual.

In some embodiments, provided herein is a compound of Formula (I) or stereoisomer thereof, or a pharmaceutically acceptable salt thereof for use in a method of treating a disease characterized by activation of the ATF6 pathway in an individual comprising administering an effective amount of a compound of Formula (I), or a stereoisomer thereof, or a pharmaceutically acceptable salt thereof, to the individual. In some embodiments, provided herein is a compound of Formula (I) or stereoisomer thereof, or a pharmaceutically acceptable salt thereof for use in a method of treating a disease characterized by activation of ATF6 in an individual comprising administering an effective amount of a compound of Formula (I), or a stereoisomer thereof, or a pharmaceutically acceptable salt thereof, to the individual. In some embodiments, provided herein is a compound of Formula (I) or stereoisomer thereof, or a pharmaceutically acceptable salt thereof for use in a method of treating a disease characterized by activation of ATF6α in an individual comprising administering an effective amount of a compound of Formula (I), or a stereoisomer thereof, or a pharmaceutically acceptable salt thereof, to the individual. In some embodiments, provided herein is a compound of Formula (I) or stereoisomer thereof, or a pharmaceutically acceptable salt thereof for use in a method of treating a disease characterized by increased expression of an ATF6 target gene in an individual comprising administering an effective amount of a compound of Formula (I), or a stereoisomer thereof, or a pharmaceutically acceptable salt thereof, to the individual. In some embodiments, provided herein is a compound of Formula (I) or stereoisomer thereof, or a pharmaceutically acceptable salt thereof for use in a method of treating a disease characterized by increased expression of an ATF6α target gene in an individual comprising administering an effective amount of a compound of Formula (I), or a stereoisomer thereof, or a pharmaceutically acceptable salt thereof, to the individual. In some embodiments, the increased expression is in comparison to a non-diseased tissue or cell.

The present compounds or stereoisomers thereof, or pharmaceutically acceptable salts thereof are believed to be effective for use in treating a variety of diseases and disorders, such as diseases wherein ATF6-activated transcription targets play a role in the pathogenesis or development of the disease. For example, in some embodiments, the present compounds and compositions may be used to treat viral infection, cancer, a neurodegenerative disease, or a vascular disease, such as a cardiovascular disease. In some embodiments, the disease is viral infection, hereditary cerebellar atrophy and ataxia, or Alzheimer's disease. In some embodiments, the disease is type 2 diabetes mellitus or diabetic nephropathy. In some embodiments, the disease is myocardial atrophy, heart failure, atherosclerosis, or ischemia such as ischemic heart disease or cerebral ischemia.

It has been demonstrated that ATF6 branch of the UPR is central for viral infection. For example, ATF6 is important for maintaining cell viability and modulating immune responses during West Nile virus infection (Ambrose R J. Virol. February 2013 vol. 87 no. 4 2206-2214). Also, African swine fever virus activates ATF6 branch to prevent early apoptosis and ensure viral replication (Galindo I, Cell Death Dis 2012 Jul 5;3:e341. doi: 10.1038/cddis.2012.81). Accordingly, in some embodiments, a compound or stereoisomer thereof, or a pharmaceutically acceptable salt thereof described herein or a composition described herein may be used in a method of treating or preventing a viral infection. In some embodiments, the viral infection is an African swine fever virus, a dengue virus, an enterovirus, a hepatitis B virus, a hepatitis C virus, influenza virus, a tick-borne encephalitis virus, or a West Nile virus infection. In some embodiments, the viral infection is caused by a virus that activates ATF6 in an infected cell.

In some embodiments, a compound or stereoisomer thereof, or a pharmaceutically acceptable salt thereof described herein or a composition described herein may be used in a method of treating cancer, such as breast cancer, colorectal cancer, ovarian cancer, prostate cancer, pancreatic cancer, kidney cancer, lung cancer, melanoma, fibrosarcoma, bone sarcoma, connective tissue sarcoma, renal cell carcinoma, giant cell carcinoma, squamous cell carcinoma, leukemia, skin cancer, soft tissue cancer, liver cancer, gastrointestinal carcinoma, or adenocarcinoma. In some embodiments, the compound, stereoisomer thereof, or a pharmaceutically acceptable salt, or composition may be used in a method of treating metastatic kidney cancer, chronic lymphocytary leukemia, pancreatic adenocarcinoma, or non-small cell lung cancer.

ATF6α transcription targets are expressed at high levels in cancer cells. For example, a correlation exists between intracellular GRP78 level and tumor size (Cai, J.W., et al., J Cell Physiol, 1993, 154(2): 229-37). Furthermore, when GRP78/BiP expression was experimentally suppressed in cancer cells that were then injected into mice, the cells were unable to form tumors due to an increased sensitivity to cytotoxic T-cell (CTL) response and tumor necrosis factor (TNF) (Jamora, C., et al., Proc Natl Acad Sci USA, 1996, 93(15): 7690-7694; Sugawara, S., et al., Cancer Res, 1993, 53(24): 6001-6005).

Cancer cells that are cellularly dormant lack proliferative markers and exist in a quiescent state. Cells known to experience cellulary dormancy include disseminated tumor cells (DTCs) and tumor cells located within the circulation (termed circulating tumor cells (CTCs)) (Hensel, J.A., et al., Nat Rev Clin Oncol, 2013, 10(1): 41-51). Minimal residual disease caused by solitary DTCs is a well-recognized event associated with unfavorable patient prognosis. DTCs, which usually stain negative for proliferation markers (e.g., Ki67), may be the source of tumor recurrence that can develop up to decades after treatment of the primary tumor (Meng, S., et al., Clin Cancer Res, 2004, 10(24): 8152-8162). ATF6α has been reported to be a transducing survival signal through an ATF6α-Rheb-mTOR pathway for dormant carcinoma cells (Schewe, D.M. et al., Proc Natl Acad Sci USA, 2008, 105(30): 10519-10524). ATF6α signaling is important for protection against ER and low glucose stress, and the interaction between ATF6α and mTOR signaling appears to confer resistance of dormant cancer cells to doxorubicin and to the mTOR inhibitor rapamycin, revealing a potential drug resistance mechanism (Schewe, D.M. et al., Proc Natl Acad Sci USA, 2008, 105(30): 10519-10524).

In addition, a multicancer study showed higher ATF6 expression in metastases vs. primary lesions and colon cancer patients with increased expression of ATF6α in their primary tumors had higher chances of relapse (Ramaswamy, S., et al., Proc Natl Acad Sci USA, 2001, 98(26): 15149-15154).

In some embodiments, a compound or stereoisomer thereof, or a pharmaceutically acceptable salt thereof described herein or a composition described herein may be used in a method of treating cancer in an individual, wherein one or more cancer cells in the individual are dormant cancer cells. In some embodiments, one or more of the dormant cancer cells are disseminated tumor cells or circulating tumor cells. In some embodiments, one or more of the dormant cancer cells are disseminated tumor cells.

In some embodiments, a compound or stereoisomer thereof, or a pharmaceutically acceptable salt thereof described herein or a composition described herein may be used in a method of treating cancer in an individual, wherein the individual has had a prior treatment. In some embodiments, the cancer is resistant or refractory to the prior treatment. In some embodiments, the cancer has progressed on the prior treatment. In the embodiments, the cancer is a recurrent cancer. In some embodiments, the prior treatment was treatment with a ubiquitin-proteasome pathway inhibitor (e.g., bortezomib), a taxane (e.g., paclitaxel or docetaxel), a Cox-2 inhibitor (e.g., celecoxib), a platinum-based antineoplastic drug (e.g., cisplatin or oxaliplatin), an anthracycline (e.g. doxorubicin), a pyrimidine analog (e.g. 5-fluorouracil or gemcitabine), a topoisomerase inhibitor (e.g., etoposide), an mTOR inhibitor (e.g., rapamycin), an immune-check point inhibitor, or an agent that is used in immune oncology. In some embodiments, the cancer is resistant to treatment with a ubiquitin-proteasome pathway inhibitor (e.g., bortezomib), a taxane (e.g., paclitaxel or docetaxel), a Cox-2 inhibitor (e.g., celecoxib), a platinum-based antineoplastic drug (e.g., cisplatin or oxaliplatin), an anthracycline (e.g. doxorubicin), a pyrimidine analog (e.g. 5-fluorouracil or gemcitabine), a topoisomerase inhibitor (e.g., etoposide), an mTOR inhibitor (e.g., rapamycin), an immune-check point inhibitor, or an agent that is used in immune oncology. In some embodiments, the cancer is resistant to treatment with doxorubicin and/or rapamycin.

In some embodiments, the compound, stereoisomer thereof, or a pharmaceutically acceptable salt, or composition for use in reducing tumor growth, tumor proliferation, or tumorigenicity in the individual comprising administering the compound, stereoisomer thereof, or a pharmaceutically acceptable salt, or composition. In some embodiments, the compound, stereoisomer thereof, or a pharmaceutically acceptable salt, or composition may be for use in a method of reducing tumor growth, tumor proliferation, or tumorigenicity in an individual in need thereof. In some embodiments, tumor growth is slowed or arrested. In some embodiments, tumor growth is reduced by at least about 10%, 20%, 30%, 40%, 50%, 60%, 70%, 80%, or 90%. In some embodiments, the tumor is reduced in size. In some embodiments, tumor size is reduced by at least about 10%, 20%, 30%, 40%, 50%, 60%, 70%, 80%, or 90%. In some embodiments, tumor metastasis is prevented or slowed. In some embodiments, the tumor growth, tumor proliferation, or tumorigenicity is compared to the tumor growth, tumor proliferation, or tumorigenicity in the individual prior to the administration of the compound, stereoisomer thereof, or a pharmaceutically acceptable salt, or composition. In some embodiments, the tumor growth, tumor proliferation, or tumorigenicity is compared to the tumor growth, tumor proliferation, or tumorigenicity in a similar individual or group of individuals. Methods of measuring tumor growth, tumor proliferation, and tumorigenicity are known in the art, for example by repeated imaging of the individual.

The present compounds or stereoisomers thereof, or pharmaceutically acceptable salts thereof are also believed to be effective at inhibiting angiogenesis. Activation of ATF6 and PERK contributes to the survival effect of vascular endothelial growth factor (VEGF) on endothelial cells (ECs) by positively regulating mTORC2-mediated phosphorylation of AKT on Ser473, which is required for full activity of AKT. Depletion of PLC_{γ}, ATF6, or eIF2a dramatically inhibited VEGF-induced vascularization *in vivo* in mouse Matrigel plugs, a standard angiogenesis assay (Karali, E. et al, Molecular Cell, 2014, 54:559-572). Accordingly, the present compounds or stereoisomers thereof, or pharmaceutically acceptable salts thereof are believed to be effective for treating a variety of diseases and disorders associated with angiogenesis.

Angiogenesis has been implicated in the pathogenesis of a variety of diseases disorders including solid tumors and metastasis, atherosclerosis, retrolental fibroplasia, hemangiomas, chronic inflammation, intraocular neovascular diseases such as proliferative retinopathies, e.g., diabetic retinopathy, age-related macular degeneration (AMD), neovascular glaucoma, immune rejection of transplanted corneal tissue and other tissues, rheumatoid arthritis, and psoriasis. Accordingly, in some embodiments, the present compounds and compositions are for use in a method to treat cancer, such as any cancer described herein, undesired or aberrant hypertrophy, arthritis, rheumatoid arthritis (RA), psoriasis, psoriatic plaques, sarcoidosis, atherosclerosis, atherosclerotic plaques, diabetic and other proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, chronic inflammation, lung inflammation, acute lung injury/ARDS, sepsis, primary pulmonary hypertension, malignant pulmonary effusions, cerebral edema (e.g., associated with acute stroke/closed head injury/trauma), synovial inflammation, pannus formation in RA, myositis ossificans, hypertropic bone formation, osteoarthritis (OA), refractory ascites, polycystic ovarian disease, endometriosis, 3rd spacing of fluid diseases (pancreatitis, compartment syndrome, bums, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), hemophilic joints, hypertrophic scars, inhibition of hair growth, Osler-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), and pleural effusion.

A breakdown in gut barrier defenses in conjunction with microbial dysbiosis is emerging as a key contributor to several disorders, including inflammatory bowel disease, type 1 diabetes, Alzheimer's disease, and cancer. Particularly, in patients with colorectal cancer (CRC), high expression levels of ATF6 in tumor tissues were associated with increased tumor size and reduced disease-free survival. On the other hand, an altered microbiota has been associated with CRC. These data suggest a connection between activation of the UPR, the microbiota, and colon tumorigenesis. It has been demonstrated that a novel relationship between UPR activation via ATF6 and microbiota dependent colon tumorigenesis. Goblet cell loss and bacterial infiltration into epithelial crypts occur before tumor formation and antibiotic treatment of nATF6IEC mice significantly decreased tumor burden. In an inducible mouse model of ATF6 activation, there was 100% tumor incidence at 26 weeks. Four days after activated ATF6 induction, there was a notable increase in the proximity of bacteria to the colonic epithelium with increased cell proliferation, suggesting that these alterations are early events downstream of ATF6 activation. Some researchers found that microbial dysbiosis along with decreased microbial diversity was present in the cecal contents of nATF6IEC mice, as assessed by 16S rRNA gene amplicon sequencing at 5 weeks of age, which is before the onset of tumorigenesis. This dysbiotic microbiota enhanced tumor formation upon transfer into germ-free nATF6IEC mice as compared with transfer of control microbiota into nATF6IEC mice. These data suggest that microbial dysbiosis and subsequent STAT3 signaling in the epithelium significantly contribute to tumorigenesis in this model.

Accordingly, in some embodiments, a compound or stereoisomer thereof, or a pharmaceutically acceptable salt thereof described herein or a composition described herein may be used in a method for preventing or treating CRC through inhibition of ATF6 preventing goblet cell loss and dysbiosis. In some embodiments, a compound or stereoisomer thereof, or a pharmaceutically acceptable salt thereof described herein or a composition described herein may be used in a method for blocking ATF6 signaling and reversing dysbiosis to antagonize tumor progression in a subset of CRC patients.

The capacity of the UPR signaling arms to distinctly influence ER proteostasis and function suggests that selective activation of these pathways has significant potential to alleviate pathologic imbalances in ER proteostasis associated with etiologically diverse human diseases. In particular, activation of the ATF6 signaling arm has been shown to be useful for ameliorating disease-associated imbalances in ER proteostasis and function. The stress-independent activation of the ATF6 transcription factor using a chemical genetic approach induces protective remodeling of ER proteostasis pathways to selectively reduce secretion and extracellular aggregation of destabilized, amyloid disease-associated proteins, such as transthyretin and immunoglobulin light chain, without significantly impacting the secretion of the endogenous proteome (Shoulders et al., 2013; Chen et al., 2014; Cooley et al., 2014; Plate et al., 2016). Accordingly, a compound or stereoisomer thereof, or a pharmaceutically acceptable salt thereof described herein or a composition described herein may be used in a method for correcting pathologic imbalances in ER proteostasis in cellular and animal models of protein misfolding and aggregation diseases.

One aspect of the present invention is based on the unexpected discovery that overexpression of ATF6 in a cell prevents cell death that would otherwise occur when an undesired accumulation of proteins occurs in that cell. Accordingly, in some embodiments, a compound or stereoisomer thereof, or a pharmaceutically acceptable salt thereof described herein or a composition described herein may be used in a method for treating a condition such as Parkinson's disease (PD) associated with the abnormal accumulation of molecules that interact with parkin and that are not properly disposed of within a cell.

In some embodiments, a compound or stereoisomer thereof, or a pharmaceutically acceptable salt thereof described herein or a composition described herein may be used in a method for preventing cell death. For example, preventing neuronal cell death is contemplated within the present invention, including preventing the death of nigral neurons in a mammal, including humans.

In some embodiments, a compound or stereoisomer thereof, or a pharmaceutically acceptable salt thereof described herein or a composition described herein may be used in a method for treating neurodegenerative diseases associated with abnormal precipitation and/or aggregation of proteins. For example, the brains of patients with Alzheimer's disease exhibit neurofibrillary tangles (NFT), senile plaques, and cerebrovascular deposits of amyloid-beta; the brains of patients with prion disorders exhibit plaques comprising prion proteins; the brains of patients with Huntington's disease exhibit huntingtin precipitates; patients with dominantly inherited spinocerebellar ataxias exhibit corresponding ataxin protein precipitates; patients with multiple system atrophy exhibit alpha-synuclein deposits; patients with progressive supranuclear palsy exhibit tau precipitates; and patients with familial amyotrophic lateral sclerosis exhibit SOD1 precipitates (Johnson, W.G., J. Anat. 4:609-616 (2000)). Because these various diseases share common pathological mechanisms, it is likely that they share pathways that lead to aberrant aggregation and/or precipitation of proteins (Hardy, J. and Gwinn-Hardy, K., Science 282(5391):1075-1079 (1998)).

In some embodiments, a compound or stereoisomer thereof, or a pharmaceutically acceptable salt thereof described herein or a composition described herein may be used in a method as either a stand-alone therapy, or as a conjunctive therapy with other agents that are either palliative (e.g., agents that relieve the symptoms of the disorder to be treated), and/or agents that target the etiology of the disorder. For example, the administration to a subject of a composition that increases the expression of ATF6 may be carried out in conjunction with the administration of L-DOPA, dopamine agonists, monoamine oxidase B inhibitors, or any other composition useful in the treatment of a neurodegenerative disease, such as Parkinson's disease.

Overexpression of the active ATF6 transcription factor in the heart also has been shown to improve cardiac performance in mouse models of ischemic heart disease, through a mechanism involving ATF6-dependent regulation of the antioxidant gene, catalase (Jin et al., 2017). Similarly, overexpression of the active ATF6 transcription factor in the liver improves insulin sensitivity in obese mice (Ozcan et al., 2016). These results indicate that ATF6 activation offers a unique therapeutic opportunity to ameliorate ER proteostasis defects implicated in diverse diseases.

In some embodiments, a compound or stereoisomer thereof, or a pharmaceutically acceptable salt thereof described herein or a composition described herein may be used in a method for enhancing myocardial recovery from I/R damage, specifically by activating the endogenous adaptive ATF6 gene program in the heart.

The ATF6α pathway also plays a role in stress-induced lipid accumulation. p50ATF6 interacts with the nuclear form of SREBP-2, thereby antagonizing SREBP-2-regulated transcription of lipogenic genes and lipid accumulation in cultured hepatocytes and kidney cells. Moreover, Atf6α-deleted mice displayed hepatic dysfunction and steatosis much longer than wild-type mice in response to pharmacological induction of ER stress. This could be explained by chronic expression of CHOP and sustained suppression of C/EBPα and/or a failure of ATF6α-mediated induction of genes encoding protein chaperone, trafficking, and ERAD functions. When fed a HFD, Atf6α^{-/-} mice developed hepatic steatosis and glucose intolerance in association with increased expression of SREBP-1c. On the other hand, overexpression of a functionally active nuclear fragment of ATF6 in zebrafish caused fatty liver, suggesting that fine-tuning of ATF6α may be important to prevent liver steatosis.

In some embodiments, a compound or stereoisomer thereof, or a pharmaceutically acceptable salt thereof described herein or a composition described herein may be used in a method of treating metabolic disorders, such as obesity, type I-and type II diabetes, pancreatitis, dyslipidemia, hyperlipidemia conditions, non-alcoholic fatty liver disease (NAFLD), non-alcoholic steatohepatitis (NASH), insulin resistance, hyperinsulinemia, glucose intolerance, hyperglycemia, metabolic syndrome, acute myocardial infarction, hypertension, cardiovascular diseases, atherosclerosis, peripheral arterial disease, apoplexy, heart failure, coronary artery heart disease, renal disease, diabetic complications, neuropathy, gastroparesis, disorder associated with a serious inactivation mutation in insulin receptor, and other metabolic disorders.

In some embodiments, a compound or stereoisomer thereof, or a pharmaceutically acceptable salt thereof described herein or a composition described herein may be used in a method of treating ischemic heart disease or myocardial recovery from ischemia/reperfusion (I/R).

In accordance with the present disclosure, in some embodiments, the individual is a mammal. In some embodiments, the individual is a primate, bovine, ovine, porcine, equine, canine, feline, rabbit, or rodent. In some embodiments, the individual is a human. In some embodiments, the individual has any of the diseases or disorders disclosed herein. In some embodiments, the individual is a risk for developing any of the diseases or disorders disclosed herein.

### Combination Therapy

As provided herein, compounds or stereoisomers thereof, or pharmaceutically acceptable salts thereof described herein and compositions described herein may be administered with an agent to treat any of the diseases and disorders disclosed herein. In some embodiments, the agent modulates the Unfolded Protein Response or the Integrated Stress Response. In some embodiments, the agent is an anti-angiogenesis agent. In some embodiments, the agent is an anticancer agent. In some embodiments, the agent targets an immune checkpoint protein.

In some embodiments, (a) a compound described herein, or a stereoisomer thereof, or a pharmaceutically acceptable salt thereof, or a pharmaceutical composition described herein and (b) an agent are sequentially administered, concurrently administered or simultaneously administered. In certain embodiments, (a) a compound described herein, or a stereoisomer thereof, or a pharmaceutically acceptable salt thereof, or a pharmaceutical composition described herein and (b) an agent are administered with a time separation of about 15 minutes or less, such as about any of 10, 5, or 1 minutes or less. In certain embodiments, (a) a compound described herein, or a stereoisomer thereof, or a pharmaceutically acceptable salt thereof, or a pharmaceutical composition described herein and (b) an agent are administered with a time separation of about 15 minutes or more, such as about any of 20, 30, 40, 50, 60, or more minutes. Either (a) a compound described herein, or a stereoisomer thereof, or a pharmaceutically acceptable salt thereof, or a pharmaceutical composition described herein and (b) an agent may be administered first. In certain embodiments, (a) a compound described herein, or a stereoisomer thereof, or a pharmaceutically acceptable salt thereof, or a pharmaceutical composition described herein and (b) an agent are administered simultaneously.

In some embodiments, the agent modulates the Unfolded Protein Response or the Integrated Stress Response. In some embodiments, the agent inhibits the Unfolded Protein Response or the Integrated Stress Response. In some embodiments, the agent modulates the PERK pathway. In some embodiments, the agent inhibits the PERK pathway. In some embodiments, the agent inhibits PERK. ATF6 is known to work in partnership with IRE1, as one of the target genes of ATF6 is XBP1, the key substrate of IRE1 (Yoshida, H., et al., Cell, 2001, 107(7): 881-891), for example ATF6 and IRE1 signaling are important for survival of melanoma cells undergoing ER stress, suggesting a potential benefit in the use of ATF6 inhibitors in combination with IRE1 inhibitors (Tay, K.H., et al., Cell Signal, 2014, 26(2): 287-294). Accordingly, in some embodiments, the agent modulates the IRE1/XBP1 pathway. In some embodiments, the agent inhibits the IRE1/XBP1 pathway. In some embodiments, the agent inhibits IRE1 or XBP1.

In some embodiments, the agent is an anti-angiogenesis agent. The present compounds or stereoisomers thereof, or pharmaceutically acceptable salts thereof are believed to be effective at inhibiting angiogenesis and for treating diseases and disorders associated with angiogenesis. Accordingly, provided herein is a compound or a stereoisomer thereof, or pharmaceutically acceptable salt thereof or a pharmaceutical composition described herein for use in inhibiting angiogenesis, comprising administering to an individual (a) a compound described herein, or a stereoisomer thereof, or a pharmaceutically acceptable salt thereof, or a pharmaceutical composition described herein and (b) an anti-angiogenesis agent. Also provided herein is a compound or a stereoisomer thereof, or a pharmaceutically acceptable salt thereof or a pharmaceutical composition described herein for use in treating a disease or disorder associated with angiogenesis, such as any disease or disorder associated with angiogenesis disclosed herein, comprising administering to an individual (a) a compound described herein, or a stereoisomer thereof, or a pharmaceutically acceptable salt thereof, or a pharmaceutical composition described herein and (b) an anti-angiogenesis agent. In some embodiments, the anti-angiogenesis agent is a VEGF antagonist. In some embodiments, the anti-angiogenesis agent is bevacizumab or ranibizumab.

The role of angiogenesis as a mediator of immune regulation in the tumor microenvironment has recently come into focus. Furthermore, emerging evidence indicates that immunotherapy can lead to immune-mediated vasculopathy in the tumor, suggesting that the tumor vasculature may be an important interface between the tumor-directed immune response and the cancer itself. The introduction of immune checkpoint inhibition as an effective immunotherapeutic strategy for many cancers has led to a better understanding of this interface. Initial studies of the complex relationship between angiogenesis, VEGF signaling and the immune system suggest that the combination of immune checkpoint blockade with angiogenesis inhibition has potential and efforts to enhance immunotherapy will broadly impact the future of oncology. The effect of ATF6 over VEGF signaling reinforces the idea of the use of ATF6 inhibitors as a combination with immune checkpoint inhibitors (Ott, P.A., F.S. Hodi, and E.I. Buchbinder, Inhibition of Immune Checkpoints and Vascular Endothelial Growth Factor as Combination Therapy for Metastatic Melanoma: An Overview of Rationale, Preclinical Evidence, and Initial Clinical Data. Front Oncol, 2015. 5: p. 202).

Accordingly, in some embodiments, the agent targets an immune checkpoint protein. In some embodiments, the agent is an antibody that targets an immune checkpoint protein. In some embodiments, the agent targets PD-1, PD-L1, PD-L2, CTLA4, TIM3, LAG3, CCR4, OX40, OX40L, IDO, and A2AR. In some embodiments, the agent is an anti-PD-1 antibody, an anti-PD-L1 antibody, or an anti-CTLA-4 antibody.

Provided herein is a compound or a stereoisomer thereof, or pharmaceutically acceptable salt thereof or a pharmaceutical composition described herein for use in a method of enhancing an immune response in an individual comprising administering to the individual (a) a compound described herein, or a stereoisomer thereof, or a pharmaceutically acceptable salt thereof, or a pharmaceutical composition described herein and (b) an agent that targets an immune checkpoint protein. In some embodiments, the individual has cancer. In some embodiments, the enhanced immune response is directed to a tumor or cancerous cell.

Also provided herein is a compound or a stereoisomer thereof, or a pharmaceutically acceptable salt thereof or a pharmaceutical composition described herein for use in methods of treating cancer in an individual in need thereof comprising administering to the individual (a) a compound described herein, or a stereoisomer thereof, or a pharmaceutically acceptable salt thereof, or a pharmaceutical composition described herein and (b) an agent that targets an immune checkpoint protein, wherein an immune response of the individual is increased.

In some embodiments, the agent is an anticancer agent. In some embodiments, anticancer agent is an ubiquitin-proteasome pathway inhibitor (e.g., bortezomib), a taxane (e.g., paclitaxel or docetaxil), a Cox-2 inhibitor (e.g., celecoxib), a platinum-based antineoplastic drug (e.g., cisplatin or oxaliplatin), an anthracycline (e.g. doxorubicin), a pyrimidine analog (e.g. 5-fluorouracil or gemcitabine), a topoisomerase inhibitor (e.g., etoposide), or an agent that modulates the Unfolded Protein Response or the Integrated Stress Response (e.g. an IRE1/XBP1 inhibitor or a PERK inhibitor). In some embodiments, the anticancer agent is oxaliplatin, 5-fluorouracil, or gemcitabine. In some embodiments, the anticancer agent is an immune-check point inhibitor, or an agent that is used in immune oncology.

In some embodiments, an effective amount of a compound described herein, or a stereoisomer thereof, or a pharmaceutically acceptable salt thereof, or a pharmaceutical composition described herein is administered to an individual with cancer to increase sensitivity to one or more anticancer treatments.

Therapeutic resistance is a major barrier to improvement of outcomes for patients with cancer. Radiation can induce ER stress and its downstream signaling and appears to be linked to changes in ROS balance secondary to irradiation. Previously, knockdown of ATF6 was sufficient to enhance radiation induced cell death (Dadey, D.Y., et al., Oncotarget, 2016, 7(2): 2080-2092). This suggests ATF6 as a potential therapeutic target to enhance the efficacy of radiation therapy.

In some embodiments, an effective amount of a compound described herein, or a stereoisomer thereof, or a pharmaceutically acceptable salt thereof, or a pharmaceutical composition described herein is administered to an individual with cancer to increase sensitivity to radiation. In some embodiments, provided herein is a compound or stereoisomer thereof, or pharmaceutically acceptable salts thereof or a pharmaceutical composition for use in treating cancer in an individual in need thereof comprising administering to the individual (a) a compound described herein, or a stereoisomer thereof, or a pharmaceutically acceptable salt thereof, or a pharmaceutical composition described herein and (b) radiation.

In some embodiments, an effective amount of a compound described herein, or a stereoisomer thereof, or a pharmaceutically acceptable salt thereof, or a pharmaceutical composition described herein is administered to an individual with cancer to increase sensitivity to one or more anticancer agents. In some embodiments, the anticancer agent is an ubiquitin-proteasome pathway inhibitor (e.g., bortezomib), a taxane (e.g., paclitaxel or docetaxil), a Cox-2 inhibitor (e.g., celecoxib), a platinum-based antineoplastic drug (e.g., cisplatin or oxaliplatin), an anthracycline (e.g. doxorubicin), a pyrimidine analog (e.g. 5-fluorouracil or gemcitabine), a topoisomerase inhibitor (e.g., etoposide), or an agent that modulates the Unfolded Protein Response or the Integrated Stress Response (e.g. an IRE1/XBP1 inhibitor or a PERK inhibitor). In some embodiments, the anticancer agent is oxaliplatin, 5-fluorouracil, or gemcitabine. In some embodiments, the anticancer agent is an immune-check point inhibitor, or an agent that is used in immune oncology.

Provided herein is a compound or stereoisomer thereof, or pharmaceutically acceptable salt thereof or a pharmaceutical composition described herein for use in treating metabolic and/or fibrotic diseases in an individual comprising administering to the individual (a) a compound described herein, or a stereoisomer thereof, or a pharmaceutically acceptable salt thereof, or a pharmaceutical composition described herein and (b) an agent. In some embodiments, the agent is a proteasome inhibitor, e.g., bortezomib, carfilzomib and ixazomib. In some embodiments, the agent is a monoclonal antibody, e.g., daratumumab and elotuzumab. In some embodiments, the agent is an Inhibitors of Histone deacetylases (HDACs) protein, e.g., panobinostat, romidepsin and vorinostat. In some embodiments, the agent is an Immunomodulatory drug (IMiD), e.g., thalidomide, lenalidomide, and pomalidomide. In some embodiments, the agent is an adrenal corticosteroid, e.g., dexamethasone, prednisone, prednisolone, and methylprednisolone. In some embodiments, the agent is a therapy targeting the IRE1-XBP1.

### Dosing and Method of Administration

The dose of a compound administered to an individual (such as a human) may vary with the particular compound or stereoisomer thereof, or a pharmaceutically acceptable salt thereof, the method of administration, and the particular disease, such as type and stage of cancer, being treated. In some embodiments, the amount of the compound or stereoisomer thereof, or a pharmaceutically acceptable salt thereof is a therapeutically effective amount.

The effective amount of the compound may in one aspect be a dose of between about 0.01 and about 100 mg/kg. Effective amounts or doses of the compounds of the invention may be ascertained by routine methods, such as modeling, dose escalation, or clinical trials, taking into account factors, e.g., the mode or route of administration or drug delivery, the pharmacokinetics of the agent, the severity and course of the disease to be treated, the subject's health status, condition, and weight. An exemplary dose is in the range of about from about 0.1 mg to 10 g daily.

Any of the compounds or stereoisomers thereof, or pharmaceutically acceptable salts thereof or pharmaceutical compositions for use provided herein may in one aspect comprise administering to an individual a pharmaceutical composition that contains an effective amount of a compound provided herein or a stereoisomer thereof, or a pharmaceutically acceptable salt thereof and a pharmaceutically acceptable excipient.

A compound or composition of the invention may be administered to an individual in accordance with an effective dosing regimen for a desired period of time or duration, such as at least about one month, at least about 2 months, at least about 3 months, at least about 6 months, or at least about 12 months or longer, which in some variations may be for the duration of the individual's life. In one variation, the compound is administered on a daily or intermittent schedule. The compound can be administered to an individual continuously (for example, at least once daily) over a period of time. The dosing frequency can also be less than once daily, *e*.*g*., about a once weekly dosing. The dosing frequency can be more than once daily, *e*.*g*., twice or three times daily. The dosing frequency can also be intermittent, including a 'drug holiday' (e.g., once daily dosing for 7 days followed by no doses for 7 days, repeated for any 14 day time period, such as about 2 months, about 4 months, about 6 months or more). Any of the dosing frequencies can employ any of the compounds described herein together with any of the dosages described herein.

The compounds provided herein or a stereoisomer thereof, or a pharmaceutically acceptable salt thereof may be administered to an individual via various routes, including, e.g., intravenous, intramuscular, subcutaneous, oral, and transdermal. In some embodiments, the compound or composition is administered orally. A compound provided herein can be administered frequently at low doses, known as 'metronomic therapy,' or as part of a maintenance therapy using compound alone or in combination with one or more additional drugs. Metronomic therapy or maintenance therapy can comprise administration of a compound provided herein in cycles. Metronomic therapy or maintenance therapy can comprise intra-tumoral administration of a compound provided herein.

Also provided herein are compositions (including pharmaceutical compositions) as described herein for the use in treating, preventing, and/or delaying the onset and/or development of a disease described herein and other methods described herein. In certain embodiments, the composition comprises a pharmaceutical formulation which is present in a unit dosage form.

### Articles of Manufacture and Kits

The present disclosure further provides articles of manufacture comprising a compound of the disclosure or a stereoisomer thereof, or a pharmaceutically acceptable salt thereof, composition, and unit dosages described herein in suitable packaging. In certain embodiments, the article of manufacture is for use in any of the methods described herein. Suitable packaging is known in the art and includes, for example, vials, vessels, ampules, bottles, jars, flexible packaging and the like. An article of manufacture may further be sterilized and/or sealed.

The present disclosure further provides kits for carrying out the methods of the disclosure, which comprises one or more compounds described herein or a composition comprising a compound described herein. The kits may employ any of the compounds disclosed herein. In one variation, the kit employs a compound described herein or a stereoisomer thereof, or a pharmaceutically acceptable salt thereof. The kits may be used for any one or more of the uses described herein, and, accordingly, may contain instructions for the treatment of disease described herein, such as cancer.

Kits generally comprise suitable packaging. The kits may comprise one or more containers comprising any compound described herein. Each component (if there is more than one component) can be packaged in separate containers or some components can be combined in one container where cross-reactivity and shelf-life permit.

The kits may be in unit dosage forms, bulk packages (*e*.*g*., multi-dose packages) or sub-unit doses. For example, kits may be provided that contain sufficient dosages of a compound as disclosed herein and/or a second pharmaceutically active compound useful for a disease detailed herein (e.g., hypertension) to provide effective treatment of an individual for an extended period, such as any of a week, 2 weeks, 3 weeks, 4 weeks, 6 weeks, 8 weeks, 3 months, 4 months, 5 months, 7 months, 8 months, 9 months, or more. Kits may also include multiple unit doses of the compounds and instructions for use and be packaged in quantities sufficient for storage and use in pharmacies (*e*.*g*., hospital pharmacies and compounding pharmacies).

The kits may optionally include a set of instructions, generally written instructions, although electronic storage media (*e*.*g*., magnetic diskette or optical disk) containing instructions are also acceptable, relating to the use of component(s) of the methods of the present invention. The instructions included with the kit generally include information as to the components and their administration to an individual.

The invention can be further understood by reference to the following examples, which are provided by way of illustration and are not meant to be limiting.

### EXAMPLES

### Synthetic Examples

The following examples are offered to illustrate but not to limit the present disclosure. One of skill in the art will recognize that the following synthetic reactions and schemes may be modified by choice of suitable starting materials and reagents in order to access other compounds of formula (I), or a stereoisomer thereof, or a pharmaceutically acceptable salt thereof. The compounds are prepared using the general methods described above.

The following abbreviations are used throughout the Examples: DCM (dichloromethane), DIAD (diisopropyl azodicarboxylate), DIPEA or DIEA (*N,N-*diisopropylethylamine), DMF (N,N-dimethylformamide), DMSO (dimethyl sulfoxide), HATU ((1-[bis(dimethylamino)methylene]-1H-1,2,3-triazolo[4,5-b]pyridinium 3-oxide hexafluorophosphate), HPLC (high-pressure liquid chromatography), IPA (isopropyl alcohol), LCMS (liquid chromatography mass spectrometry), NMR (nuclear magnetic resonance), PPh₃ (triphenylphosphine), RT (room temperature), TEA (triethylamine), THF (tetrahydrofuran), and TLC (thin layer chromatography).

### Example S1

### Example S1-1. Synthesis of N-(1-(1-(2,6-dichlorophenyl)ethyl)-1H-ppyrazol-4-yl)-5-(furan-2-yl)isoxazole-3-carboxamide (Compound 1-1).

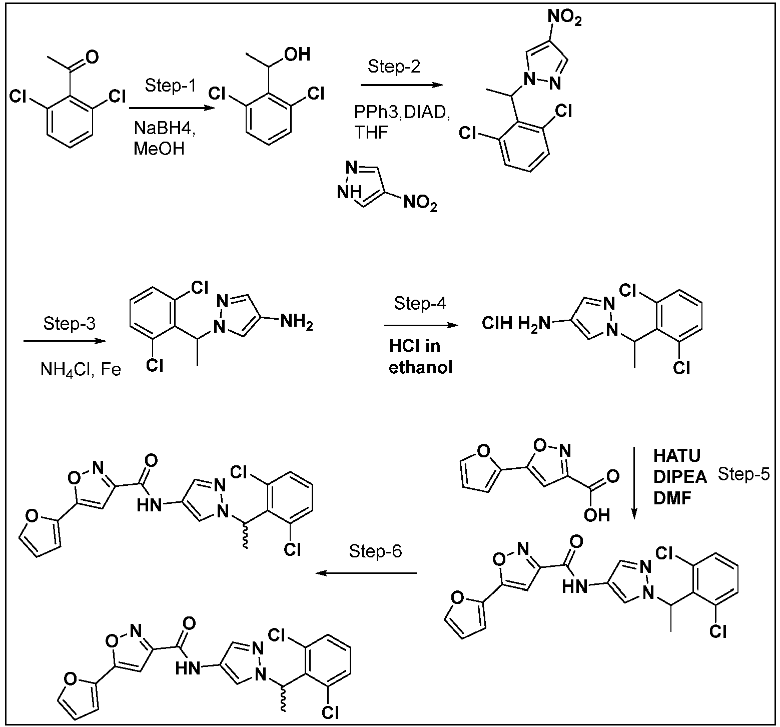

**Step 1: Synthesis of 1-(2,6-dichlorophenyl)ethan-1-ol.** To a stirred solution of 1-(2,6-dichlorophenyl)ethan-1-one (1 gm, 5.28 mmol, 1.0 equiv) in methanol (5 mL) was added NaBH₄ (303 mg, 8.0 mmol, 1.5 equiv) portion wise at 0°C and stirred for 10 minutes. The reaction mixture was allowed to stir for 1 hour at RT. Product formation was confirmed by TLC & LCMS. After completion of reaction, reaction mixture was quenched with water and extracted with ethyl acetate (50 mL x 3). Combined organic extracts were washed with water (50 mL x 2), dried over anhydrous Na₂SO₄ and concentrated under reduced pressure to obtain 1-(2,6-dichlorophenyl)ethan-1-ol (990 mg, as colourless liquid), **¹H NMR (400 MHz, DMSO-*d*₆)** δ 7.47 - 7.63 (m, 2H), 7.30 - 7.47 (m, 1H), 5.55 (br. s., 1H), 4.20 (br. s., 1H), 1.45 (d, *J* = 6.58 Hz, 3H).

**Step 2: Synthesis of 1-(1-(2,6-dichlorophenyl)ethyl)-4-nitro-1H-pyrazole.** To a stirred solution of PPh₃ (413 mg, 1.578 mmol, 1.5 equiv) and DIAD (318.94 mg, 1.578 mmol, 1.5 equiv) in THF (2 mL), was added 4-nitro-1H-pyrazole (118.947 mg, 1.05 mmol, 1 equiv), followed by the addition of 1-(2,6-dichlorophenyl)ethan-1-ol (200 mg, 1.05 mmol, 1.0 equiv). The resultant reaction mixture was stirred at RT for 1 h. Product formation was confirmed with TLC & LCMS. After completion of reaction mixture were diluted with EtOAc (50 mL) & washed with water (50 mL x 3). Organic layer dried over Na₂SO₄ & concentrated under reduced pressure to obtain crude which was further purified by flash column chromatography to obtain pure product 1-(1-(2,6-dichlorophenyl)ethyl)-4-nitro-1H-pyrazole (160 mg), **¹H NMR (400 MHz, DMSO-*d*₆)** δ 9.14 (s, 1H), 8.27 (s, 1H), 7.49 (d, *J =* 7.89 Hz, 2H), 7.29 - 7.44 (m, 1H), 6.13 - 6.36 (m, 1H), 1.99 (d, *J =* 7.02 Hz, 3H).

**Step 3: Synthesis of 1-(1-(2,6-dichlorophenyl)ethyl)-1H-pyrazol-4-amine.** To a stirred solution of 1-(1-(2,6-dichlorophenyl)ethyl)-4-nitro-1H-pyrazole (160 mg, 0.6235 mmol, 1 equiv.) in 10 mL EtOH/ water (1:1), Fe (172.5 mg, 3.135 mmol, 5 equiv.) and ammonium chloride (175.68 mg, 3.136 mmol, 5 equiv.) was added and allowed to heat at 80°C for 2 hr. Reaction progress was monitored by TLC and LCMS. After reaction completion, reaction mixture was filtered through celite pad and filtrate was evaporated and extracted with DCM/ water 2 times. The organic layer was collected and evaporated under reduced pressure to give 1-(1-(2,6-dichlorophenyl)ethyl)-1H-pyrazol-4-amine (110 mg), **LCMS:** 256 [M+H] ⁺.

**Step 4: Synthesis of 1-(1-(2,6-dichlorophenyl)ethyl)-1H-pyrazol-4-amine hydrochloride.** To a stirred solution 1-(1-(2,6-dichlorophenyl)ethyl)-1H-pyrazol-4-amine (100 mg) in ethanol was added HCl in ethanol (1 mL) and allowed to stir at RT for 1 hr. After reaction completion, the reaction mixture was evaporated and lyophilised to give product 1-(1-(2,6-dichlorophenyl)ethyl)-1H-pyrazol-4-amine hydrochloride (125 mg), **LCMS:** 256 [M+H]⁺.

**Step 5: Synthesis of N-(1-(1-(2,6-dichlorophenyl)ethyl)-1H-pyrazol-4-yl)-5-(furan-2-yl)isoxazole-3-carboxamide.** To a solution of 5-(furan-2-yl)isoxazole-3-carboxylic acid (50 mg, 0.277 mmol, 1 equiv) in DMF (1 mL), were added HATU (105.5 mg, 0.277 mmol, 1.0 equiv). The mixture was treated drop wise with DIPEA (107.99 ml, 0.831 mmol, 3.0 equiv). After stirring at RT for 15 minutes, the mixture was treated drop wise with a solution of the 1-(1-(2,6-dichlorophenyl)ethyl)-1H-pyrazol-4-amine hydrochloride (80.55 mg, 0.555 mmol, 1 equiv) in DMF (1 mL). The reaction mixture was kept under stirring for 24 h. After completion of reaction mixture were diluted with EtOAc (50 mL) & washed with water (10 mL x 3). The organic layer was dried over Na₂SO₄ & concentrated under reduced pressure to obtain a crude which was further purified by trituration with acetone: hexane (8:2) ml to afford precipitate as N-(1-(1-(2,6-dichlorophenyl)ethyl)-1H-pyrazol-4-yl)-5-(furan-2-yl)isoxazole-3-carboxamide, **LCMS:** 417 [M+H] ⁺.

**Step 6: Synthesis of (R) & (S)- N-(1-(1-(2,6-dichlorophenyl)ethyl)-1H-pyrazol-4-yl)-5-(furan-2-yl)isoxazole-3-carboxamide.** The racemic mixtue of N-(1-(1-(2,6-dichlorophenyl)ethyl)-1H-pyrazol-4-yl)-5-(furan-2-yl)isoxazole-3-carboxamide was purified by chiral normal phase HPLC (Daicel Chiralpak^{®}-IC, 250 × 20 mm, 5µm). Isocratic program with HPLC grade n-hexane and HPLC grade isopropanol, total flow: 56 ml/min, Co-Solvent Percentage: 10% to obtain Enantiomer A (12 mg) and Enantiomer B (10 mg). **LCMS** 417 [M+H]⁺; **Enantiomer A, ¹H NMR (400 MHz, DMSO-*d*₆)** δ ppm 11.03 (br. s., 1H), 8.10 (s, 1H), 8.00 (s, 1H), 7.64 (s, 1H), 7.49 (d, *J =* 7.89 Hz, 2H), 7.34 - 7.41 (m, 1H), 7.28 (d, *J =* 3.51 Hz, 1H), 7.12 (s, 1H), 6.77 (dd, *J* = 1.75, 3.51 Hz, 1H), 6.11 - 6.22 (m, 1H), 1.96 (d, *J =* 7.02 Hz, 3H). **Enantiomer B, ¹H NMR (400 MHz, DMSO-*d*₆)** δ 11.02 (s, 1H), 8.11 (s, 1H), 8.01 (s, 1H), 7.66 (s, 1H), 7.49 (d, *J =* 7.89 Hz, 2H), 7.34 - 7.41 (m, 1H), 7.29 (d, *J =* 3.95 Hz, 1H), 7.14 (s, 1H), 6.78 (br. s., 1H), 6.10-6.21 (m, 1H), 1.96 (d, *J =* 7.45 Hz, 3H).

### Reference Example S1-2. Synthesis of N-(1-(5-chloro-2,3-dihydro-1H-inden-1-yl)-1H-pyrazol-4-yl)-5-(furan-2-yl)isoxazole-3-carboxamide (Reference Compound 1-2)

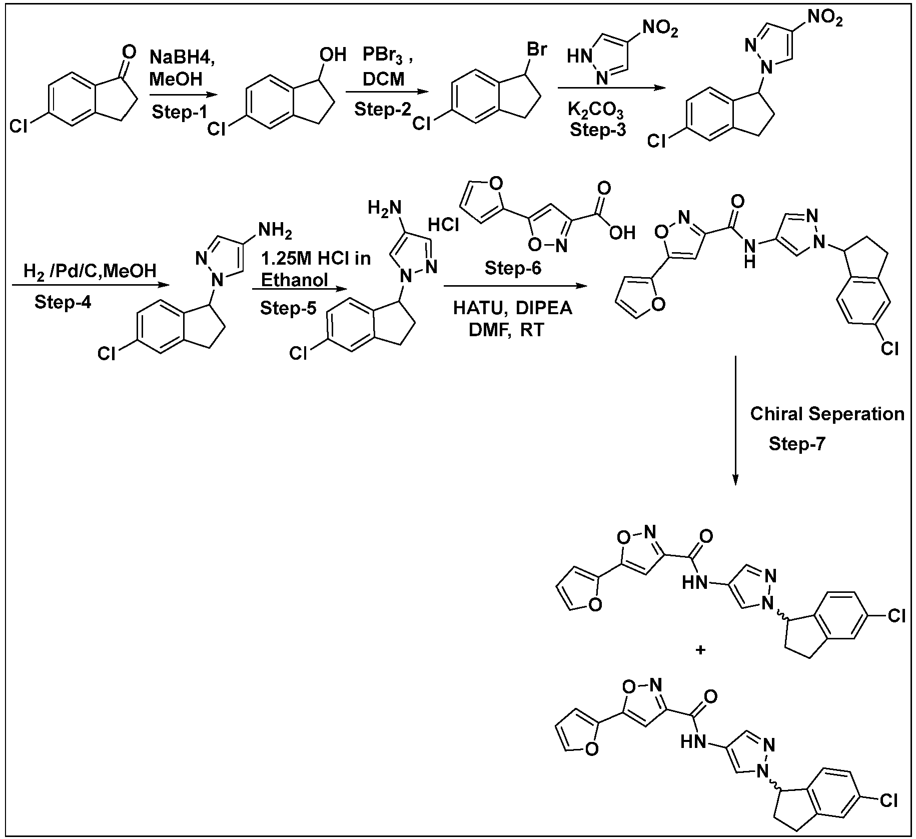

**Step 1: Synthesis of 5-chloro-2,3-dihydro-1H-inden-1-ol.** To a stirred solution of 5-chloro-2,3-dihydro-1H-inden-1-one (500 mg, 3.01 mmol, 1.0 eq) in methanol (20 mL) was added NaBH₄ (120 mg, 4.518 mmol, 1.5 eq) portion wise at 0 °C and stirred for 10 minutes. The reaction mixture was allowed to stir for 1 hour at RT. Product formation was confirmed by TLC and NMR. After completion of reaction, the reaction mixture was quenched with water and extracted with ethyl acetate (3 x 50 mL). The combined organic extracts were washed with water (2 x 50 mL), dried over anhydrous Na₂SO₄ and concentrated under reduced pressure to obtain 5-chloro-2,3-dihydro-1H-inden-1-ol (500 mg).

**Step 2: Synthesis of 1-bromo-5-chloro-2,3-dihydro-1H-indene.** To a stirred solution of 5-chloro-2,3-dihydro-1H-inden-1-ol (500 mg, 2.747 mmole, 1eq.) in DCM (10 mL) was added PBr₃ (893 mg, 3.29 mmol, 1.2 eq) drop wise at 0 degree Celsius. After that the reaction mixture was stirred at RT for 2 hr. Product formation was confirmed with TLC and ¹HNMR. After completion of reaction mixture was diluted with EtOAc (50 mL) and washed with water (50 mL x 3). The orgainc layer was dried over Na₂SO₄ and concentrated under reduced pressure to obtained crude which was further purified by flash column chromatography to obtain pure product 1-bromo-5-chloro-2,3-dihydro-1H-indene pyrazole (702 mg, as brown liquid).

**Step 3: Synthesis of 1-(5-chloro-2,3-dihydro-1H-inden-1-yl)-4-nitro-1H-pyrazole.** To a solution of 4-nitro-1H-pyrazole (300 mg, 1.304 mmol, 1 eq.) in DMF (1 mL) was added K₂CO₃ (270 mg, 1.955 mmol, 1.5 eq.) at 0 degree Celsius. After stirring for 15 minutes, the mixture was treated drop wise with a solution of the 1-bromo-5-chloro-2,3-dihydro-1H-indene (147 mg, 1.304 mmol, 1 eq.) in DMF (1 mL). The reaction mixture was kept under stirring for 24 h. Product formation was confirmed with TLC and LCMS and reaction mixture was diluted with EtOAc (50 mL) and washed with water (2 x 50 mL). The organic layer was dried over Na₂SO₄ and concentrated under reduced pressure to obtain crude which was further purified by flash column chromatography to obtain pure 1-(5-chloro-2,3-dihydro-1H-inden-1-yl)-4-nitro-1H-pyrazole(225 mg, as white solid), **¹H NMR (400 MHz, DMSO-*d*₆)** δ 9.00 (s, 1H), 8.27 (s, 1H), 7.43 (s, 1H), 7.25 (d, *J =* 8.77 Hz, 1H), 7.17 (d, *J =* 8.33 Hz, 1H), 5.92 - 6.03 (m, 1H), 3.14 - 3.24 (m, 1H), 2.90 - 3.04 (m, 1H), 2.62 - 2.73 (m, 2H), 2.41 - 2.48 (m, 1H).

**Step 4: Synthesis of 1-(5-chloro-2,3-dihydro-1H-inden-1-yl)-1H-pyrazol-4-amine.** To a stirred solution of 1-(5-chloro-2,3-dihydro-1H-inden-1-yl)-4-nitro-1H-pyrazole (150 mg, 0.566 mmol, 1 equiv.) in 10 mL EtOH/water (1:1), Fe (155.66 mg, 2.83 mmol, 5 equiv.) and ammonium chloride (158 mg, 2.83 mmol, 5 equiv.) was added and allowed to heat at 80°C for 2 hr. Reaction progress was monitored by TLC and LCMS. After reaction completion, the reaction mixture was filtered through Celite pad and filtrate was evaporated and extracted with DCM (100 ml x 2). The organic layer was collected and evaporated under reduced pressure to give 1-(5-chloro-2,3-dihydro-1H-inden-1-yl)-1H-pyrazol-4-amine (180 mg), **LCMS:** 234 [M+H] ⁺, **¹H NMR (400 MHz, DMSO-*d*₆)** δ 7.38 (s, 1H), 7.21 (d, *J =* 6.14 Hz, 1H), 7.02 (d, *J =* 8.33 Hz, 1H), 6.97 (s, 1H), 6.93 (s, 1H), 5.62 - 5.70 (m, 1H), 3.85 (br. s., 2H), 3.02 - 3.13 (m, 1H), 2.83 - 2.96 (m, 1H), ).

**Step 5: Synthesis of 1-(5-chloro-2,3-dihydro-1H-inden-1-yl)-1H-pyrazol-4-amine hydrochloride.** To a stirred solution of 1-(5-chloro-2,3-dihydro-1H-inden-1-yl)-1H-pyrazol-4-amine (180 mg.) in ethanol (1 mL), were added 4M HCl in ethanol (2 ml) for 2h at RT. Product formation was confirmed with TLC and ¹HNMR. The reaction mixture was concentrated under reduced pressure to obtain crude which was further triturated with diethyl ether and lyophilized to obtain pure product 1-(5-chloro-2,3-dihydro-1H-inden-1-yl)-1H-pyrazol-4-amine hydrochloride (150 mg, brown solid), **¹H NMR (400 MHz, DMSO-*d*₆)** δ 10.07 (br. s., 2H), 8.01 (s, 1H), 7.56 (s, 1H), 7.43 (s, 1H), 7.25 (d, *J =* 8.33 Hz, 1H), 7.05 (d, *J* = 8.33 Hz, 1H), 5.87 - 6.01 (m, 1H), 3.07 - 3.19 (m, 1H), 2.95 (td, *J =* 7.73, 15.68 Hz, 1H), 2.63 (dtd, *J* = 5.26, 8.17, 13.48 Hz, 1H), 2.28 - 2.43 (m, 2H).

**Step 6: Synthesis of N-(1-(5-chloro-2,3-dihydro-1H-inden-1-yl)-1H-pyrazol-4-yl)-5-(furan-2-yl)isoxazole-3-carboxamide.** To a solution of 5-(furan-2-yl)isoxazole-3-carboxylic acid (50 mg, 0.277 mmol, 1 eq) in DMF (1 mL), was added HATU (105.8 mg, 0.277 mmol, 1 eq). The mixture was treated drop wise with DIPEA (107.5 mg, 0.833 mmol, 3 eq). After stirring at RT for 15 minutes, the mixture was treated drop wise with a solution of 1-(5-chloro-2,3-dihydro-1H-inden-1-yl)-1H-pyrazol-4-amine hydrochloride (64.722 mg, 0.277 mmol, 1 eq) in DMF (1 mL). The reaction mixture was kept under stirring for 24 h. Product formation was confirmed with TLC and LCMS and the reaction mixture was diluted EtOAc (50 mL) and washed with water (2 x 50 mL). The organic layer was dried over Na₂SO₄ and concentrated under reduced pressure to obtain crude which was further purified by flash column chromatography to obtain pure N-(1-(5-chloro-2,3-dihydro-1H-inden-1-yl)-1H-pyrazol-4-yl)-5-(furan-2-yl)isoxazole-3-carboxamide (50 mg, as white solid), **LCMS:** 395[M+H] **⁺,¹H NMR (400 MHz, DMSO-*d*₆)** δ 11.01 (s, 1H), 8.07 (s, 1H), 8.00 (s, 1H), 7.66 (s, 1H), 7.42 (s, 1H), 7.28 (d, *J =* 3.07 Hz, 1H), 7.24 (d, *J =* 7.89 Hz, 1H), 7.14 (s, 1H), 7.07 (d, *J* = 7.89 Hz, 1H), 6.77 (dd, *J =* 1.75, 3.51 Hz, 1H), 5.84 - 5.94 (m, 1H), 3.04 - 3.20 (m, H1), 2.94 (td, *J=* 7.67, 15.35 Hz, 1H), 2.56 - 2.70 (m, 1H), 2.28 - 2.42 (m, 1H).

**Step 7: Separation of isomers of N-(1-(5-chloro-2,3-dihydro-1H-inden-1-yl)-1H-pyrazol-4-yl)-5-(furan-2-yl)isoxazole-3-carboxamide.** The enantiomers of N-(1-(1-(5-chloro-2,3-dihydro-1H-inden-1-yl)ethyl)-1H-pyrazol-4-yl)-5-(furan-2-yl)isoxazole-3-carboxamide (50 mg, elution time 14.12 min & 15.87 min) were separated by chiral SFC (Daicel Chiralpak-IC 250 ×20 mm, 5µm). Isocratic program with analytical grade liquid carbon dioxide and HPLC grade methanol, total flow:4 g/min, Co-Solvent Percentage: 40% to obtain a first-eluting enantiomer, **Enantiomer A** (8 mg), **LCMS:** 395 [M+H] ⁺, **¹H NMR (400 MHz, DMSO-*d*₆)** δ 11.01 (s, 1H), 8.07 (s, 1H), 8.00 (s, 1H), 7.66 (s, 1H), 7.42 (s, 1H), 7.28 (d, *J* = 3.07 Hz, 1H), 7.24 (d, *J* = 7.89 Hz, 1H), 7.14 (s, 1H), 7.07 (d, *J* = 7.89 Hz, 1H), 6.77 (dd, *J =* 1.75, 3.51 Hz, 1H), 5.84 - 5.94 (m, 1H), 3.04 - 3.20 (m, 1H), 2.94 (td, *J =* 7.67, 15.35 Hz, 1H), 2.56 - 2.70 (m, 1H), 2.28 - 2.42 (m, 1H) and a second-eluting enantiomer, **Enantiomer B** (8 mg), **LCMS:** 395 [M+H]⁺, **¹H NMR (400 MHz, DMSO-*d*₆)** δ 11.01 (s, 1H), 8.07 (s, 1H), 8.00 (s, 1H), 7.66 (s, 1H), 7.43 (s, 1H), 7.28 (d, J = 3.07 Hz, 1H), 7.24 (d, J = 10.09 Hz, 1H), 7.14 (s, 1H), 7.07 (d, J = 7.89 Hz, 1H), 6.77 (dd, J = 1.75, 3.51 Hz, 1H), 5.88 - 5.94 (m, 1H), 3.07 - 3.20 (m, 1H), 2.94 (td, J = 7.73, 15.68 Hz, 1H), 2.55 - 2.69 (m, 1H), 2.29 - 2.44 (m, 1H).

### Reference Example S1-3. Synthesis of 5-(furan-2-yl)-N-(1-(1,2,3,4-tetrahydronaphthalen-1-yl)-1H-pyrazol-4-yl)isoxazole-3-carboxamide (Reference Compound 1-3).

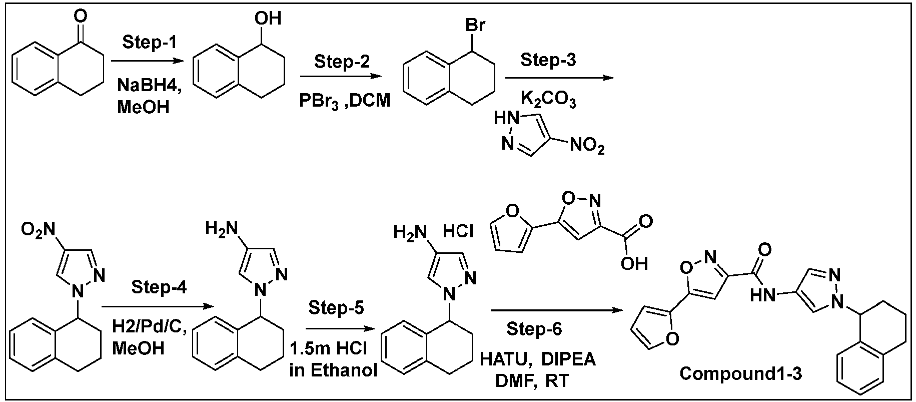

**Step 1: Synthesis of 1,2,3,4-tetrahydronaphthalen-1-ol.** To a stirred solution of 3,4-dihydronaphthalen-1(2H)-one (500 mg, 3.4 mmol, 1.0 eq) in methanol (20 mL) was added NaBH₄ (120 mg, 5.1 mmol, 1.5 eq) portion wise at 0°C and stirred for 10 minutes. The reaction mixture was allowed to stir for 1 hour at RT. Product formation was confirmed by TLC and NMR. After completion of reaction, the reaction mixture was quenched with water and extracted with ethyl acetate (3 x 50 mL). Combined organic extracts were washed with water (2 x 50 mL), dried over anhydrous Na₂SO₄ and concentrated under reduced pressure to obtain 1, 2, 3, 4-tetrahydronaphthalen-1-ol (500 mg).

**Step 2: Synthesis of 1-bromo-1,2,3,4-tetrahydronaphthalene.** To a stirred solution of 1, 2, 3, 4-tetrahydronaphthalen-1-ol (500 mg, 3.37 mmole, 1 eq.) in DCM (10 mL) was added PBr₃ (1098 mg, 4.05 mmol, 1.2 eq.) drop wise at zero degree Celsius. After that the reaction mixture was stirred at RT for 2 hr. Product formation was confirmed with TLC and ¹H NMR. After completion of reaction mixture were diluted with EtOAc (50 mL) and washed with water (50 mL x 3). Organic layer dried over Na₂SO₄ and concentrated under reduced pressure to obtained crude which was further purified by flash column chromatography to obtain pure 1-bromo-1,2,3,4-tetrahydronaphthalene (632 mg crude, as brown liquid).
**¹H NMR (400 MHz, DMSO-d₆):** δ 8.61 (d, *J =* 5.26 Hz, 1H), 8.15 (d, *J =* 7.45 Hz, 2H), 7.50 - 7.63 (m, 1H), 5.88 (d, *J=* 4.38 Hz, 1H), , 3.18 - 3.34 (m, 2H), 3.01 - 3.15 (m, 2H), 2.76 (td, *J* = 7.56, 14.69 Hz, 2H)

**Step 3: Synthesis of 4-nitro-1-(1,2,3,4-tetrahydronaphthalen-1-yl)-1H-pyrazole.** To a solution of 4-nitro-1H-pyrazole (161.4 mg, 1.428 mmol, 1 eq.) in DMF (1 mL), were added K₂CO₃ (295.71 mg, 2.14 mmol, 1.5 eq.) at zero degree Celsius. After stirring for 15 minutes, the mixture was treated dropwise with a solution of the 1-bromo-1,2,3,4-tetrahydronaphthalene (300 mg, 1.428 mmol, 1 eq.) in DMF (1 mL). The reaction mixture was kept under stirring for 24 h. Product formation was confirmed with TLC and LCMS and reaction mixture was diluted EtOAc (50 mL) and washed with water (2 x 50 mL). The organic layer was dried over Na₂SO₄ and concentrated under reduced pressure to obtain crude which was further purified by flash column chromatography to obtain pure 4-nitro-1-(1,2,3,4-tetrahydronaphthalen-1-yl)-1H-pyrazole (250 mg, as white solid), **¹H NMR (400 MHz, DMSO-d₆):** ¹H NMR (400 MHz, DMSO-d₆) δ 8.98 (s, 1H), 8.26 (s, 1H), 7.26 - 7.41 (m, 2H), 7.11 - 7.26 (m, 2H), 5.93 - 6.06 (m, 1H), 3.07 - 3.20 (m, 1H), 2.90 - 3.03 (m, 1H), 2.60 - 2.71 (m, 2H), 2.40 - 2.48 (m, 2H).

**Step 4: Synthesis of 1-(1,2,3,4-tetrahydronaphthalen-1-yl)-1H-pyrazol-4-amine.** To a stirred solution of 4-nitro-1-(1,2,3,4-tetrahydronaphthalen-1-yl)-1H-pyrazole (150 mg, 0.617 mmol, 1 equiv.) in 10 mL EtOH/ water (1:1), Fe (169.7 mg, 3.085 mmol, 5 equiv.) and ammonium chloride (172 mg, 3.085mmol, 5 equiv.) was added and allowed to heat at 80°C for 2 hr. Reaction progress was monitored by TLC and LCMS. After reaction completion, reaction mixture was filtered through Celite pad and filtrate was evaporated and extracted with DCM (100 ml x 2), Organic layer was collected and evaporated under reduced pressure to give 1-(1,2,3,4-tetrahydronaphthalen-1-yl)-1H-pyrazol-4-amine (121 mg), **LCMS:** 214 [M+H] ⁺, **¹H NMR (400 MHz, DMSO-d₆):** ¹H NMR (400 MHz, DMSO-d₆) δ 7.38 (s, 1H), 7.16 - 7.28 (m, 1H), 6.92 - 7.07 (m, 4H), 5.57 - 5.74 (m, 1H), 3.90 - 3.82 (br, 2H), 2.89 - 3.10 (m, 2H), 2.83 - 2.89 (m, 2H), 2.25 - 2.39 (m, 2H).

**Step 5: Synthesis of 1-(1,2,3,4-tetrahydronaphthalen-1-yl)-1H-pyrazol-4-amine hydrochloride.** To a solution of 1-(5-chloro-2,3-dihydro-1H-inden-1-yl)-1H-pyrazol-4-amine (120 mg.) in ethanol (1 mL), was added 4M HCl in ethanol (2 ml). Product formation was confirmed with TLC and ¹HNMR. The reaction mixture was concentrated under reduced pressure to obtain crude which was further triturated with diethylether and lyophilized to obtain pure product 1-(1,2,3,4-tetrahydronaphthalen-1-yl)-1H-pyrazol-4-amine hydrochloride (123 mg, brown solid), **¹H NMR (400 MHz, DMSO-d₆):** δ 10.16 (br. s., 2H), 7.81 (s, 1H), 7.58 (s, 1H), 7.14 - 7.27 (m, 2H), 7.11 (t, *J =* 7.24 Hz, 1H), 6.73 (d, *J =* 7.89 Hz, 1H), 5.63 (t, *J =* 6.58 Hz, 1H), 2.69 - 2.94 (m, 2H), 2.10 - 2.26 (m, 2H), 1.71 - 1.91 (m, 2H).

**Step 6: Synthesis of 5-(furan-2-yl)-N-(1-(1, 2, 3, 4-tetrahydronaphthalen-1-yl)-1H-pyrazol-4-yl) isoxazole-3-carboxamide.** To a solution of 5-(furan-2-yl) isoxazole-3-carboxylic acid (50 mg, 0.277 mmol, 1 eq) in DMF (1 mL), were added HATU (105.8 mg, 0.277 mmol, 1 eq). The mixture was treated drop wise with DIPEA (107.5 mg, 0.833 mmol, 3 eq). After stirring at RT for 15 minutes, the mixture was treated drop wise with a solution of 1-(1,2,3,4-tetrahydronaphthalen-1-yl)-1H-pyrazol-4-amine hydrochloride (68.88 mg, 0.277 mmol, 1 eq) in DMF (1 mL). The reaction mixture was kept under stirring for 24 h. Product formation was confirmed with TLC and LCMS and reaction mixture was diluted EtOAc (50 mL) and washed with water (2x50 mL). The organic layer was dried over Na₂SO₄ and concentrated under reduced pressure to obtain crude which was further purified by flash column chromatography to obtain pure 5-(furan-2-yl)-N-(1-(1,2,3,4-tetrahydronaphthalen-1-yl)-1H-pyrazol-4-yl)isoxazole-3-carboxamide (40 mg, 42.59 % as white solid), **LCMS:** 375 [M+H] ⁺, **¹H NMR (400 MHz, DMSO-d₆):** δ 11.00 (s, 1H), 8.00 (s, 1H), 7.89 (s, 1H), 7.69 (s, 1H), 7.23 - 7.32 (m, 1H), 7.15 - 7.23 (m, 2H), 7.04 - 7.15 (m, 2H), 6.68 - 6.83 (m, 2H), 5.52 - 5.66 (m, 1H), 2.84 - 3.00 (m, 1H), 2.71 - 2.82 (m, 1H), 2.17 (br. s., 2H), 1.84 (br. s, 2H).

### Example SI-4. Synthesis of N-(1-(1-(2-chloro-6-fluorophenyl)ethyl)-1H-ppyrazol-4-yl)-5-(furan-2-yl)isoxazole-3-carboxamide (Compound 1-4).

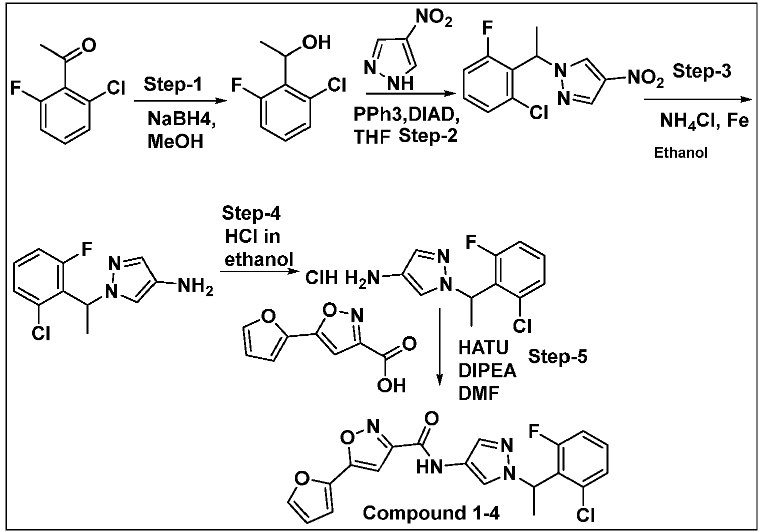

**Step 1: Synthesis of 1-(2-chloro-6-fluorophenyl)ethan-1-ol.** To a stirred solution of 1-(2-chloro-6-fluorophenyl)ethan-1-one (1 gm, 5.8 mmol, 1.0 equiv) in methanol (5 mL) was added NaBH₄ (465 mg, 12.3 mmol, 2 equiv ) portion wise at 0°C and stirred for 10 minutes. The reaction mixture was allowed to stir for 1 hour at RT. Product formation was confirmed by TLC & LCMS. After completion of the reaction, the reaction mixture was quenched with water and extracted with ethyl acetate (50 mL x 3). The combined organic extracts were washed with water (50 mL X 2), dried over anhydrous Na₂SO₄ and concentrated under reduced pressure to obtain 1-(2-chloro-6-fluorophenyl)ethan-1-ol (1 gm, as colourless liquid).

**Step 2: Synthesis of 1-(1-(2-chloro-6-fluorophenyl)ethyl)-4-nitro-1H-pyrazole.** To a stirred solution of PPh₃ (903 mg, 3.44 mmol, 1.5 equiv) and DIAD (0.669 ml, 3.44 mmol, 1.0 equiv) in THF (2mL) was added 4-nitro-1H-pyrazole (260 mg, 2.29 mmol, 1 equiv), and followed by the addition of 1-(2-chloro-6-fluorophenyl)ethan-1-ol (400 mg, 2.29 mmol, 1.0 equiv). The resultant reaction mixture was stirred at RT for 1 h. Product formation was confirmed with TLC & LCMS. After completion of reaction mixture were diluted with EtOAc (50 mL) & washed with water (50 mL x 3). The organic layer was dried over Na₂SO₄ & concentrated under reduced pressure to obtain crude which was further purified by flash column chromatography to obtain pure product 1-(1-(2-chloro-6-fluorophenyl)ethyl)-4-nitro-1H-pyrazole (320 mg).

**Step 3: Synthesis of 1-(1-(2-chloro-6-fluorophenyl)ethyl)-1H-pyrazol-4-amine.** To a stirred solution of 1-(1-(2-chloro-6-fluorophenyl)ethyl)-4-nitro-1H-pyrazole (200 mg, 0.743 mmol, 1 equiv.) in 10 mL EtOH/water (1:1), Fe (200 mg, 3.71 mmol, 5 equiv.) and ammonium chloride (208 mg, 3.71 mmol, 5 equiv. ) was added and allowed to heat at 80°C for 2 hr. Reaction progress was monitored by TLC and LCMS. After reaction completion, the reaction mixture was filtered through a Celite pad and filtrate was evaporated and extracted with DCM/ water 2 times. The organic layer was collected and evaporated under reduced pressure to 1-(1-(2-chloro-6-fluorophenyl)ethyl)-1H-pyrazol-4-amine (170 mg), **LCMS:** 240 [M+H] ⁺.

**Step 4: Synthesis of 1-(1-(2-chloro-6-fluorophenyl)ethyl)-1H-pyrazol-4-amine hydrochloride.** To a stirred solution of 1-(1-(2-chloro-6-fluorophenyl)ethyl)-1H-pyrazol-4-amine (150 mg) in ethanol was added HCl in ethanol (1 mL) and allowed to stir at RT for 1 hr. After 1 hour reaction the mixture was evaporated and lyophilised to give product 1-(1-(2-chloro-6-fluorophenyl)ethyl)-1H-pyrazol-4-amine hydrochloride (155 mg). **LCMS:** 240 [M+H] ⁺.

**Step 5: Synthesis of N-(1-(1-(2-chloro-6-fluorophenyl)ethyl)-1H-pyrazol-4-yl)-5-(furan-2-yl)isoxazole-3-carboxamide.** To a solution of 5-(furan-2-yl) isoxazole-3-carboxylic acid (50 mg, 0.277 mmol, 1 equiv) in DMF (1 mL) was added HATU (105.5 mg, 0.277 mmol, 1.0 equiv) and DIPEA (107.99 ml, 0.831 mmol, 3.0 equiv). After stirring at room temperature for 15 minutes a solution of the 1-(1-(2-chloro-6-fluorophenyl)ethyl)-1H-pyrazol-4-amine hydrochloride (77 mg, 0.555 mmol, 1 equiv) in DMF (1 mL) was added. The reaction mixture was kept under stirring for 24 h. After completion of reaction mixture were diluted with EtOAc (50 mL) & washed with water (10 mL x 3). The organic layer was dried over Na₂SO₄ & concentrated under reduced pressure to obtain crude which was further purified by trituration with acetone:hexane (8:2) to afford precipitate as N-(1-(1-(2-chloro-6-fluorophenyl)ethyl)-1H-pyrazol-4-yl)-5-(furan-2-yl)isoxazole-3-carboxamide (30 mg). **LCMS:** 401 [M+H] ⁺, **¹H NMR (400 MHz, DMSO-d₆):** δ 11.01 (s, 1H), 8.14 (s, 1H), 8.00 (d, *J* = 1.32 Hz, 1H), 7.64 (s, 1H), 7.35 - 7.48 (m, 2H), 7.19 - 7.33 (m, 2H), 7.14 (s, 1H), 6.77 (dd, *J* = 1.75, 3.51 Hz, 1H), 6.02 (q, *J =* 7.31 Hz, 1H), 1.92 (d, *J =* 7.45 Hz, 3H).

### Reference Example SI-5. Synthesis of N-(1-(1-(2,4-difluorophenyl)ethyl)-1H-ppyrazol-4-yl)-5-(furan-2-yl)isoxazole-3-carboxamide (Reference Compound 1-5)

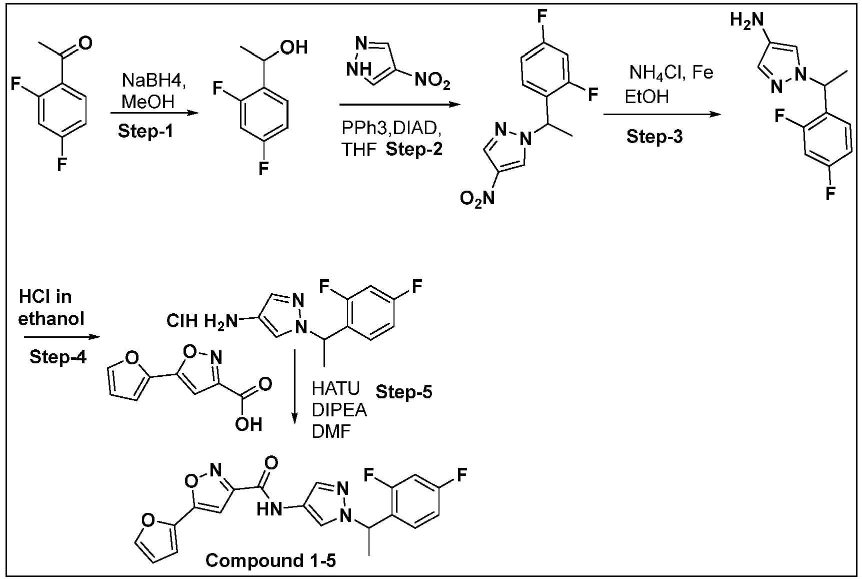

**Step 1: Synthesis of 1-(2,4-difluorophenyl)ethan-1-ol.** To a stirred solution of 1-(2,4-difluorophenyl)ethan-1-one (1 gm, 6.4 mmol, 1.0 equiv) in methanol (5 mL) was added NaBH₄ (465 mg, 12.3 mmol, 2 equiv ) portion wise at 0°C and stirred for 10 minutes. The reaction mixture was allowed to stir for 1 hour at RT. Product formation was confirmed by TLC & LCMS. After completion of reaction, the reaction mixture was quenched with water and extracted with ethyl acetate (50 mL x 3). The combined organic extracts were washed with water (50 mL x 2), dried over anhydrous Na₂SO₄ and concentrated under reduced pressure to obtain 1-(2,4-difluorophenyl)ethan-1-ol (1 gm, as colourless liquid), **¹H NMR (400 MHz, DMSO-d₆):** δ 7.45 - 7.61 (m, 1H), 7.00 - 7.20 (m, 2H), 5.33 (d, *J* = 4.38 Hz, 1H), 4.87 - 5.00 (m, 1H), 1.31 (d, *J* = 6.58 Hz, 3H).

**Step 2: Synthesis of 1-(1-(2,4-difluorophenyl)ethyl)-4-nitro-1H-pyrazole.** To a stirred solution of PPh₃ (678 mg, 2.53 mmol, 1 equiv) and DIAD (0.511 ml, 2.53 mmol, 1.0 equiv) in THF (2mL), was added 4-nitro-1H-pyrazole (286 mg, 2.53 mmol, 1 equiv), followed by the addition of 1-(2,4-difluorophenyl)ethan-1-ol (400 mg, 2.53 mmol, 1.0 equiv). The resultant reaction mixture was stirred at RT for 1 h. Product formation was confirmed with TLC & LCMS. After completion of reaction the mixture was diluted with EtOAc (50 mL) & washed with water (50 mL x 3). The organic layer was dried over Na₂SO₄ & concentrated under reduced pressure to obtain crude which was further purified by flash column chromatography to obtain pure product 1-(1-(2,4-difluorophenyl)ethyl)-4-nitro-1H-pyrazole (170 mg), **¹H NMR (400 MHz, DMSO-*d*₆):** δ 9.10 (s, 1H), 8.30 (s, 1H), 7.51 (dd, *J* = 2.63, 8.77 Hz, 1H), 7.42 (dd, *J* = 6.14, 8.77 Hz, 1H), 7.26 (dt, *J =* 2.41, 8.44 Hz, 1H), 6.00 (q, *J* = 6.87 Hz, 1H), 1.83 (d, *J* = 7.02 Hz, 3H).

**Step 3: Synthesis of 1-(1-(2,4-difluorophenyl)ethyl)-1H-pyrazol-4-amine.** To a stirred solution of 1-(1-(2,4-difluorophenyl)ethyl)-4-nitro-1H-pyrazole (100 mg, 0.395 mmol, 1 equiv.) in 10 mL EtOH/water (5:5) was added Fe (106 mg, 1.976 mmol, 5 equiv.) and ammonium chloride (111 mg, 1.976 mmol, 5 equiv. ) and allowed to heat at 80°C for 2 hr. Reaction progress was monitored by TLC and LCMS. After reaction completion, the reaction mixture was filtered through a Celite pad and filtrate was evaporated and extracted with DCM/ water 2 times. The organic layer was collected and evaporated under reduced pressure to 1-(1-(2,4-difluorophenyl)ethyl)-1H-pyrazol-4-amine (89 mg). **LCMS:** 224[M+H] ⁺.

**Step 4: Synthesis of 1-(1-(2,4-difluorophenyl)ethyl)-1H-pyrazol-4-amine** hydrochloride. To a stirred solution of 1-(1-(2,4-difluorophenyl)ethyl)-1H-pyrazol-4-amine (89 mg) in ethanol was added HCl in ethanol (1 mL) and allowed to stir at RT for 1 hr. After the reaction completion, the reaction mixture was evaporated and lyophilised to give product 1-(1-(2,4-difluorophenyl)ethyl)-1H-pyrazol-4-amine hydrochloride (60 mg), **LCMS:** 224 [M+H] ⁺.

**Step 5: Synthesis of N-(1-(1-(2,4-difluorophenyl)ethyl)-1H-pyrazol-4-yl)-5-(furan-2-yl)isoxazole-3-carboxamide.** To a solution of 5-(furan-2-yl)isoxazole-3-carboxylic acid (31.09 mg, 0.171 mmol, 1 equiv) in DMF (1 mL), were added HATU (65.2 mg, 0.171 mmol, 1.0 equiv) and DIPEA (0.089 ml, 0.515 mmol, 3.0 equiv). After stirring at RT for 15 minutes a solution of 1-(1-(2,4-difluorophenyl)ethyl)-1H-pyrazol-4-amine hydrochloride (50 mg, 0.171 mmol, 1 equiv) in DMF (1 mL) was added drop wise. The reaction mixture was kept under stirring for 24 h. After completion of reaction, the mixture was diluted with EtOAc (50 mL) & washed with water (10 mL x 3). The organic layer was dried over Na₂SO₄ & concentrated under reduced pressure to obtain crude which was further purified by trituration with acetone: hexane (8:2) ml to afford precipitate as N-(1-(1-(2,4-difluorophenyl)ethyl)-1H-pyrazol-4-yl)-5-(furan-2-yl)isoxazole-3-carboxamide (30 mg) LCMS: 385 [M+H] ⁺, **¹H NMR (400 MHz, DMSO-*d*₆):** δ 11.02 (s, 1H), 8.14 (s, 1H), 8.00 (d, *J* = 0.88 Hz, 1H), 7.69 (s, 1H), 7.22 - 7.38 (m, 3H), 7.14 (s, 1H), 7.03 - 7.10 (m, 1H), 6.77 (dd, *J* = 1.75, 3.51 Hz, 1H), 5.85 (q, *J =* 7.02 Hz, 1H), 1.80 (d, *J =* 7.02 Hz, 3H).

### Reference Example SI-6. Synthesis of N-(1-(1-(2-chloro-4-fluorophenyl)ethyl)-1H-pyrazol-4-yl)-5-(furan-2-yl)isoxazole-3-carboxamide (Reference Compound 1-6).

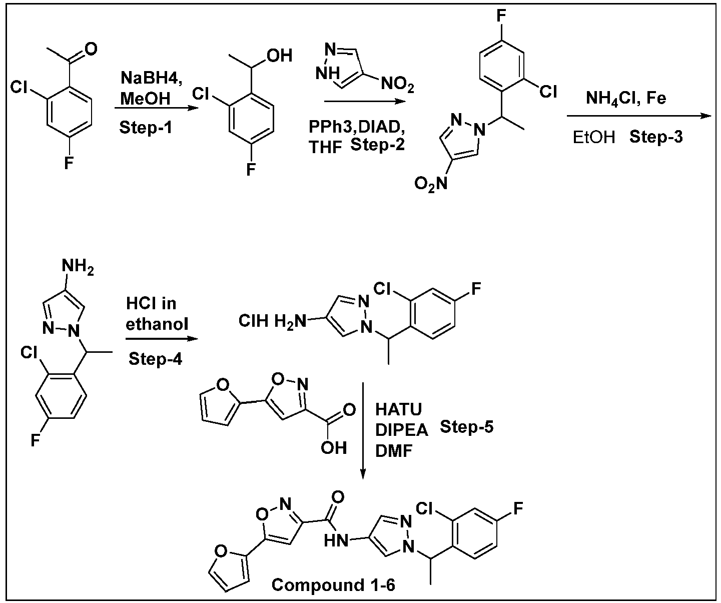

**Step 1: Synthesis of 1-(2-chloro-4-fluorophenyl)ethan-1-ol.** To a stirred solution of 1-(2-chloro-4-fluorophenyl)ethan-1-oneone (0.5 gm, 3.205 mmol, 1.0 equiv) in methanol (5 mL) was added NaBH₄ (183 mg, 4.807 mmol, 1.5 equiv ) portion wise at 0°C and stirred for 10 minutes. The reaction mixture was allowed to stir for 1 hour at RT. Product formation was confirmed by TLC & LCMS. After completion of the reaction, the reaction mixture was quenched with water and extracted with ethyl acetate (50 mL x 3), Combined organic extracts were washed with water (50 mL x 2), dried over anhydrous Na₂SO₄ and concentrated under reduced pressure to obtain 1-(2-chloro-4-fluorophenyl)ethan-1-ol (0.4 gm., as colourless liquid), **¹H NMR (400 MHz, DMSO-*d*₆):** δ 7.43 - 7.64 (m, 1H), 6.97 - 7.19 (m, 2H), 5.33 (d, *J* = 4.38 Hz, 1H), 4.84 - 5.00 (m, 1H), 1.31 (d, *J* = 6.58 Hz, 3H).

**Step 2: Synthesis of 1-(1-(2-chloro-4-fluorophenyl)ethyl)-4-nitro-1H-pyrazole.** To a stirred solution of PPh₃ (611 mg, 2.28 mmol, 1equiv) and DIAD (0.460 mg, 2.29 mmol, 1.0 equiv) in THF (2mL), was added 4-nitro-1H-pyrazole (260 mg, 2.29 mmol, 1 equiv), followed by addition of 1-(2-chloro-4-fluorophenyl)ethan-1-ol (400 mg, 2.29 mmol, 1.0 equiv). The resultant reaction mixture was stirred at RT for 1 h. Product formation was confirmed with TLC & LCMS. After completion of reaction the mixture was diluted with EtOAc (50 mL) & washed with water (50 mL x 3), The organic layer was dried over Na₂SO₄ & concentrated under reduced pressure to obtain crude which was further purified by flash column chromatography to obtain pure product 1-(1-(2-chloro-4-fluorophenyl)ethyl)-4-nitro-1H-pyrazole ( 220 mg), **¹H NMR (400 MHz, DMSO-d₆):** δ 9.10 (s, 1H), 8.30 (s, 1H), 7.51 (dd, *J* = 2.63, 8.77 Hz, 1H), 7.42 (dd, *J =* 6.14, 8.77 Hz, 1H), 7.26 (dt, *J* = 2.41, 8.44 Hz, 1H), 6.00 (q, *J* = 6.87 Hz, 1H), 1.83 (d, *J* = 7.02 Hz, 3H).

**Step 3: Synthesis of 1-(1-(2-chloro-4-fluorophenyl)ethyl)-1H-pyrazol-4-amine.** To a stirred solution of 1-(1-(2-chloro-4-fluorophenyl)ethyl)-4-nitro-1H-pyrazole (100 mg, 0.395 mmol, 1 equiv.) in 10 mL EtOH/ water (1:1) was added Fe (107 mg, 1.97 mmol, 5 equiv.) and ammonium chloride (111 mg, 1.976 mmol, 5 equiv.) and allowed to heat at 80 °C for 2 hr. Reaction progress was monitored by TLC and LCMS. After reaction completion, the reaction mixture was filtered through a Celite pad and filtrate was evaporated and extracted with DCM/ water 2 times. The organic layer was collected and evaporated under reduced pressure to 1-(1-(2-chloro-4-fluorophenyl)ethyl)-1H-pyrazol-4-amine (89 mg), **¹H NMR (400 MHz, DMSO-*d*₆):** δ 7.15 - 7.24 (m, 1H), 6.97 - 7.15 (m, 2H), 6.93 (s, 1H), 5.74 (br. s., 1H), 5.59 (m, *J* = 6.58 Hz, 1H), 3.82 (br. s., 2H), 1.68 (d, *J* = 7.45 Hz, 3H).

**Step 4: Synthesis of 1-(1-(2-chloro-4-fluorophenyl)ethyl)-1H-pyrazol-4-aminehydrochloride.** To a stirred solution 1-(1-(2-chloro-4-fluorophenyl)ethyl)-1H-pyrazol-4-amine (118 mg,) in ethanol was added HCl in ethanol (2 mL) and allowed to stir at room temperature for 1 hr. The reaction mixture was evaporated and lyophilised to give product 1-(1-(2-chloro-4-fluorophenyl)ethyl)-1H-pyrazol-4-amine hydrochloride (80 mg). **¹H NMR (400 MHz, DMSO-*d*₆):** δ 9.9 (s, 2H), 8.04 (s, 1H), 7.60 (s, 1H), 7.49 (dd, *J* = 2.63, 8.77 Hz, 1H), 7.18 - 7.41 (m, 2H), 5.95 (q, *J* = 6.87 Hz, 1H), 1.78 (d, *J=* 7.02 Hz, 3H).

**Step 5: Synthesis of N-(1-(1-(2-chloro-4-fluorophenyl)ethyl)-1H-pyrazol-4-yl)-5-(furan-2-yl)isoxazole-3-carboxamide.** To a solution of 5-(furan-2-yl)isoxazole-3-carboxylic acid (32.72 mg, 0.18 1mmol, 1 equiv) in DMF (1 mL), were added HATU (69.72 mg, 0.181 mmol, 1.0 equiv) and DIPEA (0.094 ml, 0.54 mmol, 3.0 equiv). After stirring at RT for 15minutes, a solution of the 1-(1-(2-chloro-4-fluorophenyl)ethyl)-1H-pyrazol-4-amine hydrochloride (50 mg, 0.181 mmol, 1 equiv) in DMF (1 mL) was added. The reaction mixture was kept under stirring for 24 h. After completion of reaction the mixture were diluted with EtOAc (50 mL) & washed with water (10 mL x 3). The organic layer was dried over Na₂SO₄ & concentrated under reduced pressure to obtain crude which was further purified by trituration with acetone: hexane (8:2)ml to afford a precipitate as N-(1-(1-(2-chloro-4-fluorophenyl)ethyl)-1H-pyrazol-4-yl)-5-(furan-2-yl)isoxazole-3-carboxamide (30 mg). **LCMS:** 401 [M+H] ⁺, **¹H NMR (400 MHz, DMSO-*d*₆):** δ 11.04 (s, 1H), 8.14 (s, 1H), 8.00 (d, *J* = 1.75 Hz, 1H), 7.71 (s, 1H), 7.46 - 7.55 (m, 1H), 7.19 - 7.30 (m, 3H), 7.14 (s, 1H), 6.77 (dd, *J* = 1.75, 3.51 Hz, 1H), 5.91 (q, *J* = 6.87 Hz, 1H), 1.79 (d, *J* = 7.02 Hz, 3H).

### Reference Example S1-7. Synthesis of N-(1-(1-(2,4-bis(trifluoromethyl)phenyl)ethyl)-lH-pyrazol-4-yl)-3-(pyrazin-2-yl)isoxazole-5-carboxamide (Reference Compound 1-11).

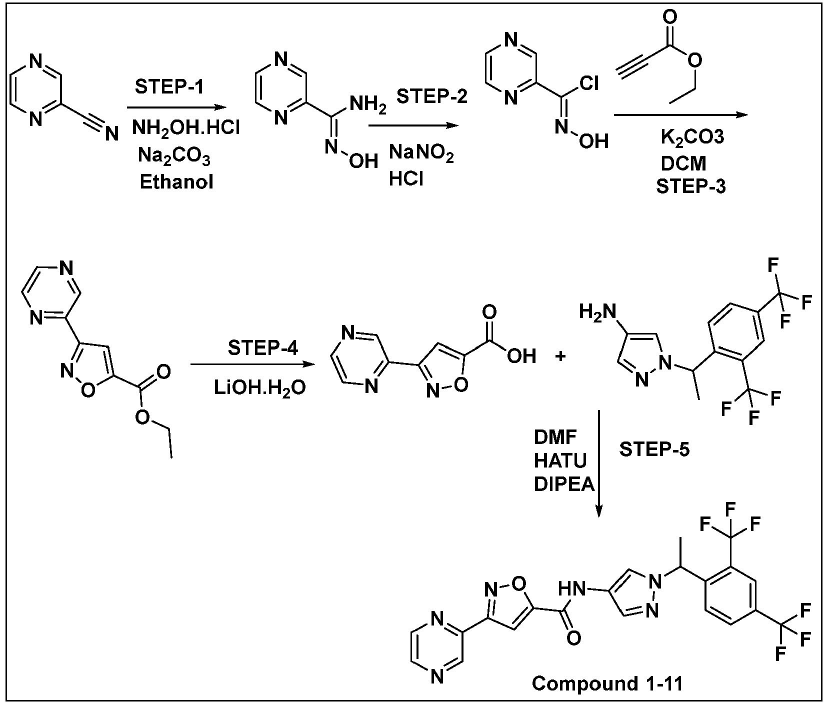

**Step 1: Synthesis of N'-hydroxypyrazine-2-carboximidamide.** To a mixture of pyrazine-2-carbonitrile (1 gm, 9.5 mmol, and 1 eq) was added NH₂OH.HCl (1.32 gm, 0.018 mole, 2 eq) in ethanol: water (10:10) ml was added Na₂CO₃ (2 gm, 0.018 mole, 2 eq). Reaction mixture was refluxed for 16 hours. Reaction mixture was cooled to RT. Obtained suspension was filtered out. Obtained precipitate was confirmed as our product N'-hydroxypyrazine-2-carboximidamide (1.1 gm, White precipitate). **¹H NMR (400 MHz, DMSO-*d*₆)** δ 10.23 (s, 1H), 9.06 (d, *J* = 0.88 Hz, 1H), 8.63 (q, *J* = 2.63 Hz, 2H), 5.95 (br. s., 2H).

**Step 2: Synthesis of N-hydroxypyrazine-2-carbimidoyl chloride.** To a solution of N'-hydroxypyrazine-2-carboximidamide (200 mg. 1.449 mmol, and 1 eq) in H₂O (5 ml) at zero degree Celsius was added NaCl (256.34 mg. 4.34 mmol, and 3eq), 0.1 N HCl (0.4 ml) and acetic acid (1 ml). Reaction mixture was stirred until it became transparent. To the reaction mixture was added NaNO₂ (100 mg, 1.449 mmole, 1 eq) and stirred at zero degree Celsius for half an hour and the obtained suspension was filtered out. Obtained precipitate was confirmed as our product N-hydroxypyrazine-2-carbimidoyl chloride (180 mg, white precipitate). **¹H NMR (400 MHz, DMSO-*d*₆)** δ13.04 (s, 1H), 9.09 (s, 1H), 8.70 - 8.78 (m, 2H).

**Step 3: Synthesis of ethyl 3-(pyrazin-2-yl)isoxazole-5-carboxylate.** To a mixture of N-hydroxypyrazine-2-carbimidoyl chloride (100 mg, 0.632 mmol, 1 eq) and ethyl propionate (124.05 mg, 1.265 m mole, 2eq) in DCM (20 mL) at room temperature was added a solution of K₂CO₃ (218.3 mg., 2.5 m mole, 2eq) in DCM (20 mL) portion wise over 60 minutes. The reaction mixture was stirred for 16 h at room temperature. The reaction mixture was concentrated to dryness, and work up was done by adding water (10 ml) and extracted with ethyl acetate (2 x 25 ml x 2 times) which was purified by flash silica gel chromatography by using a mixture of ethyl acetate in hexane to afford ethyl 3-(pyrazin-2-yl)isoxazole-5-carboxylateas a white solid (80 mg). **LCMS:** 220 [M+H] ⁺, **¹H NMR (400 MHz, DMSO-*d*₆)** δ 9.27 - 9.39 (m, 1H), 8.76 - 8.92 (m, 2H), 7.82 (s, 1H), 4.42 (q, *J =* 7.02 Hz, 2H), 1.36 (t, *J* = 7.24 Hz, H 3H).

**Step 4: Synthesis of 3-(pyrazin-2-yl)isoxazole-5-carboxylic acid.** To a solution of ethyl 3-(pyrazin-2-yl)isoxazole-5-carboxylate (60 mg, 0.272 mmol, 1 eq) in THF (2 mL) and water (2 mL) was slowly added lithium hydroxide (13.05 mg, 0.326 mmol, 1.2 eq). The resulting mixture was stirred for 16 hrs. Reaction mixture was concentrated under reduced pressure to obtain crude which was acidified with 1N HCl and obtained suspension was lyophilized. Obtained crude was triturated with ether. Obtained ppt. 3-(pyrazin-2-yl)isoxazole-5-carboxylic acid was our product (52 mg, off white solid). **LCMS:** 192 [M+H]⁺.

**Step 5: Synthesis of N-(1-(1-(2,4-bis(trifluoromethyl)phenyl)ethyl)-1H-pyrazol-4-yl)-3-(pyrazin-2-yl)isoxazole-5-carboxamide.** To a solution of 3-(pyrazin-2-yl)isoxazole-5-carboxylic acid (50 mg, 0.2613 mmol, 1 equiv) in DMF (2 mL), were added HATU (100.2 mg, 0.261 mmol, 1 equiv). The mixture was treated drop wise with DIPEA (0.183 ml, 0.783 mmol, 3 equiv). After stirring at RT for 15minutes, the mixture was treated drop wise with a solution of 1-(1-(2,4-bis(trifluoromethyl)phenyl)ethyl)-1H-pyrazol-4-amine (84.56 mg, 0.261 mmol, 1 equiv) in DMF (1 mL). The reaction mixture was kept under stirring for 24 h. The reaction mixture was diluted with water (50 mL) and the resulting precipitate was filtered off. The crude material obtained was purified by flash silica gel chromatography using a mixture of ethyl acetate in hexane and trituration with DCM : hexane (2:8) to obtained N-(1-(1-(2,4-bis(trifluoromethyl)phenyl)ethyl)-1H-pyrazol-4-yl)-3-(pyrazin-2-yl)isoxazole-5-carboxamide (40 mg). LCMS: 497[M+H]⁺, **¹H NMR (400 MHz, DMSO-*d*₆)** δ 11.23 (s, 1H), 9.32 (s, 1H), 8.85 (d, *J =* 7.02 Hz, 2H), 8.21 (s, 1H), 8.02 - 8.12 (m, 2H), 7.74 - 7.81 (m, 3H), 5.95 (m, *J* = 7.02 Hz, 1H), 1.88 (d, *J* = 6.58 Hz, 3H).

### Reference Example S1-8. Synthesis of 5-(pyrazin-2-yl)-N-(1-(5-(trifluoromethyl)-2,3-dihydro-1H-inden-1-yl)-1H-pyrazol-4-yl)isoxazole-3-carboxamide (Reference Compound 1-12).

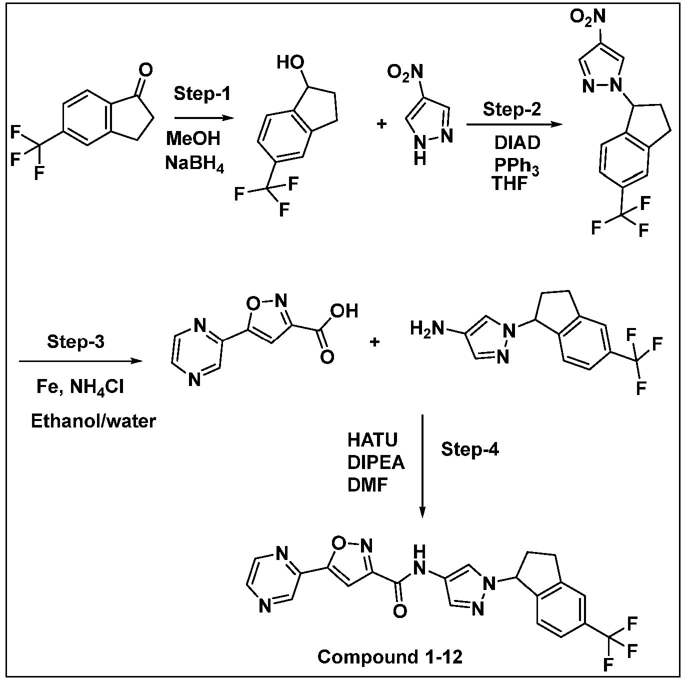

**Step 1: Synthesis of 5-(trifluoromethyl)-2,3-dihydro-1H-inden-1-ol.** To the stirred solution of 5-(trifluoromethyl)-2,3-dihydro-1H-inden-1-one (300 mg, 1.5 mmol, 1 eq.) in 5 mL MeOH, NABH₄ (88.87 mg, 2.25 mmol, 1.5 eq.) added portion wise and allowed to stir at RT for 1 hr. The reaction mixture was concentrated and extracted with ethylacetate and water (2 x 25 mL). The organic layer was collected and evaporated to give 5-(trifluoromethyl)-2,3-dihydro-1H-inden-1-ol. **¹H NMR (400 MHz, DMSO-*d*₆)** δ ppm 7.49 - 7.60 (m, 3 H) 5.46 (d, *J*=6.14 Hz, 1 H) 5.09 (q, *J*=6.43 Hz, 1 H) 2.98 (ddd, *J*=16.01, 8.55, 3.51 Hz, 2 H) 2.78 (dt, *J*=15.90, 8.06 Hz, 1 H) 2.33 - 2.43 (m, 2 H) 1.75 - 1.87 (m, 1 H).

**Step 2: Synthesis of 4-nitro-1-(5-(trifluoromethyl)-2,3-dihydro-1H-inden-1-yl)-1H-pyrazole.** To a stirred solution of PPh₃ (583.60 mg, 2.227 mmol, 1.5 eq.) and DIAD (449.95 mg, 2.227 mmol, 1.5 eq.) in THF (10 mL), 5-(trifluoromethyl)-2,3-dihydro-1H-inden-1-ol (300 mg, 1.485 mmol, 1 eq.) and 4-nitro-1H-pyrazole (167.82 mg, 1.485 mmol, 1 eq.) were added and allowed to stir at RT for 16 hr. Reaction progress was monitored by TLC and LCMS. After the reaction completion, the reaction mixture was extracted with ethyl acetate and water (2 x 25 mL). The organic layer was separated and evaporated under reduced pressure to give crude product which was further purified by using CombiFlash chromatography to obtain 4-nitro-1-(5-(trifluoromethyl)-2,3-dihydro-1H-inden-1-yl)-1H-pyrazole. **LCMS:** 297 [M+H]⁺, **¹H NMR (400 MHz, DMSO-*d*₆)** δ ppm 9.11 (s, 1 H) 8.49 (s, 1 H) 7.68 - 7.74 (m, 2 H) 7.50 (dd, *J*=5.04, 1.97 Hz, 3 H) 7.38 - 7.41 (m, 1 H).

**Step 3: Synthesis of 1-(5-(trifluoromethyl)-2,3-dihydro-1H-inden-1-yl)-1H-pyrazol-4-amine.** To the stirred solution of 4-nitro-1-(2,2,2-trifluoro-1-phenylethyl)-1H-pyrazole (200 mg, 0.738 mmol, 1 eq.) in 10 mL EtOH/ water (1:1), Fe (202.95 mg, 3.69 mmol, 5 eq.) and ammonium chloride (195.57 mg, 3.69 mmol, 5 eq.) were added and allowed to heat at 80°C for 2 hr. Reaction progress was monitored by TLC and LCMS. After reaction completion, the reaction mixture was filtered through a Celite pad and filtrate was evaporated and extracted with DCM/ water (2 x 25 mL). The organic layer was collected and evaporated under reduced pressure to give 1-(5-(trifluoromethyl)-2,3-dihydro-1H-inden-1-yl)-1H-pyrazol-4-amine. **LCMS:** 268 [M+H]⁺.

**Step 4: Synthesis of 5-(pyrazin-2-yl)-N-(1-(5-(trifluoromethyl)-2,3-dihydro-1H-inden-1-yl)-1H-pyrazol-4-yl)isoxazole-3-carboxamide.** To a stirred solution of 5-(pyrazin-2-yl) isoxazole-3-carboxylic acid (100 mg, 0.524 mmol, 1 eq.) in DMF (4 mL), HATU (199.12 mg, 0.524 mmol, 1 eq.) was added and allowed to stir at RT for 15 min. Then, a stirred solution of 1-(5-(trifluoromethyl)-2,3-dihydro-1H-inden-1-yl)-1H-pyrazol-4-amine (140 mg, 0.524 mmol, 1 eq.) and DIPEA (202.72 mg, 1.572 mmol, 3 eq.) was added. The reaction mixture was allowed to stir at RT for 18 hr. After the reaction completion, the reaction mixture was poured into ice cold water and the precipitate obtained was filtered off to obtain crude product which was purified by using CombiFlash chromatography to obtain 5-(pyrazin-2-yl)-N-(1-(5-(trifluoromethyl)-2,3-dihydro-1H-inden-1-yl)-1H-pyrazol-4-yl)isoxazole-3-carboxamide (30 mg white solid). **LCMS:** 441 [M+H]⁺, **¹H NMR (400 MHz, DMSO-*d*₆)** δ ppm 11.12 (s, 1 H) 9.35 (s, 1 H) 8.82 (s, 1 H) 8.85 (s, 1 H) 8.18 (s, 1 H) 7.65 - 7.75 (m, 3 H) 7.55 (d, *J*=7.89 Hz, 1 H) 7.24 (d, *J*=7.89 Hz, 1 H) 6.04 (t, *J*=6.58 Hz, 1 H) 3.20 (d, *J*=4.82 Hz, 2 H) 3.03 (d, J=8.33 Hz, 2 H).

### Reference Example S1-9. Synthesis of N-(1-(1-(2,6-dichlorophenyl)ethyl)-1H-pyrazol-4-yl)-5-(thiophen-2-yl)isoxazole-3-carboxamide (Reference Compound 1-13).

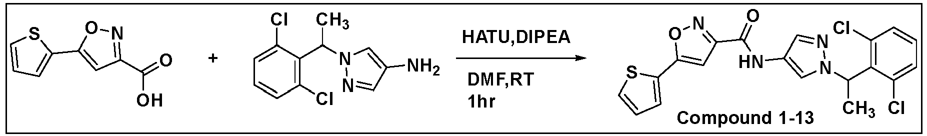

**Step 1: Synthesis of N-(1-(1-(2,6-dichlorophenyl)ethyl)-1H-pyrazol-4-yl)-5-(thiophen-2-yl)isoxazole-3-carboxamide.** To a stirred solution of 5-(thiophen-2-yl)isoxazole-3-carboxylic acid (50 mg, 0.256 mmole, 1.0 eq) in DMF (2 ml) was added HATU (97 mg, 0.256 mmole, 1.0 eq) and stirred at room temperature for 15 min. 1-(1-(2,6-dichlorophenyl)ethyl)-1H-pyrazol-4-amine (65 mg, 0.256 mmole, 1.0 eq) was added to the reaction mixture followed by the addition of DIPEA (0.13 mL, 0.769 mmole, 3.0 eq) and again stirred at room temperature for 1 hr. Progress of the reaction was analyzed by TLC, LCMS. After the completion of reaction, RM was poured into ice cold water, filtered and purified using flash chromatography to obtain N-(1-(1-(2,6-dichlorophenyl)ethyl)-1H-pyrazol-4-yl)-5-(thiophen-2-yl)isoxazole-3-carboxamide **LCMS Mass** [M+1]: 433.3, **¹H NMR** (400 MHz, DMSO-d₆) δ ppm 10.99 (s, 1 H) 8.11 (s, 1 H) 7.83 (s,1H) 7.90 (s, 1 H) 7.66 (s, 1 H) 7.38 (d, J=8.33 Hz, 2 H) 7.17 (s,1H) 7.30 (s, 2 H) 6.17 (d, J=7.02 Hz, 1 H) 1.96 (d, J=7.45 Hz, 3 H).

### Example S1-10. Synthesis of N-(1-(1-(2-chloro-6-fluorophenyl)ethyl)-1H-pyrazol-4-yl)-5-(furan-2-yl)isoxazole-3-carboxamide (Compound 1-14 and Compound 1-15).

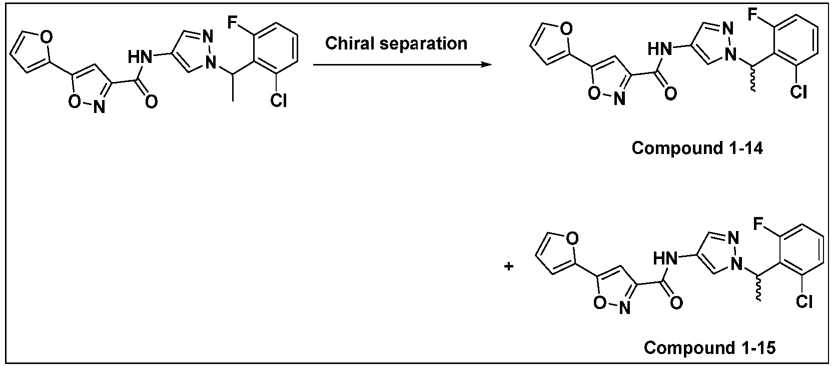

The racemic mixture of N-(1-(1-(2-chloro-6-fluorophenyl)ethyl)-1H-pyrazol-4-yl)-5-(furan-2-yl)isoxazole-3-carboxamide (30 mg) was purified by chiral HPLC to obtain N-(1-(1-(2-chloro-6-fluorophenyl)ethyl)-1H-pyrazol-4-yl)-5-(furan-2-yl)isoxazole-3-carboxamide as a first-eluting isomer **(Compound 1-14)** and a second-eluting isomer **(Compound 1-15).** The isomers were separated by chiral SFC (Daicel Chiralpak-IA, 250 x20 mm,5um). isocratic program with analytical grade liquid carbon dioxide and HPLC grade isopropanol, total flow: 56 g/min, co-Solvent Percentage: 20%.

**Compound 1-14:** yield = 4 mg; elution time = 9.01 min **Compound 1-15:** yield = 4 mg; elution time = 17.68 min.

**Compound 1-14: LCMS:** 401 [M+1]; **¹H NMR (400 MHz, DMSO-d₆)** δ 11.01 (S,1H), 8.14 (S, 1H), 8.01 (S, 1H), 7.64 (S, 1H), 7.39 (m, 2H), 7.29 (m, J = 3.51 Hz, 2H), 7.14 (5, 1H), 6.78 @5.,1H), 6.02 (m, 1H), 1.92 (d, J = 6.58 Hz, 3H). **Compound 1-15:** LCMS: 401 [M+H]⁺; **¹H NMR (400 HZ, DMSO-d₆)** m 11.01 (5, 1H), 8.14 (5, 1H), 8.00 (s, 1H), 7.64 (s, 1H), 7.36 -7.44 (m, 2H), 7.20 -7.30 (m, 2H), 7.14 (5, 1H), 6.77 (m, 1H), 6.04 (m, 1H), 1.92 (d, J = 6.14 Hz, 3H).

### Reference Example S1-11. Synthesis of N-(1-(1-(2,4-difluorophenyl)ethyl)-1H-pyrazol-4-yl)-5-(furan-2-yl)isoxazole-3-carboxamide (Reference Compound 1-16 and Reference Compound 1-17).

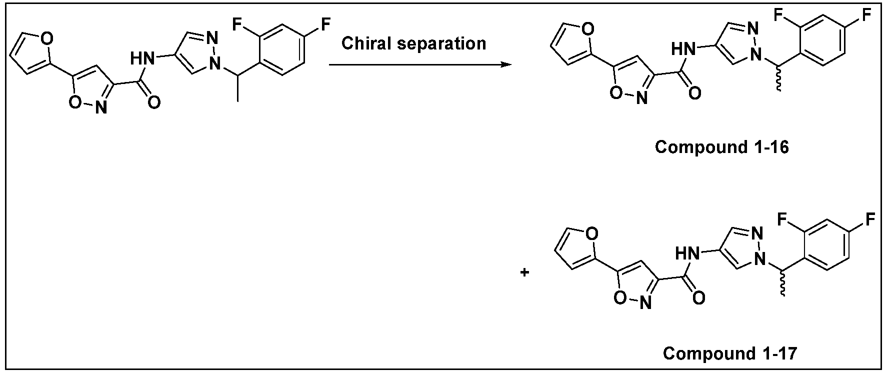

The racemic mixture of N-(1-(1-(2,4-difluorophenyl)ethyl)-1H-pyrazol-4-yl)-5-(furan-2-yl)isoxazole-3-carboxamide (30 mg) was purified by chiral HPLC to obtain N-(1-(1-(2,4-difluorophenyl)ethyl)-1H-pyrazol-4-yl)-5-(furan-2-yl)isoxazole-3-carboxamide as a first-eluting isomer (Compound 1-16) and a second-eluting isomer (Compound 1-17). The enantiomers were separated by chiral SFC(Daicel Chiralpak·-IA, 250 x20 mm, 5 um). Isocratic program with analytical grade liquid carbon dioxide and HPLC grade methanol, total flow:51g/min, co-Solvent Percentage: 15%.

**Reference Compound 1-16:** yield = 2 mg; elution time = 6.2 min. **Reference Compound 1-17:** yield = 4 mg; elution time = 7.5 min.
**Reference Compound 1-16: LCMS:** 385 [M+H]; **¹H NMR (400 MHz, DMSO-d₆)** δ, 11.01 (s, 1H), 8.14 (s, IH), 8.00 (s, 1H), 7.69 (s, IH), 7.22 - 7.34 (m, 3H), 7.14(s, 1H), 7.08(m, 1H), 6.77(m, 1H), 5.85 (m, J = 7.45 Hz, 1H), 1.79 (d, J = 7.02 Hz, 3H). **Reference Compound 1-17: LCMS:** 385 [M+1], **¹H NMR (400 MHz, DMSO-d₆)** δ 11.03 (s, 1H), 8.13 (s, 1H), 8.01(s, 1H), 7.68 (s, 1H), 7.23 -7.35 (m, 3H), 7.15 (s, 1H), 7.06 (m,1H), 6.78 (m, 1H), 5.84 (m, J = 7.45 Hz,1H), 1.78 (d, 3H).

### Reference Example S1-12. Synthesis of N-(1-(1-(2,6-difluorophenyl)ethyl)-1H-pyrazol-4-yl)-5-(thiophen-2-yl)isoxazole-3-carboxamide (Reference Compound 1-18 and Reference Compound 1-19)

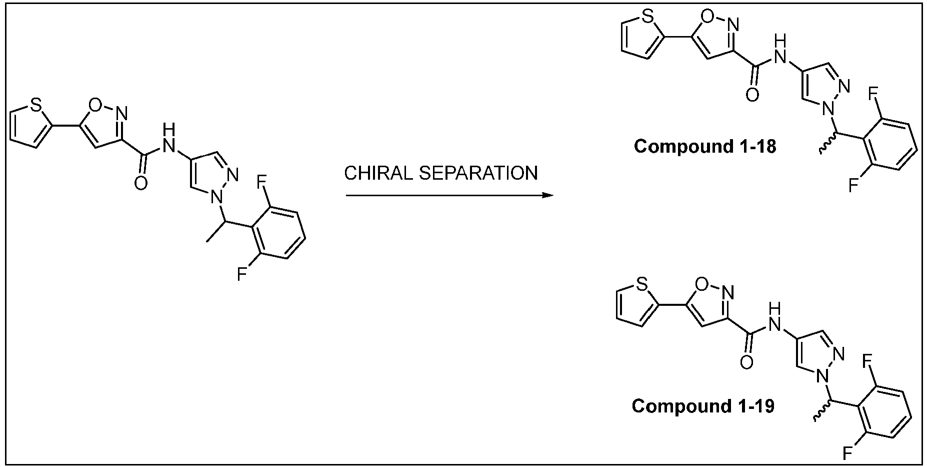

The racemic mixture of N-(1-(1-(2,6-difluorophenyl)ethyl)-1H-pyrazol-4-yl)-5-(thiophen-2-yl)isoxazole-3-carboxamide (185mg,) was purified by chiral HPLC to obtain N-(1-(1-(2,6-difluorophenyl)ethyl)-1H-pyrazol-4-yl)-5-(thiophen-2-yl)isoxazole-3-carboxamide as a first-eluting isomer **(Reference Compound 1-18)** and a second-eluting isomer **(Reference Compound 1-19).** The isomers were separated by chiral SFC (Daicel Chiralpak^{®}-IA, 250×20 mm, 5µm). Isocratic program with analytical grade liquid carbon dioxide and HPLC grade methanol, total flow: 56g/min, co-Solvent Percentage: 25%.

**Reference Compound 1-18:** yield = 50 mg; elution time = 6.3 min. **Reference Compound 1-19:** yield = 50 mg; elution time = 9.3 min.

LCMS Mass [M+1]: 401.40; **¹H NMR (400 MHz, DMSO-d₆)** δ 11.01 (s, 1H), 8.14 (s, 1H), 8.00 (d, *J* = 1.32 Hz, 1H), 7.64 (s, 1H), 7.35 - 7.45 (m, 2H), 7.28 (d, *J* = 3.51 Hz, 1H), 7.19 - 7.26 (m, 1H), 7.14 (s, 1H), 6.77 (dd, *J =* 1.75, 3.51 Hz, 1H), 5.98 - 6.06 (m, 1H), 1.92 (d, *J =* 6.58 Hz, 3H).

### Reference Example S1-13. Synthesis of 5-(furan-2-yl)-N-(1-(1-(2,4, 6-trifluorophenyl)ethyl)-1H-pyrazol-4-yl)isoxazole-3-carboxamide (Reference Compound 1-20 and Reference Compound 1-21)

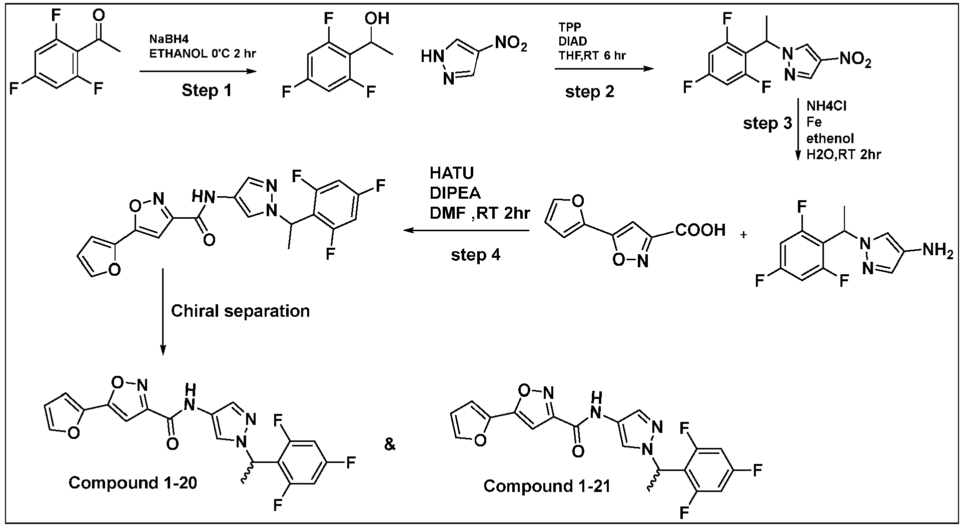

**Step 1: Synthesis of 1-(2,4,6-trifluorophenyl)ethan-1-ol.** To a stirred solution of 1-(2,4,6-trifluorophenyl)ethanone (1.00 g, 5.74 mmol, 1.0 eq.) in ethanol (15 ml) was added NaBH4 (0.437 g 11.49 mmol, 2 eq.) portion wise at 0 deg C and allowed to stir at RT for 2 hr, reaction progress was monitored by TLC and LCMS. After completion of the reaction, the reaction mixture was distilled & workup done by water 10 ml & extracted with ethyl acetate (2 x 20 mL) times. The organic layer was separated and evaporated under reduced pressure to give product 1-(2,4,6-trifluorophenyl)ethan-1-ol (1.00 g).

**Step 2: Synthesis of 4-nitro-1-(1-(2,4,6-trifluorophenyl)ethyl)-1H-pyrazole.** To a solution of 1-(2,4,6-trifluorophenyl)ethan-1-ol ( 1.00 g, 5.68 mmol, 1 eq) & 4-nitro-1H-pyrazole (642 mg, 5.68 mmol, 1.0 eq), in THF (15 ml) was added TPP (2.23 gm 8.522 mmol, 1.5 eq) was cooled to 0 deg C & DIAD (1.72 ml, 8.522 mmol, 1.5 eq.) was added drop wise under inert condition . After addition the reaction mixture was stirred at RT, and reaction progress was monitored by TLC and LCMS. After reaction completion, workup by water 10 ml & extracted with ethyl acetate (2 x 20 ml) times. The organic layer was separated and evaporated under reduced pressure to give crude which was purified by CombiFlash chromatography to obtain pure product 4-nitro-1-(1-(2,4,6-trifluorophenyl)ethyl)-1H-pyrazole (1.2 g)

**Step 3: Synthesis of 1-(1-(2,4,6-trifluorophenyl)ethyl)-1H-pyrazol-4-amine.** To a solution of 4-nitro-1-(1-(2,4,6-trifluorophenyl)ethyl)-1H-pyrazole (1.2 g, 4.428 mmol, 1 eq.) in EtOH : H2O (1:1) 10 ml, was added NH₄Cl (1.195 g, 22.140 mmol , 5 eq) & Fe (1.239 g , 22.140 mmol, 5 eq). Reaction progress was monitored by TLC and LCMS. After reaction completion, the reaction mixture was distilled & workup done by water (10 ml) & extracted with ethyl acetate (2 x 20 ml). The organic layer was collected and concentrated to give product 1-(1-(2,4,6-trifluorophenyl)ethyl)-1H-pyrazol-4-amine (920 mg).

**Step 4: Synthesis of 5-(furan-2-yl)-N-(1-(1-(2,4,6-trifluorophenyl)ethyl)-1H-pyrazol-4-yl)isoxazole-3-carboxamide.** To a solution of 1-(1-(2,4,6-trifluorophenyl)ethyl)-1H-pyrazol-4-amine (900 mg, 5.027 mmol, 1 eq.), in DMF (10 ml), HATU (1.91 g, 5.027 mmol, 1 eq.) & DIPEA (2.62 ml, 15.08 mmol, 3 eq ) was added & stirred for 5 min then was added 5-(furan-2-yl)isoxazole-3-carboxylic acid (1.21 g, 5.027 mmol, 1 eq), and stirred the reaction mixture at RT. Reaction progress was monitored by TLC and LCMS. After reaction completion of the reaction workup done by cold water (30 ml) & extracted with ethyl acetate (2 x 50 mL). The organic layer was collected and dried over sodium sulphate and concentrated to give crude product which was purified by CombiFlash chromatography by using hexane : ethyl acetate to obtain product 5-(furan-2-yl)-N-(1-(1-(2,4,6-trifluorophenyl)ethyl)-1H-pyrazol-4-yl)isoxazole-3-carboxamide (350 mg). **LCMS:** 403 [M+H].

### Example S-14. Synthesis of N-(1-(1-(2,6-dichlorophenyl)ethyl)-5-methyl-1H-pyrazol-4-yl)-5-(furan-2-yl)isoxazole-3-carboxamide (Compound 1-22)

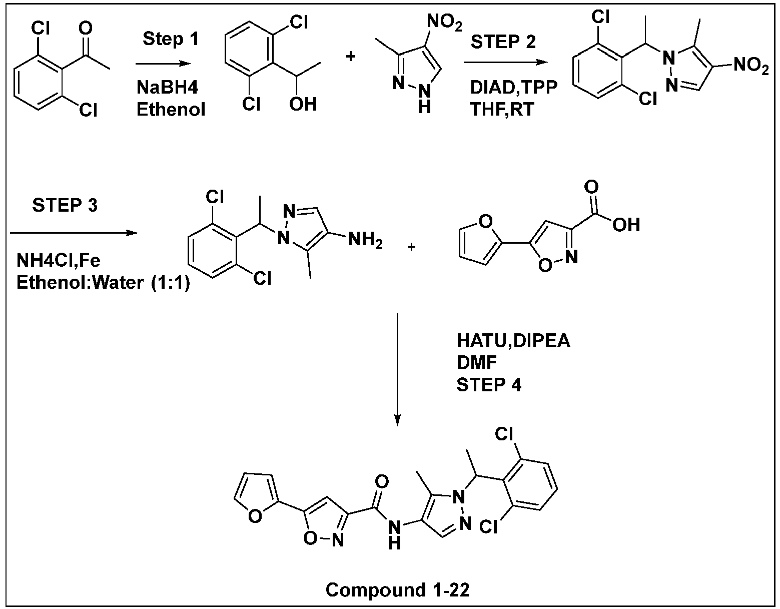

**Step 1: Synthesis of 1-(2,6-dichlorophenyl)ethan-1-ol.** To a stirred solution of 1-(2,6-dichlorophenyl)ethan-1-one (1.00 g, 5.29 mmol, 1 eq.) in ethanol (10 ml) at 0°C was added NaBH₄ (0.391 g, 10.58 mmol, 2 eq.), stir the resulting reaction mixture at RT for 2 hr, and reaction progress was monitored by TLC and LCMS. After reaction completion, the reaction mixture was diluted with water 3 ml & distils whole ethanol & the reaction mixture was extracted with ethyl acetate (100 ml x 2) and water (100 mL). The organic layer was separated and evaporated under reduced pressure to give crude product 1-(2,6-dichlorophenyl)ethan-1-ol (1.01 g ). **LCMS:** 191 [M+H]

**Step 2: Synthesis of 1-(1-(2,6-dichlorophenyl)ethyl)-5-methyl-4-nitro-1H-pyrazole.** To a stirred solution of 1-(2,6-dichlorophenyl)ethan-1-ol (1.00 g, 5.23 mmol, 1 eq.) 3-methyl-4-nitro-1H-pyrazole (0.700 g, 5.23 mmol, 1 eq.) & TPP (2.05 g , 7.85 mmol, 1.5 eq.) in THF (10 ml ) was added DIAD (1.6 ml, 7.85 mmol, 1.5 eq.) at 0°C drop wise, and allowed to stir at RT for 4 hr. Reaction progress was monitored by TLC and LCMS. After reaction completion, the reaction mixture was extracted with ethyl acetate and water (2 x 500 mL). The organic layer was separated and evaporated under reduced pressure to give crude product which was further purified by CombiFlash chromatography to give 1-(1-(2,6-dichlorophenyl)ethyl)-5-methyl-4-nitro-1H-pyrazole (1.32 g). **LCMS:** 299.9 [M+H]

**Step 3: Synthesis of 1-(1-(2,6-dichlorophenyl)ethyl)-5-methyl-1H-pyrazol-4-amine.** To a solution of 1-(1-(2,6-dichlorophenyl)ethyl)-5-methyl-4-nitro-1H-pyrazole (1.0 g, 3.344 mmol, 1 eq.), in ethanol : water (1:1, 10 ml) was added NH4Cl (0.903 g, 16.722 mmol, 5 eq.), & Fe (0.936 g, 16.722 mmol, 5 eq.), stirred the resulting reaction mixture at 80°C for 4 hr and the reaction progress was monitored by TLC and LCMS. After completion of reaction, RM was filtered through celite bed, ethanol evaporated and extracted with ethyl acetate and water (2 x 50 mL). Organic layer was collected and concentrated to give crude product 1-(1-(2,6-dichlorophenyl)ethyl)-5-methyl-1H-pyrazol-4-amine (0.560 g). **LCMS:** 270.02 [M+H].

**Step 4: Synthesis of N-(1-(1-(2,6-dichlorophenyl)ethyl)-5-methyl-1H-pyrazol-4-yl)-5-(furan-2-yl)isoxazole-3-carboxamide.** A solution of 5-(furan-2-yl)isoxazole-3-carboxylic acid (0.365 g, 2.044 mmol, 1 eq.) & HATU (0.776 gm, 2.044 mmol, 1 eq.) in DMF (5 mL) was stirred for 5 min & then DIPEA (1 ml, 6.133 mmol, 3 eq.) & 1-(1-(2,6-dichlorophenyl)ethyl)-5-methyl-1H-pyrazol-4-amine (0.550 g, 2.044 mmol, 1 eq.) was added. The resulting reaction mixture was stirred at RT for 4 hr and reaction progress was monitored by TLC and LCMS. After completion of the reaction, the mixture was extracted with ethyl acetate and water (2 x 25 mL). The organic layer was collected and concentrated to give crude product which was purified by CombiFlash chromatography to give N-(1-(1-(2,6-dichlorophenyl)ethyl)-5-methyl-1H-pyrazol-4-yl)-5-(furan-2-yl)isoxazole-3-carboxamide (80 mg). **LCMS:** 431.0 [M+H], **¹H NMR (400MHz, DMSO-d₆)** 10.19 (s, 1H), 7.99 (s, 1 H), 7.64 (s,1 H), 7.49 -7.13 (s, 5H), 6.76 (d, *J =* 3.5 Hz, 1 H), 6.26 (m, 1 H), 1.92 (d, *J* = 7.0 Hz, 3 H), 1.80 (s, 3 H).

### Example S1-15. Synthesis of N-(1-(1-(2,6-dichlorophenyl)ethyl)-3-methyl-1H-pyrazol-4-yl)-5-(furan-2-yl)isoxazole-3-carboxamide (Compound 1-23 and Compound 1-24).

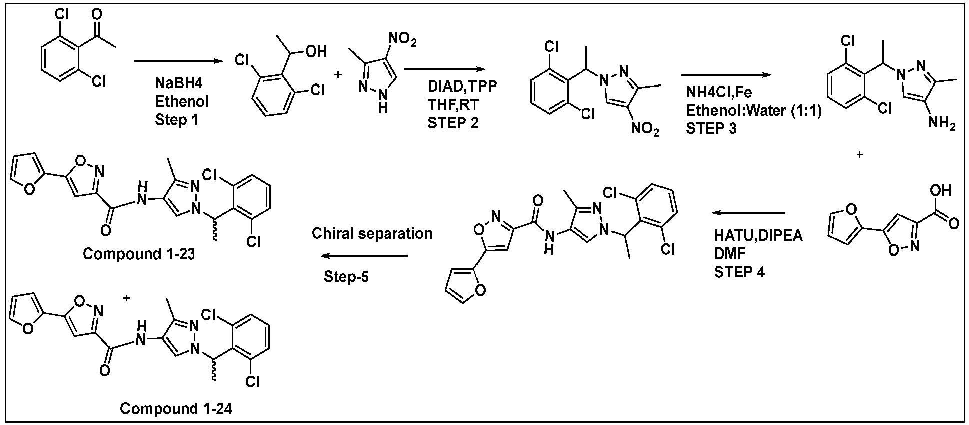

**Step 1: Synthesis of 1-(2,6-dichlorophenyl)ethan-1-ol.** To a stirred solution of 1-(2,6-dichlorophenyl)ethan-1-one (1.00 g, 5.29 mmol, 1 eq.) in ethanol (10 ml) at 0°C was added NaBH4 (0.391 g, 10.58 mmol, 2 eq. ) and the resulting reaction mixture was stirred at RT for 2 hr, Reaction progress was monitored by TLC and LCMS. After the reaction completion was added water (3 ml) & distil whole ethanol & the reaction mixture was extracted with ethyl acetate (100 ml x 2) and water (100 mL). The organic layer was separated and evaporated under reduced pressure to give crude product 1-(2,6-dichlorophenyl)ethan-1-ol (1.01 g). **LCMS:** 191 (M+H)⁺

**Step 2: Synthesis of 1-(1-(2,6-dichlorophenyl)ethyl)-3-methyl-4-nitro-1H-pyrazole.** To a stirred solution of 1-(2,6-dichlorophenyl)ethan-1-ol (1.00 g, 5.23 mmol, 1 eq.) and 3-methyl-4-nitro-1H-pyrazole (0.700 g, 5.23 mmol, 1 eq. ) & TPP (2.05 g, 7.85 mmol, 1.5 eq.) in THF (10 ml) was added DIAD (1.6 ml, 7.85 mmol, 1.5 eq. ) at 0°C drop wise, and allowed to stir at RT for 4 hr. Reaction progress was monitored by TLC and LCMS. After reaction completion, the reaction mixture was extracted with ethyl acetate (100 ml x 2) and water (100 mL). The organic layer was separated and evaporated under reduced pressure to give crude product which was further purified by CombiFlash chromatography to give 1-(1-(2,6-dichlorophenyl)ethyl)-3-methyl-4-nitro-1H-pyrazole (1.32 g). **LCMS:** 299.9 [M+H].

**Step 3: Synthesis of 1-(1-(2,6-dichlorophenyl)ethyl)-3-methyl-1H-pyrazol-4-amine.** To a solution of 1-(1-(2,6-dichlorophenyl)ethyl)-3-methyl-4-nitro-1H-pyrazole (1.0 g, 3 344. mmol, 1 eq.) in ethanol : water (1:1, 10 ml) was added NH4Cl (0.903 g, 16.722 mmol, 5 eq.), & Fe (0.936 g, 16.722 mmol, 5 eq.). The resulting reaction mixture was stirred at 80°C for 4 hr. and reaction progress was monitored by TLC and LCMS. After completion of reaction, RM was filtered through a Celite bed, ethanol was evaporated and extracted with ethyl acetate and water (2 x 50 mL); the organic layer was collected and concentrated to give crude product 1-(1-(2,6-dichlorophenyl)ethyl)-3-methyl-1H-pyrazol-4-amine (0.560 g). **LCMS:** 270.02 [M+H].

**Step 4: Synthesis of N-(1-(1-(2,6-dichlorophenyl)ethyl)-3-methyl-1H-pyrazol-4-yl)-5-(furan-2-yl)isoxazole-3-carboxamide.** A solution of 5-(furan-2-yl)isoxazole-3-carboxylic acid (0.332 gm, 1.858 mmol, 1 eq.) & HATU (0.7063 gm, 1.858 mmol, 1 eq.) in DMF (5 mL) was stirred for 5 min & the was added DIPEA (1 ml, 5.576 mmol, 3 eq.) & 1-(1-(2,6-dichlorophenyl)ethyl)-3-methyl-1H-pyrazol-4-amine (0.500 g, 1.858 mmol, 1 eq). The resulting reaction mixture was stirred at RT for 4 hr, and the reaction progress was monitored by TLC and LCMS. After completion of the reaction, the reaction mixture was extracted with ethyl acetate and water (2 x 25 mL). The organic layer was collected and concentrated to give crude product which was purified by CombiFlash chromatography to obtain N-(1-(1-(2,6-dichlorophenyl)ethyl)-3-methyl-1H-pyrazol-4-yl)-5-(furan-2-yl)isoxazole-3-carboxamide (200 mg). **LCMS:** 431.0 [M+H], **¹H NMR (400 MHz, DMSO-d₆)** 10.28 (s, 1H), 7.91 - 8.14 (m, 2H), 7.43 - 7.51 (m, 2H), 7.37 (d, *J* = 7.89 Hz, 1H), 7.27 (d, *J* = 3.51 Hz, 1H), 7.16 (s, 1H), 6.77 (dd, *J* = 1.75, 3.51 Hz, 1H), 3.33 (s, 6H), 2.12 (s, 3H), 1.92 (d, *J* = 7.02 Hz, 3H).

**Step-5: Separation of isomers of N-(1-(1-(2,6-dichlorophenyl)ethyl)-3-methyl-1H-pyrazol-4-yl)-5-(furan-2-yl)isoxazole-3-carboxamide.** The racemic mixture of N-(1-(1-(2,6-dichlorophenyl)ethyl)-3-methyl-1H-pyrazol-4-yl)-5-(furan-2-yl)isoxazole-3-carboxamide (90 mg,) was purified by chiral HPLC to obtain a first-eluting isomer (Compound 1-23; 30 mg) and a second-eluting isomer (Compound 1-24; 30 mg).

**Compound 1-23: LCMS:** 431 [M+H] ⁺; **¹H NMR (400 MHz, DMSO-d₆)** 10.28 (s, 1H), 8.05 - 8.01 (d, *J=* 0.88 Hz, 2H), 7.48 (d, *J* = 8.33 Hz, 2H), 7.32 - 7.16 (s, 3H), 6.77 (dd, *J* = 2.19, 3.51 Hz, 1H), 6.02 - 6.17 (m, 1H), 2.04 - 2.15 (m, 3H), 1.92 (d, *J =* 7.02 Hz, 3H). **Compound 1-24: LCMS:** 431 [M+H] ⁺; **¹H NMR (400 MHz, DMSO-d₆)** 10.28 (s, 1H), 8.05 - 8.01 (d, *J* = 0.88 Hz, 2H), 7.48 (d, *J* = 8.33 Hz, 2H), 7.32 - 7.16 (s, 3H), 6.77 (dd, *J=* 2.19, 3.51 Hz, 1H), 6.02 - 6.17 (m, 1H), 2.04 - 2.15 (m, 3H), 1.92 (d, *J* = 7.02 Hz, 3H).

### Reference Example SI-16. Synthesis of N-(1-(1-(2,4-bis(trifluoromethyl)phenyl)ethyl)-1H-pyrazol-4-yl)-3-(pyrazin-2-yl)isoxazole-5-carboxamide (Reference Compound 1-25 and Reference Compound 1-26).

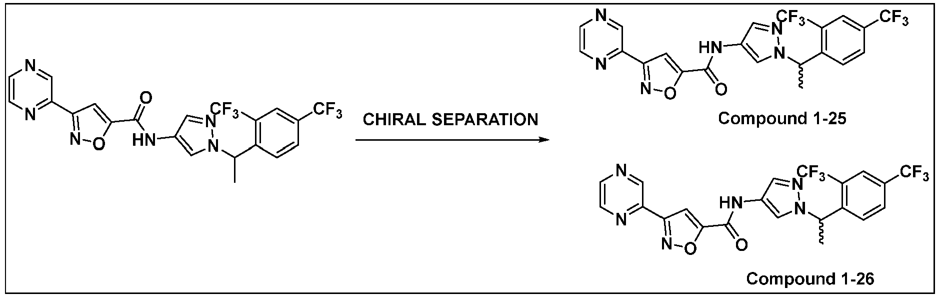

The racemic mixture of N-(1-(1-(2,4-bis(trifluoromethyl)phenyl)ethyl)-1H-pyrazol-4-yl)-3-(pyrazin-2-yl)isoxazole-5-carboxamide (130 mg) was purified by chiral HPLC to obtain a first-eluting isomer **(Reference Compound 1-25)** and a second-eluting isomer **(Reference Compound 1-26)**. The enantiomers were separated by chiral SFC (Daicel Chiralpak^{®}-IC, 250 ×21 mm, 5µm). Isocratic program with analytical grade liquid carbon dioxide and HPLC grade methanol, total flow:56g/min , co-solvent percentage: 33%.

**Reference Compound 1-25:** yield = 29 mg; elution time = 3.0 min. **Compound 1-26:** yield = 32 mg; elution time = 4.2 min.

**Reference Compound 1-25: LCMS:** 497.11 [M+H] ⁺; **¹H NMR (400 MHz, DMSO-*d*₆) δ ppm** 11.24 (s, 1 H) 9.32 (s, 1 H) 8.75 - 8.94 (m, 2 H) 8.21 (s, 1 H) 8.02 - 8.16 (m, 2 H) 7.70 - 7.86 (m, 3 H) 5.95 (d, *J*=7.02 Hz, 1 H) 1.88 (d, *J*=7.02 Hz, 3 H). **Reference Compound 1-26: LCMS:** 497.11 [M+H] ⁺, **¹H NMR (400 MHz, DMSO-*d*₆) δ ppm** 11.22 (s, 1 H) 9.30 (s, 1 H) 8.73 - 8.94 (m, 2 H) 8.21 (s, 1 H) 8.01 - 8.16 (m, 2 H) 7.74 - 7.86 (m, 3 H) 5.92 (d, J=7.02 Hz, 1 H) 1.84 (d, *J*=7.02 Hz, 3 H).

### Biological Examples

### Example B1. ERSE ATF6-luciferase assays

To understand how exemplary compounds of the invention modulate the activity of ATF6 in absence or presence of endoplasmic reticulum (ER) stress, a Human bone osteosarcoma (U2-OS)-based TRE-luciferase reporter stable cell line was generated to determine the modulation of transcription of ATF6 target genes.

U2-OS cells were obtained from the American Type Culture Collection (ATCC HTB-96, ATCC Manassas, VA) and were cultured with growing medium containing Dulbecco's Modified Eagle's Medium (DMEM) (Cat. No.:SH30023.02, HyClone) supplemented with fetal bovine serum (FBS) 10% (Cat. No.: 16000044, Gibco) and 1% penicillin-streptomycin antibiotic cocktail (Cat. No.: SV30010, Hyclone).

Cignal Lenti ATF6 luc reporter (Qiagen #CLS-6031L) was used to produce a stable cell line in U2-OS cells (U2-OS ATF6 TRE-luciferase reporter). The lenti ATF6 reporter is a preparation of replication incompetent, VSV-g pseudotyped lentivirus particles expressing the firefly luciferase gene under the control of a minimal (m)CMV promoter and tandem repeats of the ATF6 transcriptional response element (TRE). The number of response elements as well as the intervening sequence between response elements has been experimentally optimized to maximize the signal to noise ratio.

Exemplary compounds of the invention and reference compounds were prepared from powder as 10 mM stock solutions in dimethyl sulfoxide (DMSO; Cat. No. #D2650, Sigma Aldrich) and stored at - 80°C in presence of N₂ neutral atmosphere.

For the primary screening, 40,000 U2-OS ATF6 TRE-luciferase reporter cells were plated in poly-D-lysine (Cat. No.:P2636, Sigma) pre-coated white 96-well plates (Thermo Scientific Nunc #136101) with 100 µL of growing medium. Cells were incubated in humidified chambers for 24 hr.

For testing exemplary compounds in presence of ER-stress, cells were pre-treated for 30 min with 50 µL growing medium containing either vehicle (DMSO), 1 or 10 µM test compound. After this pre-incubation, 50 µL of a solution containing 0.2 µM of the ER stress-inducer thapsigargin (Tg) was added to the appropriate wells. The Tg solution also contains vehicle or test compounds at 1 or 10 µM as indicated. The final concentration of DMSO in each well was kept at 0.3%. Plates were incubated for 8 hr in humidified chambers.

After the 8 hr incubation, plates were cooled down to room temperature for 10 min prior to the luciferase assays. Luciferase reactions were performed using Luciferase Assay System (Cat. No.:E4550, Promega). Briefly, each well was washed with 100 µl of PBS 1X and, then, 20 µl of lysis reagent was added into each well. Plates were shaken for 10 min and, then, 50 µl of Luciferase Assay Reagent was added to each well. Luminescence was determined by integration of 1 s with a gain of 110 in a Synergy 4 Microplate reader. All measurements were carried out in triplicate.

The average activity determined from the wells containing vehicle only (DMSO, 0% of ATF6 activity) was used as blank and subtracted from the rest of measurements. The average activity determined from the wells containing Tg only was used as 100% of ATF6 activity. The percentage of modulation of exemplary compounds was calculated by normalizing values to potential maximal activation with Tg. In this assay, exemplary compounds showing ATF6 activity higher than 100% (positive modulation) suggest an activator activity for those molecules, while compounds showing ATF6 activity less than 100% (negative modulation) suggest an inhibitory modulation.

ATF6 activities of exemplary compounds at 1 and 10 µM in presence Tg-induced ER stress tested in the U2-OS ATF6 TRE-luciferase reporter cells were determined and are shown in Table 2.

**Table 2: ATF6 activity in presence of ER stress in ATF6-luc cell reporter**

| **Compound No.** | **ATF6 activity @ 1 µM [%]** | **ATF6 activity @ 10 µM [%]** |
|---|---|---|
| Ceapin-A7 | --- | --- |
| Ceapin-A4 | + | ++ |
| Ceapin-A8 | + | --- |
| 1-1, Enantiomer A | +++ | +++ |
| 1-1, Enantiomer B | ++ | + |
| Reference 1-2, Enantiomer A | - | ---- |
| Reference 1-2, Enantiomer B | - | - |
| Reference 1-3 | + | ++ |
| 1-4 | +++ | +++ |
| Reference 1-5 | + | +++ |
| Reference 1-6 | + | - |
| Reference 1-11 | --- | --- |
| Reference 1-12 | - | - |
| Reference 1-13 | - | + |
| 1-14 | +++ | +++ |
| 1-15 | + | + |
| Reference 1-16 | + | ++ |
| Reference 1-17 | + | + |
| 1-22 | - | + |
| Reference 1-25 | - | - |
| Reference 1-26 | --- | --- |

| | | |
|---|---|---|
| Ceapin-A4, Ceapin-A7, and Ceapin-A8 refer to compounds described in Gallagher et al. eLife 2016;5:e11878; for % ATF6 activity: --- refers to < 50% at 1 or 10 µM test compound; -- refers to 50% < % activity < 75% at 1 or 10 µM test compound; - refers to 75% < % activity < 100% at 1 or 10 µM; + refers to 100% < % activity < 125% at 1 or 10 µM; ++ refers to 125% < % activity < 150% at 1 or 10 µM test compound; +++ refers to >150% at 1 or 10 µM test compound; NT: not tested. | | |

For testing exemplary compounds in absence of ER-stress, cells were treated for 8 hr with 100 µL growing medium containing either vehicle (DMSO), 1 or 10 µM test compound or 0.1 µM Tg. The final concentration of DMSO in each well was kept at 0.3%. Plates were incubated in humidified chambers.

After 8 hr of incubation, plates were cooled down to room temperature for 10 min prior to luciferase assays. Luciferase reactions were performed as above. Luminescence was read by integration of 1 s with a gain of 110 in a Synergy 4 Microplate reader. All measurements were carried out in triplicate.

The average activity determined from the wells containing vehicle only (DMSO, 0% of ATF6 activity) was used as blank and subtracted from the rest of measurements. The percentage of modulation of exemplary compound was calculated by normalizing values to potential maximal activation with Tg.

ATF6 activities of exemplary compounds at 1 and 10 µM in absence of Tg-induced ER stress in the U2-OS ATF6 TRE-luciferase reporter cells were determined and are shown in Table 3.

**Table 3: ATF6 activity in absence of ER stress in ATF6-luc cell reporter**

| **Compound No.** | **ATF6 activity @ 1 µM - No Tg [%]** | **ATF6 activity @ 10 µM - No Tg [%]** |
|---|---|---|
| 1-1 | + | + |
| Reference 1-13 | + | + |
| 1-14 | + | + |
| 1-15 | + | + |
| Reference 1-16 | + | + |
| Reference 1-17 | + | + |
| 1-22 | + | + |

| | | |
|---|---|---|
| For % ATF6 activity: + refers to 0% < % activity < 25% at 1 or 10 µM test compound; ++ refers to 25% < % activity < 50% at 1 or 10 µM test compound; +++ refers to % activity >50 % at 1 or 10 µM test compound; NT: not tested. | | |

Although the foregoing invention has been described in some detail by way of illustration and example for purposes of clarity of understanding, it is apparent to those skilled in the art that certain minor changes and modifications will be practiced. Therefore, the description and examples should not be construed as limiting the scope of the invention.

## Claims

1. A compound of formula (I): 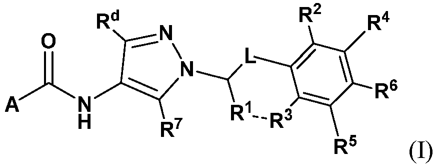
or a stereoisomer thereof, or a pharmaceutically acceptable salt of any of the foregoing,
wherein:
R^{d} is H or C₁-C₆alkyl;
R¹ is C₁-C₆alkyl, C₃-C₈cycloalkyl, or C₁-C₆ haloalkyl;
L is -CH₂- or is absent;
-- is a bond or is absent;
wherein: 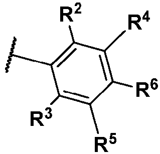 is 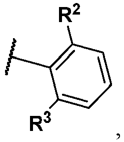
wherein R² and R³ are each halo,
A is 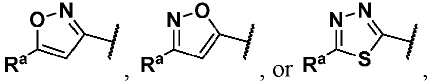
R^{a} is 5- or 6-membered heteroaryl, wherein the 5- or 6-membered heteroaryl is unsubstituted or substituted with one to four groups selected from the group consisting of OH, halo, C₁-C₆alkyl and C₁-C₆alkoxy, wherein when A is 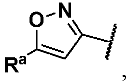 R^{a} is selected from the group consisting of 2-furyl, 2-pyridinyl, 2-pyrimidinyl, 4-pyrimidinyl, and 2-pyrazinyl, each of which is unsubstituted or substituted with one to four groups selected from the group consisting of OH, halo, C₁-C₆alkyl and C₁-C₆alkoxy;
provided that, when A is 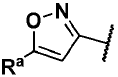 and R^{a} is 2-furyl, at least one of (*i.*)-(*ii*.) applies:
(*i*.) L is absent, and R¹ is C₁-C₆alkyl;
*(ii.)* R² and R³ are each Cl; and
R⁷ is H, C₁-C₆alkyl or C₁-C₆haloalkyl,
provided that, when R^{d} is C₁-C₆alkyl, R⁷ is H, and, when R⁷ is C₁-C₆alkyl, R^{d} is H.

2. The compound of claim 1, or a stereoisomer thereof, or a pharmaceutically acceptable salt of any of the foregoing, wherein the 5- or 6-membered heteroaryl of R^{a} is selected from the group consisting of 2-furyl, 2-pyridinyl, 2-pyrimidinyl, 4-pyrimidinyl, and 2-pyrazinyl.

3. The compound of claim 1 or 2, or a stereoisomer thereof, or a pharmaceutically acceptable salt of any of the foregoing, wherein A is 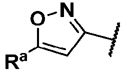 and R^{a} is 2-furyl.

4. The compound of claim 1 or 2, or a stereoisomer thereof, or a pharmaceutically acceptable salt of any of the foregoing, wherein A is 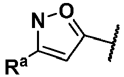 and R^{a} is selected from the group consisting of 2-furyl, 2-pyridinyl, 2-pyrimidinyl, 4-pyrimidinyl, and 2-pyrazinyl.

5. The compound of claim 1 or 2, or a stereoisomer thereof, or a pharmaceutically acceptable salt of any of the foregoing, wherein A is 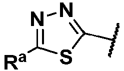 and R^{a} is selected from the group consisting of 2-furyl, 2-pyridinyl, 2-pyrimidinyl, 4-pyrimidinyl, and 2-pyrazinyl.

6. The compound of any one of claims 1 to 5, wherein L is absent.

7. The compound of any one of claims 1 to 6, wherein R¹ is C₁₋₆alkyl.

8. The compound of any one of claims 1-3, or a stereoisomer thereof, or a pharmaceutically acceptable salt of any of the foregoing, wherein A is 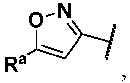 R^{a} is 2-furyl, L is absent and R¹ is C₁-C₆alkyl.

9. The compound of any one of claims 1 to 8, wherein 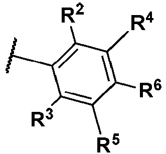 is

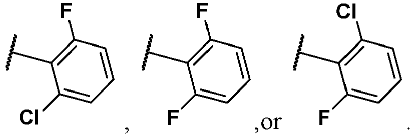

10. The compound of any one of claims 1 to 8, wherein 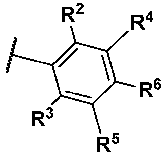 is

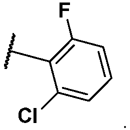

11. The compound of any one of claims 1 to 8, wherein 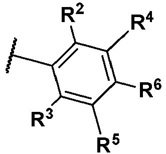 is

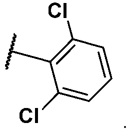

12. The compound of any one of claims 1-11, wherein the compound is selected from the group consisting of:
N-(1-(1-(2,6-dichlorophenyl)ethyl)-1H-pyrazol-4-yl)-5-(furan-2-yl)isoxazole-3-carboxamide;
N-(1-(1-(2-chloro-6-fluorophenyl)ethyl)-1H-pyrazol-4-yl)-5-(furan-2-yl)isoxazole-3-carboxamide;
N-(1-(1-(2,6-dichlorophenyl)ethyl)-1H-pyrazol-4-yl)-5-(furan-2-yl)-1,3,4-thiadiazole-2-carboxamide;
N-(1-(1-(2,6-difluorophenyl)ethyl)-1H-pyrazol-4-yl)-5-(furan-2-yl)isoxazole-3-carboxamide;
N-(1-(1-(2,6-dichlorophenyl)ethyl)-3-methyl-1H-pyrazol-4-yl)-5-(furan-2-yl)isoxazole-3-carboxamide;
N-(1-(1-(2,6-dichlorophenyl)ethyl)-1H-pyrazol-4-yl)-5-(thiophen-2-yl)isoxazole-3-carboxamide;
N-(1-(1-(2,6-difluorophenyl)ethyl)-1H-pyrazol-4-yl)-5-(thiophen-2-yl)isoxazole-3-carboxamide; and
N-(1-(1-(2,6-dichlorophenyl)ethyl)-5-methyl-1H-pyrazol-4-yl)-5-(furan-2-yl)isoxazole-3-carboxamide,
or a stereoisomer thereof, or a pharmaceutically acceptable salt of any of the foregoing.

13. The compound of any one of claims 1-12, or a pharmaceutically acceptable salt thereof, wherein the carbon bearing R¹ is in the "S" configuration.

14. The compound of any one of claims 1-12, or a pharmaceutically acceptable salt thereof, wherein the carbon bearing R¹ is in the "R" configuration.

15. A pharmaceutical composition comprising a compound of any one of claims 1-14, or a stereoisomer thereof, or a pharmaceutically acceptable salt of any of the foregoing, and a pharmaceutically acceptable carrier.

16. The compound of any of claims 1-14, or a stereoisomer thereof, or a pharmaceutically acceptable salt of any of the foregoing, or a pharmaceutical composition of claim 15, for use in therapy.

17. A therapeutically effective amount of a compound of any of claims 1-14, or a stereoisomer thereof, or a pharmaceutically acceptable salt of any of the foregoing, or a pharmaceutical composition of claim 15, for use in treating a disease or disorder mediated by activating transcription factor 6 (ATF6) in an individual in need thereof,
or treating a disease or disorder **characterized by** activation of ATF6 in an individual in need thereof,
preferably wherein ATF6 is ATF6α.

18. The compound or composition for use according to claim 17, wherein the disease or disorder is:
a) cancer, a neurodegenerative disease, or a vascular disease; or
b) a viral infection, hereditary cerebellar atrophy and ataxia, or Alzheimer's disease, type 2 diabetes mellitus, diabetic nephropathy, myocardial atrophy, heart failure, atherosclerosis, ischemia, ischemic heart disease, or cerebral ischemia.

19. A therapeutically effective amount of a compound of any one of claims 1-14, or a stereoisomer thereof, or a pharmaceutically acceptable salt of any of the foregoing, or a pharmaceutical composition of claim 15 for use in treating cancer in an individual in need thereof.

20. A therapeutically effective amount of a compound of any one of claims 1-14, or a stereoisomer thereof, or a pharmaceutically acceptable salt of any of the foregoing, or a pharmaceutical composition of claim 15, for use in treating a disease or disorder associated with angiogenesis in an individual in need thereof.

21. A therapeutically effective amount of a compound of any one of claims 1-14, or a stereoisomer thereof, or a pharmaceuticall acceptable salt of any of the foregoing, or a pharmaceutical composition of claim 15, for use in treating a metabolic disorder in an individual in need thereof.

## Patentansprüche

1. Verbindung der Formel (I): 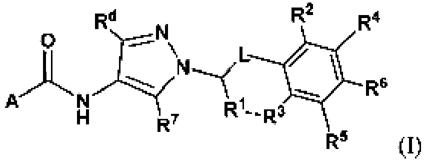
oder ein Stereoisomer davon oder ein pharmazeutisch annehmbares Salz eines beliebigen der Vorgenannten,
worin:
R^{d} H oder C₁₋₆-Alkyl ist,
R¹ C₁₋₆-Alkyl, C₃₋₈-Cycloalkyl oder C₁₋₆-Halogenalkyl ist,
L -CH₂- ist oder fehlt,
-- eine Bindung ist oder fehlt,
worin: 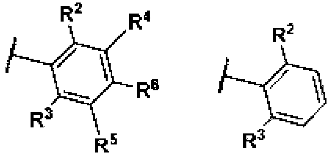 ist,
worin R² und R³ jeweils Halogen sind,
A 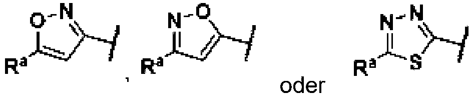 ist,
worin R^{a} ein 5- oder 6-gliedriges Heteroaryl ist, wobei das 5- oder 6-gliedrige Heteroaryl unsubstituiert oder mit einer bis vier Gruppen substituiert ist, die aus der aus OH, Halogen, C₁₋₆-Alkyl und C₁₋₆-Alkoxy bestehenden Gruppe ausgewählt sind, wobei, wenn A 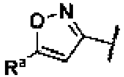 ist, R^{a} aus der aus 2-Furyl, 2-Pyridinyl, 2-Pyrimidinyl, 4-Pyrimidinyl und 2-Pyrazinyl bestehenden Gruppe ausgewählt ist, die jeweils unsubstituiert oder mit einer bis vier Gruppen substituiert sind, die aus der aus OH, Halogen, C₁₋₆-Alkyl und C₁₋₆-Alkoxy bestehenden Gruppe ausgewählt sind;
mit der Maßgabe, dass, wenn A 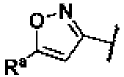 ist und R^{a} 2-Furyl ist, zumindest eines von (i.) bis (ii.) zutrifft:
(i.) L fehlt und R¹ ist C₁₋₆-Alkyl,
(ii.) R² und R³ sind jeweils Cl und
R⁷ ist H, C₁₋₆-Alkyl oder C₁₋₆-Halogenalkyl;
mit der Maßgabe, dass, wenn R^{d} C₁₋₆-Alkyl ist, R⁷ H ist und, wenn R⁷ C₁₋₆-Alkyl ist, R^{d} H ist.

2. Verbindung nach Anspruch 1 oder ein Stereoisomer davon oder ein pharmazeutisch annehmbares Salz eines beliebigen der Vorgenannten, wobei das 5- oder 6-gliedrige Heteroaryl von R^{a} aus der aus 2-Furyl, 2-Pyridinyl, 2-Pyrimidinyl, 4-Pyrimidinyl und 2-Pyrazinyl bestehenden Gruppe ausgewählt ist.

3. Verbindung nach Anspruch 1 oder 2 oder ein Stereoisomer davon oder ein pharmazeutisch annehmbares Salz eines beliebigen der Vorgenannten, worin A 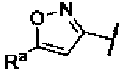 ist und R^{a} 2-Furyl ist.

4. Verbindung nach Anspruch 1 oder 2 oder ein Stereoisomer davon oder ein pharmazeutisch annehmbares Salz eines beliebigen der Vorgenannten, worin A 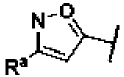 ist und R^{a} aus der aus 2-Furyl, 2-Pyridinyl, 2-Pyrimidinyl, 4-Pyrimidinyl und 2-Pyrazinyl bestehenden Gruppe ausgewählt ist.

5. Verbindung nach Anspruch 1 oder 2 oder ein Stereoisomer davon oder ein pharmazeutisch annehmbares Salz eines beliebigen der Vorgenannten, worin A 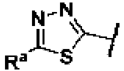 ist und R^{a} aus der aus 2-Furyl, 2-Pyridinyl, 2-Pyrimidinyl, 4-Pyrimidinyl und 2-Pyrazinyl bestehenden Gruppe ausgewählt ist.

6. Verbindung nach einem der Ansprüche 1 bis 5, worin L fehlt.

7. Verbindung nach einem der Ansprüche 1 bis 6, worin R¹ C₁₋₆-Alkyl ist.

8. Verbindung nach einem der Ansprüche 1 bis 3 oder ein Stereoisomer davon oder ein pharmazeutisch annehmbares Salz eines beliebigen der Vorgenannten, worin A 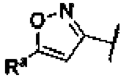 ist, R^{a} 2-Furyl ist, L fehlt und R¹ C₁₋₆-Alkyl ist.

9. Verbindung nach einem der Ansprüche 1 bis 8, worin 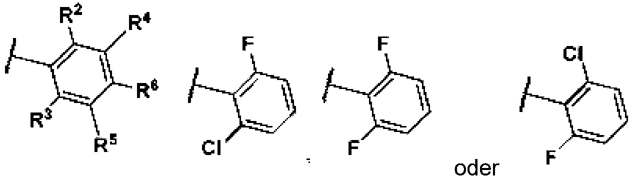 ist.

10. Verbindung nach einem der Ansprüche 1 bis 8, worin 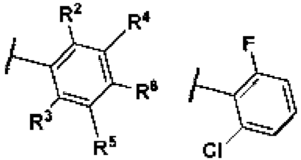 ist.

11. Verbindungen nach einem der Ansprüche 1 bis 8, worin 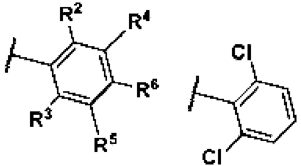 ist.

12. Verbindung nach einem der Ansprüche 1 bis 11, wobei die Verbindung aus der aus den folgenden bestehenden Gruppe ausgewählt ist:
N-(1-(1-(2,6-Dichlorphenyl)ethyl)-1H-pyrazol-4-yl)-5-(furan-2-yl)isoxazol-3-carboxamid,
N-(1-(1-(2-Chlor-6-fluorphenyl)ethyl)-1H-pyrazol-4-yl)-5-(furan-2-yl)isoxazol-3-carboxamid,
N-(1-(1-(2,6-Dichlorphenyl)ethyl)-1H-pyrazol-4-yl)-5-(furan-2-yl)-1,3,4-thiadiazol-2-carboxamid,
N-(1-(1-(2,6-Difluorphenyl)ethyl)-1H-pyrazol-4-yl)-5-(furan-2-yl)isoxazol-3-carboxamid,
N-(1-(1-(2,6-Dichlorphenyl)ethyl)-3-methyl-1H-pyrazol-4-yl)-5-(furan-2-yl)isoxazol-3-carboxamid,
N-(1-(1-(2,6-Dichlorphenyl)ethyl)-1H-pyrazol-4-yl)-5-(thiophen-2-yl)isoxazol-3-carboxamid,
N-(1-(1-(2,6-Difluorphenyl)ethyl)-1H-pyrazol-4-yl)-5-(thiophen-2-yl)isoxazol-3-carboxamid und
N-(1-(1-(2,6-Dichlorphenyl)ethyl)-5-methyl-1H-pyrazol-4-yl)-5-(furan-2-yl)isoxazol-3-carboxamid;
oder ein Stereoisomer davon oder ein pharmazeutisch annehmbares Salz einer beliebigen der Vorgenannten.

13. Verbindung nach einem der Ansprüche 1 bis 12 oder ein pharmazeutisch annehmbares Salz davon, worin das R¹ tragende Kohlenstoffatom in der "S"-Konfiguration vorliegt.

14. Verbindung nach einem der Ansprüche 1 bis 12 oder ein pharmazeutisch annehmbares Salz davon, worin das R¹ tragende Kohlenstoffatom in der "R"-Konfiguration vorliegt.

15. Pharmazeutische Zusammensetzung, die eine Verbindung nach einem der Ansprüche 1 bis 14 oder ein Stereoisomer davon oder ein pharmazeutisch annehmbares Salz eines beliebigen der Vorgenannten und einen pharmazeutisch annehmbaren Träger umfasst.

16. Verbindung nach einem der Ansprüche 1 bis 14 oder ein Stereoisomer davon oder ein pharmazeutisch annehmbares Salz eines beliebigen der Vorgenannten oder pharmazeutische Zusammensetzung nach Anspruch 15 zur Verwendung in einer Therapie.

17. Therapeutisch wirksame Menge einer Verbindung nach einem der Ansprüche 1 bis 14 oder eines Stereoisomers davon oder eines pharmazeutisch annehmbaren Salzes eines beliebigen der Vorgenannten oder pharmazeutische Zusammensetzung nach Anspruch 15 zur Verwendung bei der Behandlung einer Erkrankung oder Störung, die durch den aktivierenden Transkriptionsfaktor 6 (ATF6) vermittelt wird, bei einem bedürftigen Individuum oder zur Behandlung einer Erkrankung oder Störung, die durch Aktivierung von ATF6 gekennzeichnet ist, bei einem bedürftigen Individuum,
wobei der ATF6 vorzugsweise ATF6α ist.

18. Verbindung oder Zusammensetzung zur Verwendung nach Anspruch 17, wobei die Erkrankung oder Störung die folgende ist:
a) Krebs, eine neurodegenerative Erkrankung oder eine Gefäßerkrankung; oder
b) eine Virusinfektion, vererbte zerebelläre Atrophie und Ataxie oder Morbus Alzheimer, Typ-2-Diabetes-mellitus, diabetische Nephropathie, Myokardatrophie, Herzversagen, Atherosklerose, Ischämie, ischämische Herzerkrankung oder zerebrale Ischämie.

19. Therapeutisch wirksame Menge einer Verbindung nach einem der Ansprüche 1 bis 14 oder eines Stereoisomers davon oder eines pharmazeutisch annehmbaren Salzes eines beliebigen der Vorgenannten oder pharmazeutische Zusammensetzung nach Anspruch 15 zur Verwendung bei der Behandlung von Krebs bei einem bedürftigen Individuum.

20. Therapeutisch wirksame Menge einer Verbindung nach einem der Ansprüche 1 bis 14 oder eines Stereoisomers davon oder eines pharmazeutisch annehmbaren Salzes eines beliebigen der Vorgenannten oder pharmazeutische Zusammensetzung nach Anspruch 15 zur Verwendung bei der Behandlung einer Erkrankung oder Störung, die mit Angiogenese in Zusammenhang steht, bei einem bedürftigen Individuum.

21. Therapeutisch wirksame Menge einer Verbindung nach einem der Ansprüche 1 bis 14 oder eines Stereoisomers davon oder eines pharmazeutisch annehmbaren Salzes eines beliebigen der Vorgenannten oder pharmazeutische Zusammensetzung nach Anspruch 15 zur Verwendung einer Stoffwechselstörung bei einem bedürftigen Individuum.

## Revendications

1. Composé de formule (I) : 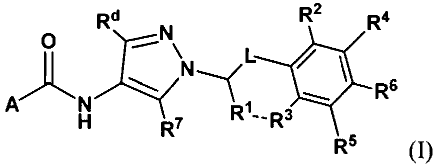 ou un stéréoisomère de celui-ci, ou un sel pharmaceutiquement acceptable de l'un quelconque des éléments précédents,
dans lequel :
R^{d} est H ou un alkyle en C₁-C₆;
R¹ est un alkyle en C₁-C₆, un cycloalkyle en C₁-C₆ ou un haloalkyle en C₁-C₆.
L est -CH₂- ou est absent ;
-- est une liaison ou est absent ;
dans lequel : 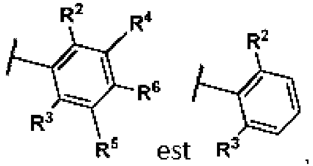
dans lequel R² et R³ sont chacun un halo,
A est 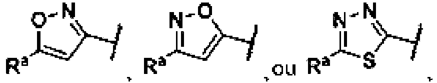
R^{a} est un hétéroaryle à 5 ou 6 chaînons, dans lequel l'hétéroaryle à 5 ou 6 chaînons est non substitué ou substitué par un à quatre groupes choisis dans le groupe comprenant OH, halo, alkyle en C₁-C₆ et alcoxy en C₁-C₆, dans lequel lorsque A est 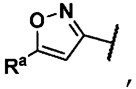 R^{a} est choisi dans le groupe comprenant 2-furyle, 2-pyridinyle, 2-pyrimidinyle, 4-pyrimidinyle et 2-pyrazinyle, dont chacun est non substitué ou substitué par un à quatre groupes choisis dans le groupe comprenant OH, halo, alkyle en C₁-C₆ et alcoxy en C₁-C₆;
à condition que, lorsque A est 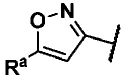 et R^{a} est 2-furyle, au moins un des *(i.)-(ii.)* s'applique :
(i.) L est absent, et R¹ est un alkyle en C₁-C₆;
(*ii.*) R² et R³ sont chacun Cl; et
R⁷ est H, un alkyle en C₁-C₆ ou un haloalkyle en C₁-C₆,
à condition que, lorsque R^{d} est un alkyle en C₁-C₆, R⁷ soit H et, lorsque R⁷ est un alkyle en C₁-C₆, R^{d} soit H.

2. Composé selon la revendication 1, ou un stéréoisomère de celui-ci, ou un sel pharmaceutiquement acceptable de l'un quelconque des éléments précédents, dans lequel l'hétéroaryle à 5 ou 6 chaînons de R^{a} est choisi dans le groupe comprenant 2-furyle, 2-pyridinyle, 2-pyrimidinyle, 4-pyrimidinyle et 2-pyrazinyle.

3. Composé selon la revendication 1 ou 2, ou un stéréoisomère de celui-ci, ou un sel pharmaceutiquement acceptable de l'un quelconque des éléments précédents, dans lequel A est 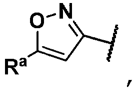 et R^{a} est 2-furyle.

4. Composé selon la revendication 1 ou 2, ou un stéréoisomère de celui-ci, ou un sel pharmaceutiquement acceptable de l'un quelconque des éléments précédents, dans lequel A est 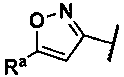 et R^{a} est choisi dans le groupe comprenant 2-furyle, 2-pyridinyle, 2-pyrimidinyle, 4-pyrimidinyle et 2-pyrazinyle.

5. Composé selon la revendication 1 ou 2, ou un stéréoisomère de celui-ci, ou un sel pharmaceutiquement acceptable de l'un quelconque des éléments précédents, dans lequel A est 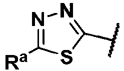 et R^{a} est choisi dans le groupe comprenant 2-furyle, 2-pyridinyle, 2-pyrimidinyle, 4-pyrimidinyle et 2-pyrazinyle.

6. Composé selon l'une quelconque des revendications 1 à 5, dans lequel L est absent.

7. Composé selon l'une quelconque des revendications 1 à 6, dans lequel R¹ est un alkyle en C₁₋₆.

8. Composé selon l'une quelconque des revendications 1 à 3, ou un stéréoisomère de celui-ci, ou un sel pharmaceutiquement acceptable selon l'un quelconque des éléments précédents, dans lequel A est 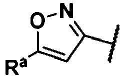 R^{a} est 2-furyle, L est absent et R¹ est un alkyle en C₁-C₆.

9. Composé selon l'une quelconque des revendications 1 à 8, dans lequel

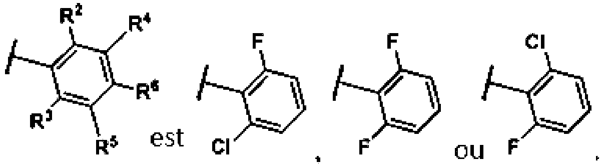

10. Composé selon l'une quelconque des revendications 1 à 8, dans lequel

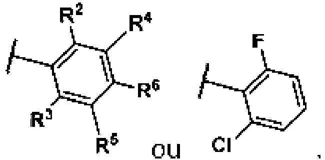

11. Composé selon l'une quelconque des revendications 1 à 8, dans lequel

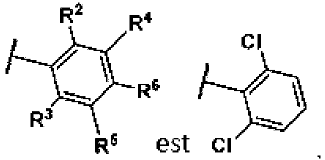

12. Composé selon l'une quelconque des revendications 1 à 11, dans lequel le composé est choisi dans le groupe comprenant :
N-(1-(1-(2,6-dichlorophényl)éthyl)-lH-pyrazol-4-yl)-5-(furan-2-yl)isoxazole-3-carboxamide ;
N-(1-(1-(2-chloro-6-fluorophényl)éthyl)-1H-pyrazol-4-yl)-5-(furan-2-yl)isoxazole-3-carboxamide ;
N-(1-(1-(2,6-dichlorophényl)éthyl)-1H-pyrazol-4-yl)-5-(furan-2-yl)-1,3,4-thiadiazole-2-carboxamide ;
N-(1-(2,6-difluorophényl)éthyle)-1H-pyrazol-4-yl)-5-(furan-2-yl)isoxazole-3-carboxamide ;
N-(1-(1- (1-(1-(2,6-dichlorophényl)éthyl)-3-méthyl-1H-pyrazol-4-yl)-5-(furan-2-yl)isoxazole-3-carboxamide ;
N-(1-(1-(1-(2,6-dichlorophényl)éthyl)-1H-pyrazol-4-yl)-5-(thiophén-2-yl)isoxazole-3-carboxamide ;
N-(1-(1-(1-(2,6-difluorophényl)éthyl)-1H-pyrazol-4-yl)-5-(thiophén-2-yl)isoxazole-3-carboxamide ; et
N-(1-(1-(1-(1-(1-(1-(2,6-dichlorophényl)éthyl)-5-méthyl-1H-pyrazol-4-yl)-5-(furan-2-yl)isoxazole-3-carboxamide,
ou un stéréoisomère de celui-ci, ou un sel pharmaceutiquement acceptable de l'un quelconque des éléments précédents.

13. Composé selon l'une quelconque des revendications 1 à 12, ou un sel pharmaceutiquement acceptable de celui-ci, dans lequel le porteur de carbone R¹ est dans la configuration "*S*"**.**

14. Composé selon l'une quelconque des revendications 1 à 12, ou un sel pharmaceutiquement acceptable de celui-ci, dans lequel le carbone portant R¹ est dans la configuration "*R*".

15. Composition pharmaceutique comprenant un composé selon l'une quelconque des revendications 1 à 14, ou un stéréoisomère de celui-ci, ou un sel pharmaceutiquement acceptable de l'un quelconque des éléments précédents, et un support pharmaceutiquement acceptable.

16. Composé selon l'une quelconque des revendications 1 à 14, ou un stéréoisomère de celui-ci, ou un sel pharmaceutiquement acceptable de l'un quelconque des éléments précédents, ou une composition pharmaceutique selon la revendication 15, pour utilisation en thérapie.

17. Quantité thérapeutiquement efficace d'un composé selon l'une quelconque des revendications 1 à 14, ou d'un stéréoisomère de celui-ci, ou d'un sel pharmaceutiquement acceptable de l'un quelconque des éléments précédents, ou d'une composition pharmaceutique selon la revendication 15, pour utilisation dans le traitement d'une maladie ou d'un trouble médié(e) par l'activation du facteur de transcription 6 (ATF6) chez un individu qui en a besoin, ou dans le traitement d'une maladie ou d'un trouble caractérisé(e) par l'activation de l'ATF6 chez un individu qui en a besoin,
l'ATF6 étant de préférence l'ATF6α.

18. Composé ou composition pour utilisation selon la revendication 17, la maladie ou le trouble étant :
a) un cancer, une maladie neurodégénérative ou une maladie vasculaire ; ou
b) une infection virale, une atrophie et une ataxie cérébelleuses héréditaires, ou la maladie d'Alzheimer, le diabète sucré de type 2, la néphropathie diabétique, l'atrophie myocardique, l'insuffisance cardiaque, l'athérosclérose, l'ischémie, la cardiopathie ischémique ou l'ischémie cérébrale.

19. Quantité thérapeutiquement efficace d'un composé selon l'une quelconque des revendications 1 à 14, ou d'un stéréoisomère de celui-ci, ou d'un sel pharmaceutiquement acceptable de l'un quelconque des éléments précédents, ou d'une composition pharmaceutique selon la revendication 15 pour utilisation dans le traitement du cancer chez un individu qui en a besoin.

20. Quantité thérapeutiquement efficace d'un composé selon l'une quelconque des revendications 1 à 14, ou d'un stéréoisomère de celui-ci, ou d'un sel pharmaceutiquement acceptable de l'un quelconque des éléments précédents, ou d'une composition pharmaceutique selon la revendication 15, pour utilisation dans le traitement d'une maladie ou d'un trouble associé(e) à l'angiogenèse chez un individu qui en a besoin.

21. Quantité thérapeutiquement efficace d'un composé selon l'une quelconque des revendications 1 à 14, ou d'un stéréoisomère de celui-ci, ou d'un sel pharmaceutiquement acceptable de l'un quelconque des éléments précédents, ou d'une composition pharmaceutique selon la revendication 15, pour utilisation dans le traitement d'un trouble métabolique chez un individu qui en a besoin.
